# EUROPEAN PATENT APPLICATION

(11) **EP 4 678 749 A1**
(43) Date of publication of application: **14.01.2026**
(21) Application number: 23912337.5
(22) Date of filing: 28.12.2023
(51) Int. Cl.: C12N 15/115, A61K 31/7088, A61K 48/00, A61P 7/02, C12N 15/11, C12Q 1/6811

(54) **NEUTRALIZABLE BLOOD COAGULATION INHIBITOR**

(30) Priority: 28.12.2022 JP 2022212461
(71) Applicant: The University of Tokyo, Bunkyo-ku, Tokyo 113-8654 (JP); Public University Corporation Nara Medical University, Nara 634-8521 (JP); LinkBIO Co., Ltd., Ibaraki 302-0021 (JP)
(72) Inventor: YOSHIMOTO, Keitaro, Tokyo 113-8654 (JP); NAGANO, Masanobu, Tokyo 113-8654 (JP); KUBOTA, Kazuki, Tokyo 113-8654 (JP); SAKATA, Asuka, Nara 634-8521 (JP); INAMI, Yuki, Ibaraki 302-0021 (JP); OSMAN, Waleed, Ibaraki 302-0021 (JP)
(74) Representative: J A Kemp LLP
(86) International application number: PCT/JP2023/047359
(87) International publication number: WO 2024/143560

(57) **Abstract**

The present invention provides a bivalent DNA aptamer and pharmaceutical use thereof. In the bivalent DNA aptamer, a DNA aptamer that binds to exosite I of thrombin and a DNA aptamer that binds to exosite II are linked via a double-stranded DNA.

## Description

### Technical Field

The present invention relates to monovalent nucleic acid aptamers, divalent nucleic acid aptamers, and neutralizable blood coagulation inhibitors comprising them.

### Background Art

Thrombin is a multifunctional serine protease and an enzyme important in a hemostatic network. It plays a role in blood coagulation through platelet activation or induction of fibrin formation by proteolysis of fibrinogen. Inhibition of the enzymatic activity of thrombin is triggered by blocking anion-binding exosites I and II, which are involved in recognition and binding of substrates. The exosite I is bound by fibrinogen, factor XI, factor XIII, and the like.

Fibrinogen is a protein present in plasma at high concentrations (2 to 4 mg/mL, 6 to 12 µM) and is converted to fibrin by thrombin to play a role in hemostasis.

Blood coagulation is a chain reaction caused by multiple factors, in which a coagulation factor is activated, the activated factor activates another coagulation factor. The initiation of this reaction is roughly divided into the intrinsic system and the extrinsic system, and both activate factor X in the common system. Activated factor Xa breaks down and activates prothrombin, together with Factor Va, to generate thrombin. Eventually, thrombin converts fibrinogen into fibrin to form a blood clot, thereby causing blood to coagulate.

Thromboembolism is a disease with high rates of morbidity and mortality. For the treatment of thromboembolism, heparin, an anticoagulant that can treat the disease, is frequently used; however, administration of heparin causes serious side effects, which has become a problem in recent years. Argatroban, a low molecular weight drug, is known as an alternative anticoagulant to heparin; however, there is a problem of the absence of neutralizing agent for reversing the drug effects if a hemorrhagic event occurs. There is a need to develop an anticoagulant that has a high anticoagulant ability and for which a neutralizing agent exists.

Nucleic acid aptamers are single-stranded functional oligonucleotides that function as molecules for specifically recognizing various molecules (e.g., metal ions, small molecules, proteins, and cells). In addition, nucleic acid aptamers have the following advantages: capable of being screened without using animals, capable of being artificially synthesized and/or modified easily and at low cost, highly thermally stable and excellent for storing, having low immunogenicity, and the like, compared to antibodies. Further, while it is difficult to develop neutralizing agents that is administered in a case where an antibody or a small molecule causes side effects, aptamers can be easily controlled by adding a complementary strand or a small molecule.

Representative thrombin nucleic acid aptamers known to date are shown below.

TBA15 (also known as HD1: 5'-GGTTGGTGTGGTTGG-3': SEQ ID NO: 1) is a 15-mer thrombin aptamer and has a guanine quadruplex structure with two TT loops and a TGT motif sandwiched therebetween. TBA15 is known to bind to and inactivate exosite I of thrombin, and its clinical trial as an anticoagulant (ARC183) was conducted during coronary artery bypass surgery. The administration of TBA15 provided anticoagulant activity, however, the amount of drug required to obtain a desirable anticoagulant activity was inappropriate, resulting in discontinuation of the clinical trial after the completion of Phase I (Non Patent Literature 1).

NU172 (5'-CGCCTAGGTTGGGTAGGGTGGTGGCG-3': SEQ ID NO: 2) is a 26-mer thrombin aptamer that binds to exosite I. This aptamer was developed as an alternative to TBA15 for the purpose of developing an anticoagulant. Similarly to TBA15, NU172 is known to inhibit blood coagulation, and its clinical trial as an anticoagulant (ARC2172) has been conducted during coronary artery bypass surgery and is currently in Phase II.

TBA29 (also known as HD22: 5'-AGTCCGTGGTAGGGCAGGTTGGGGTGACT-3': SEQ ID NO: 3) is a 29-mer thrombin aptamer and has a mixed structure of a duplex and a guanine quadruplex. It has high binding capacity; however, since it binds to exosite II, it has almost no anticoagulant activity.

The inventors of the present application had developed a 43-mer thrombin aptamer called M08s (5'-AGGTCAGATGATGGGGATGGGGGGTTGGAGGAATGGATGACCT-3': SEQ ID NO: 14; hereinafter, also referred to as M08s (43) and M08s-1(43)) but reported that further shortening its length while maintaining its activity was difficult (Non Patent Literature 5).

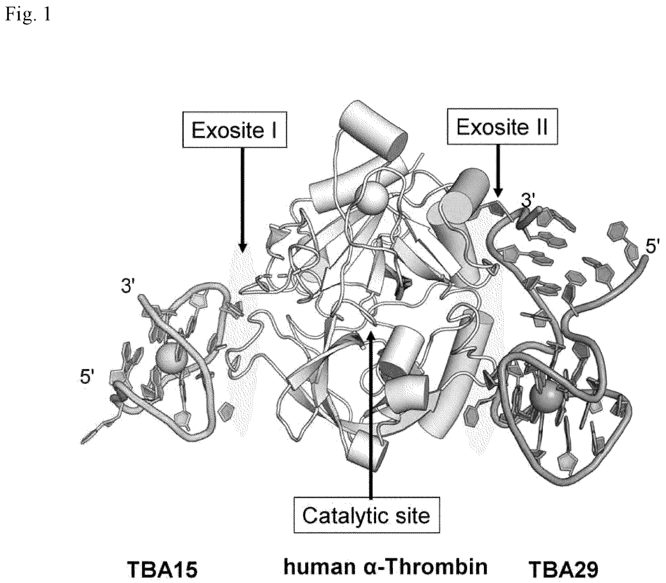

Thrombin aptamers are classified into two types according to their binding sited (exosite I and exosite II) (Fig. 1), and it has been known that an aptamer that binds to exosite I and an aptamer that binds to exosite II are linked by a linker to create one molecule of a bivalent nucleic acid aptamer. In this case, it has been known that a single-stranded polyA or polyT linker, which has a flexible structure and is advantageous in binding to thrombin, was optimal (Non Patent Literature 2).

The inventors of the present application had also prepared one molecule of a bivalent nucleic acid aptamer by linking an aptamer that binds to exosite I and an aptamer that binds to exosite II by a single-stranded polyA or polyT linker (Patent Literature 1 and Non Patent Literature 4).

### Citation List

### Non Patent Literature

Non Patent Literature 1: A. Schwienhorst, Cell. Mol. Life Sci. Vol.63, (2006) 2773-2791
Non Patent Literature 2: Jens Muller et al., ChemBioChem 2007,8, 2223-2226
Non Patent Literature 3: OSEPH N. ZADEH et al., J Comput Chem 32: 170-173, 2011
Non Patent Literature 4: Toru Yoshitomi et al., Res Pract Thromb Haemost. 2021;5:e12503.
Non Patent Literature 5: Koji Wakui et al., Molecular Therapy: Nucleic Acids Vol. 16 348-359, 2019

### Patent Literature

Patent Literature 1: International Publication No. WO2019/146676 pamphlet

### Summary of Invention

### Technical Problem

The present invention provides a monovalent nucleic acid aptamer, a bivalent nucleic acid aptamer, and a blood coagulation inhibitor containing them, and a neutralizing agent for them.

### Solution to Problem

The inventors of the present application have succeeded in newly creating 1) a modified product of M08s, and 2) a bivalent nucleic acid aptamer which are more suitable for medicine.

Further, the inventors of the present application have newly found that formation of a bivalent nucleic acid aptamer using, as a linker, a highly fixed and highly linear double stranded DNA structure, which has a double helix structure, rather than a single-stranded structure, which has a flexible structure, results in a superior ability to inhibit blood coagulation, and have completed the invention of the present application.

The present invention includes the following:
[A1] A DNA aptamer containing the following primary structure: 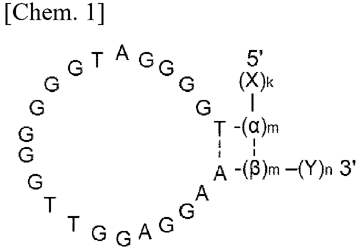 wherein
k, m, and n represent the numbers of DNA bases;
k is 0, 1, 2, or 3;
m is an integer selected from the group consisting of 1 to 15;
n is 0, 1, 2, or 3;
X and Y are each independently any bases;
α and β are bases; and
5'-(α)ₘ-3' and 5'-(β)ₘ-3' form an antiparallel double-stranded structure.

[A2] The DNA aptamer according to [A1], containing the following primary structure: 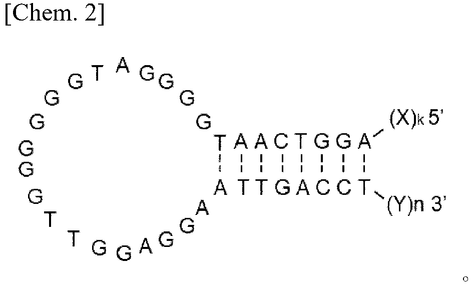
[A3] The DNA aptamer according to [A1], containing M08s-1 (36) (SEQ ID NO: 107).
[0] A bivalent DNA aptamer comprising
two DNA aptamers that bind to exosite I of thrombin;
two DNA aptamers that bind to exosite II; or
a DNA aptamer that binds to exosite I of thrombin and a DNA aptamer that binds to exosite II,
wherein the DNA aptamers are linked to each other via duplex DNA.
[1] The bivalent DNA aptamer according to [0], wherein the bivalent DNA aptamer is in a single-stranded form and contains any of the following primary structures:
a-1) 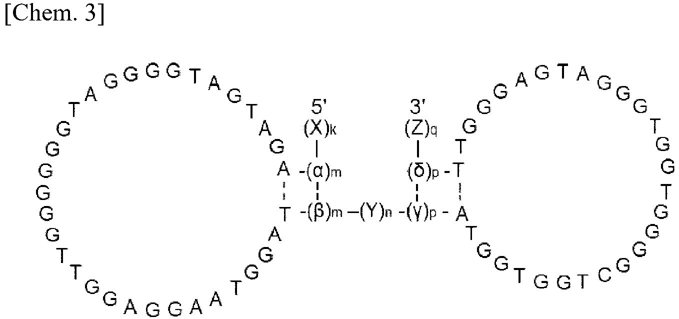
a-2) 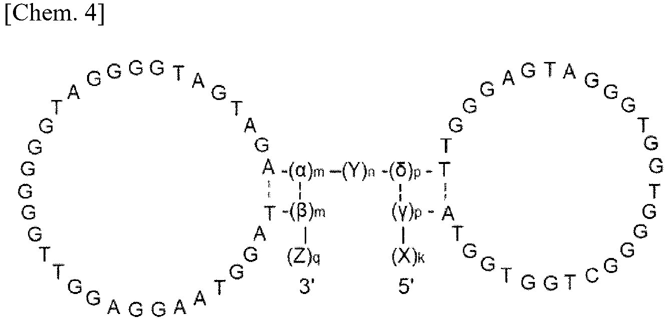 wherein
   k, m, n, p, and q represent the numbers of DNA bases;
   k is 0, 1, 2, or 3;
   m is an integer selected from the group consisting of 1 to 15;
   n is 0, 1, 2, 3, 4, or 5;
   p is an integer selected from the group consisting of 1 to 17;
   q is 0, 1, 2, or 3;
   X, Y, and Z are each independently any bases;
   α, β, γ, and δ are bases;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[2] The bivalent DNA aptamer according to [0], wherein the bivalent DNA aptamer is in a single-stranded form and contains any of the following primary structures:
a1-1) 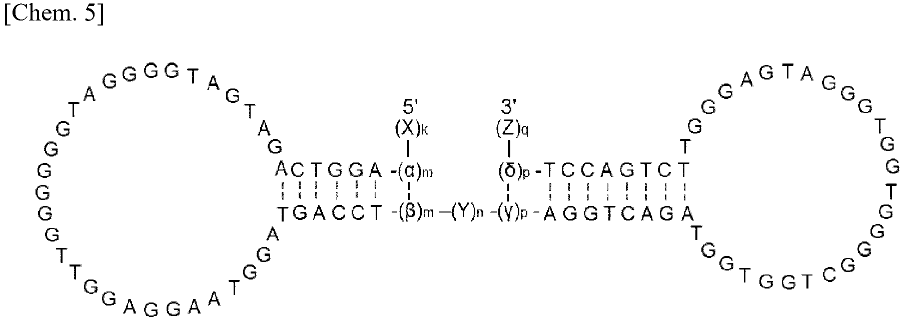
a1-2) 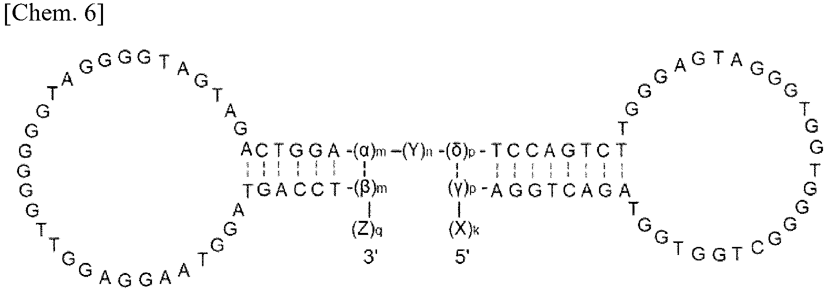 wherein
   k, m, n, p, and q represent the numbers of DNA bases;
   k is 0, 1, 2, or 3;
   m is an integer selected from the group consisting of 1 to 10;
   n is 0, 1, 2, 3, 4, or 5;
   p is an integer selected from the group consisting of 1 to 10;
   q is 0, 1, 2, or 3;
   X, Y, and Z are each independently any bases;
   α, β, γ, and δ are bases;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[3] The bivalent DNA aptamer according to [0], wherein the bivalent DNA aptamer is in a single-stranded form and contains any of the following primary structures:
a2-1) 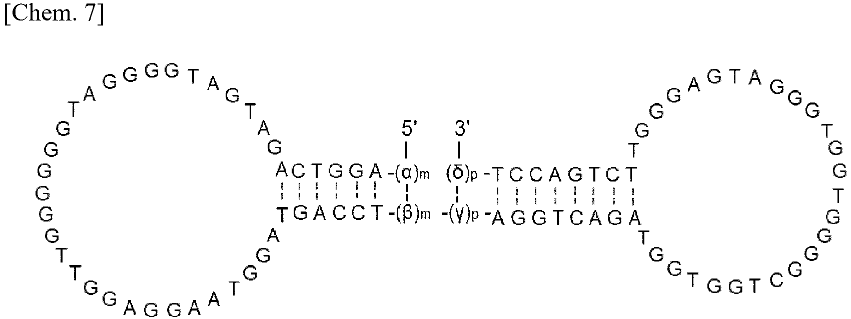
a2-2) 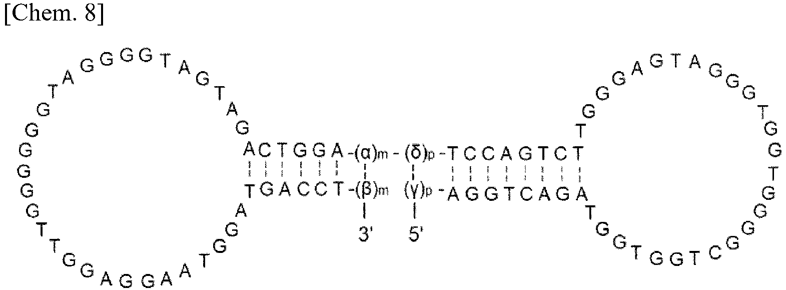 wherein
   m and p represent the numbers of DNA bases;
   m is an integer selected from the group consisting of 1 to 10;
   p is an integer selected from the group consisting of 1 to 10;
   α, β, γ, and δ are bases;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[4] The bivalent DNA aptamer according to [0], wherein the bivalent DNA aptamer is in a single-stranded form and contains any of the following primary structures:
b-1) 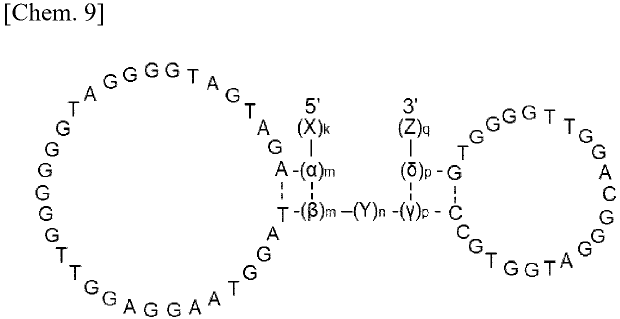
b-2) 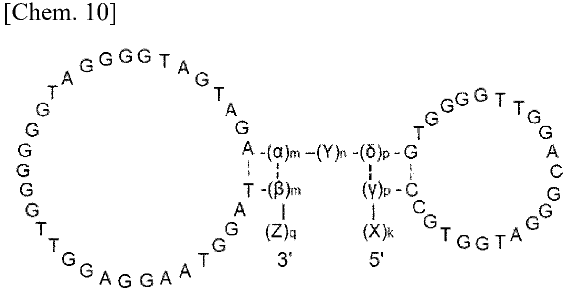 wherein
   k, m, n, p, and q represent the numbers of DNA bases;
   k is 0, 1, 2, or 3;
   m is an integer selected from the group consisting of 1 to 15;
   n is 0, 1, 2, 3, 4, or 5;
   p is an integer selected from the group consisting of 1 to 13;
   q is 0, 1, 2, or 3;
   X, Y, and Z are each independently any bases;
   α, β, γ, and δ are bases;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[5] The bivalent DNA aptamer according to [0], wherein the bivalent DNA aptamer is in a single-stranded form and contains any of the following primary structures:
b1-1) 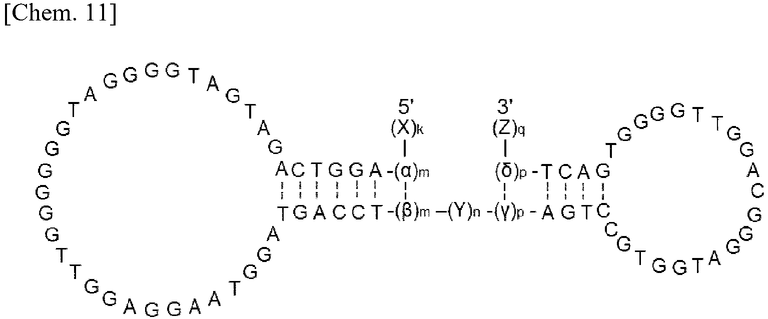
b1-2) 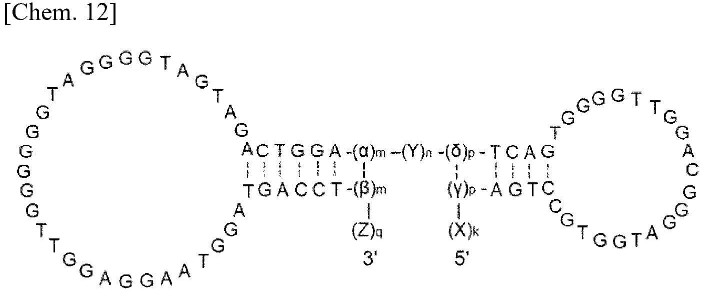 wherein
   k, m, n, p, and q represent the numbers of DNA bases;
   k is 0, 1, 2, or 3;
   m is an integer selected from the group consisting of 1 to 10;
   n is 0, 1, 2, 3, 4, or 5;
   p is an integer selected from the group consisting of 1 to 10;
   q is 0, 1, 2, or 3;
   X, Y, and Z are each independently any bases;
   α, β, γ, and δ are bases;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[6] The bivalent DNA aptamer according to [0], wherein the bivalent DNA aptamer is in a single-stranded form and contains any of the following primary structures:
b2-1) 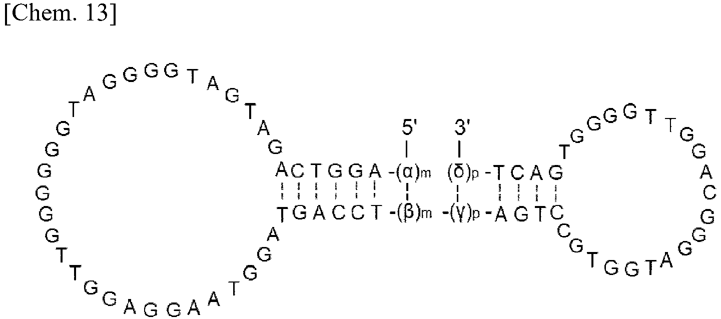
b2-2) 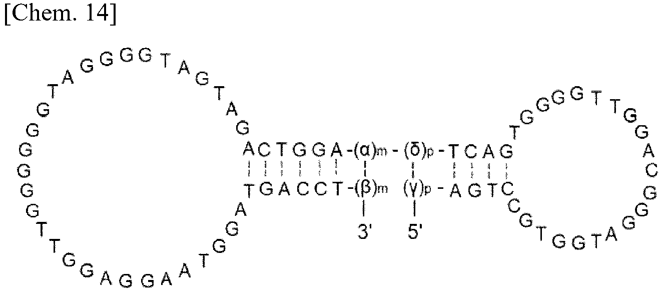 wherein
   m and p represent the numbers of DNA bases;
   m is an integer selected from the group consisting of 1 to 10;
   p is an integer selected from the group consisting of 1 to 10;
   α, β, γ, and δ are bases;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[7] The bivalent DNA aptamer according to [0], wherein the bivalent DNA aptamer is in a single-stranded form and contains the following primary structure:
c) 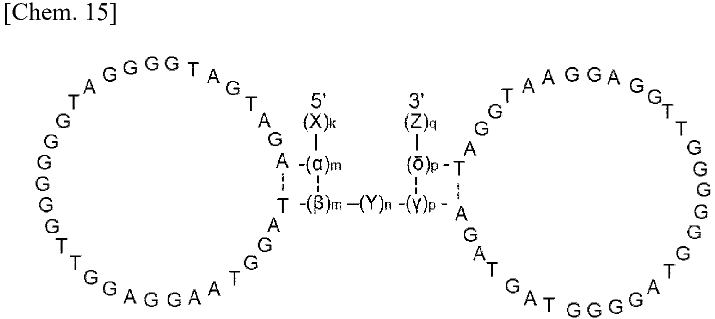 wherein
k, m, n, p, and q represent the numbers of DNA bases;
k is 0, 1, 2, or 3;
m is an integer selected from the group consisting of 1 to 15;
n is 0, 1, 2, 3, 4, or 5;
p is an integer selected from the group consisting of 1 to 15;
q is 0, 1, 2, or 3;
X, Y, and Z are each independently any bases;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

[8] The bivalent DNA aptamer according to [0], wherein the bivalent DNA aptamer is in a single-stranded form and contains the following primary structure:
c1) 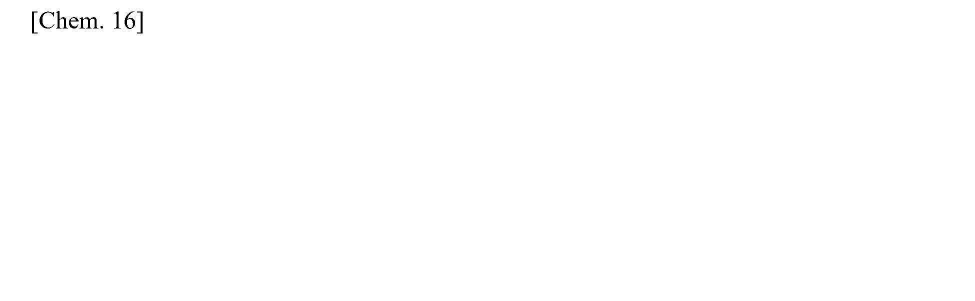 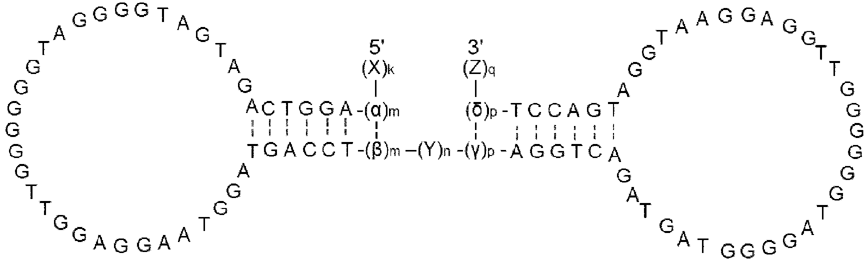 wherein
k, m, n, p, and q represent the numbers of DNA bases;
k is 0, 1, 2, or 3;
m is an integer selected from the group consisting of 1 to 10;
n is 0, 1, 2, 3, 4, or 5;
p is an integer selected from the group consisting of 1 to 10;
q is 0, 1, 2, or 3;
X, Y, and Z are each independently any bases;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

[9] The bivalent DNA aptamer according to [0], wherein the bivalent DNA aptamer is in a single-stranded form and contains the following primary structure:
c2) 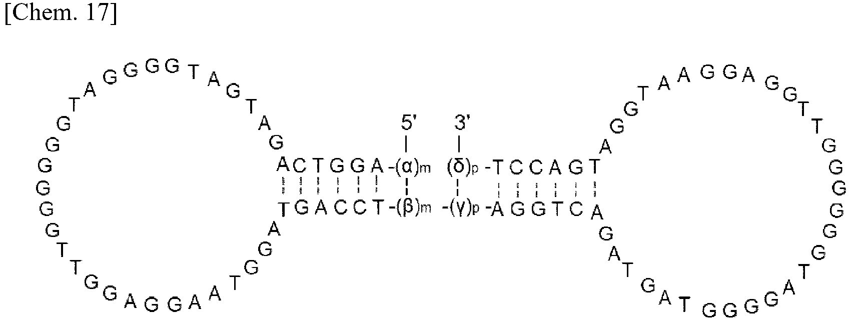 wherein
m and p represent the numbers of DNA bases;
m is an integer selected from the group consisting of 1 to 10;
p is an integer selected from the group consisting of 1 to 10;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

[B1] The bivalent DNA aptamer according to [0], wherein the bivalent DNA aptamer is in a single-stranded form and contains any one of the following primary structures:
d-1) 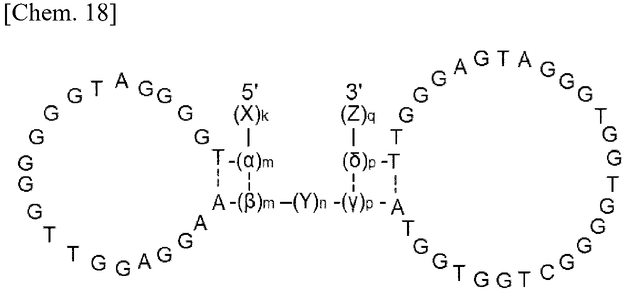
d-2) 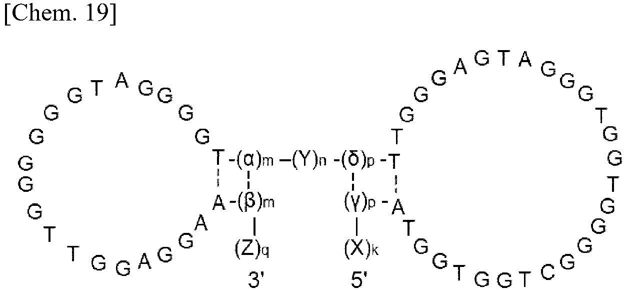
e-1) 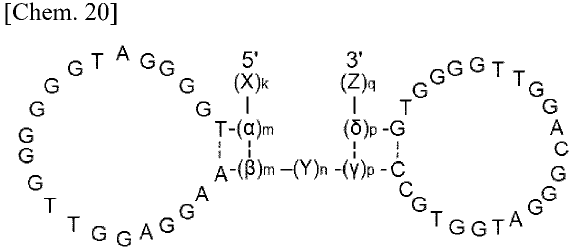
e-2) 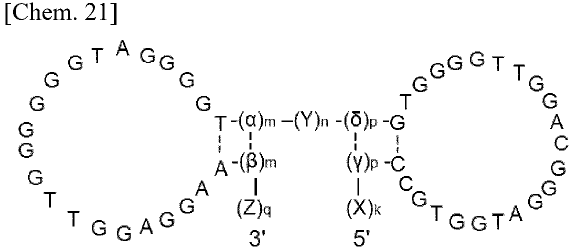
f) 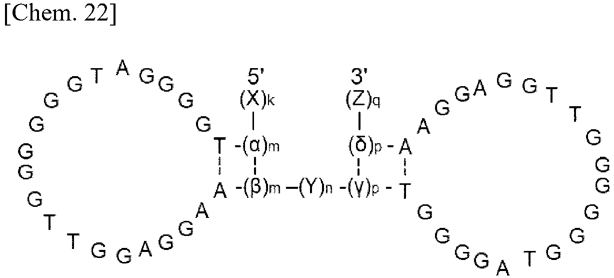 wherein
   k, m, n, p, and q represent the numbers of DNA bases;
   k is 0, 1, 2, or 3;
   m is an integer selected from the group consisting of 1 to 10;
   n is 0, 1, 2, 3, 4, or 5;
   p is an integer selected from the group consisting of 1 to 10;
   q is 0, 1, 2, or 3;
   X, Y, and Z are each independently any bases;
   α, β, γ, and δ are bases;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[C1] The bivalent DNA aptamer according to [0], wherein the bivalent DNA aptamer is in a single-stranded form and contains any one of the following primary structures:
d1-1) 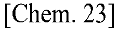 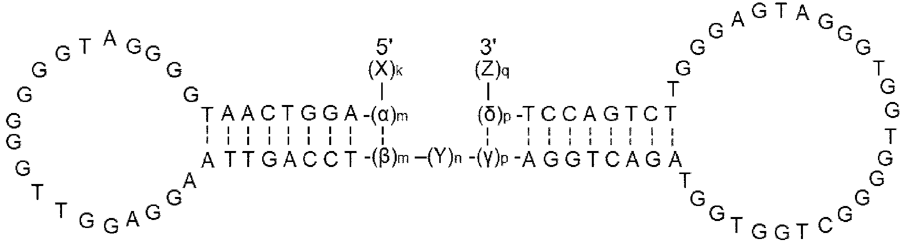
d1-2) 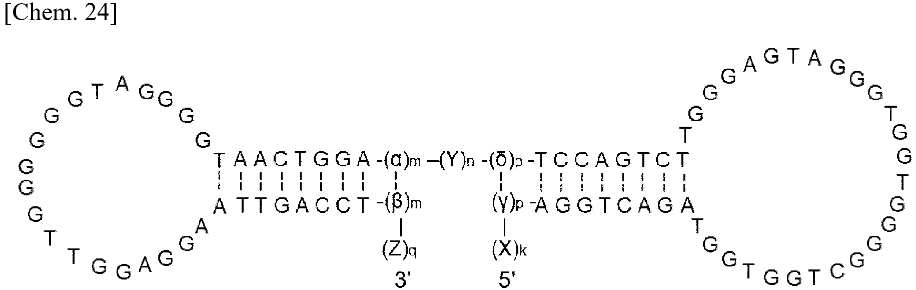
e1-1) 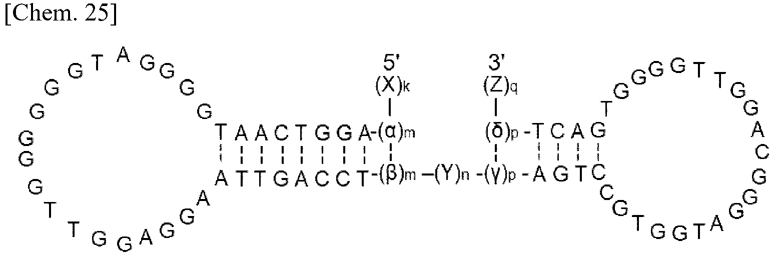
e1-2) 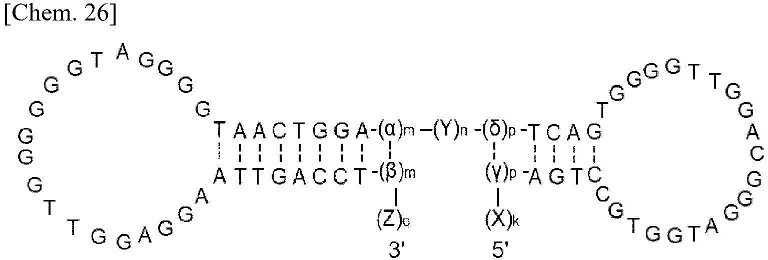
f1) 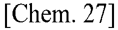 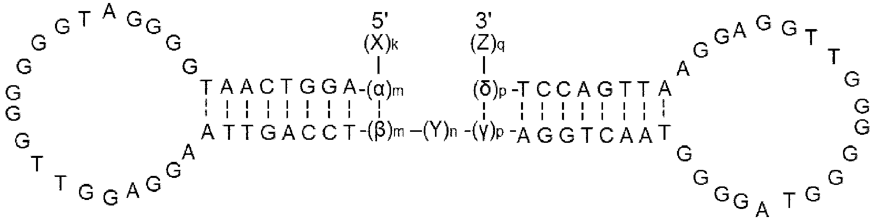 wherein
   k, m, n, p, and q represent the numbers of DNA bases;
   k is 0, 1, 2, or 3;
   m is an integer selected from the group consisting of 1 to 10;
   n is 0, 1, 2, 3, 4, or 5;
   p is an integer selected from the group consisting of 1 to 10;
   q is 0, 1, 2, or 3;
   X, Y, and Z are each independently any bases;
   α, β, γ, and δ are bases;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[D1] The bivalent DNA aptamer according to [0], wherein the bivalent DNA aptamer is in a single-stranded form and contains any one of the following primary structures:
d2-1) 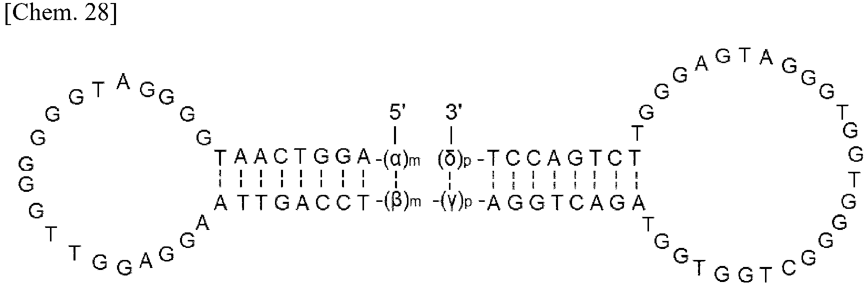
d2-2) 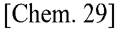 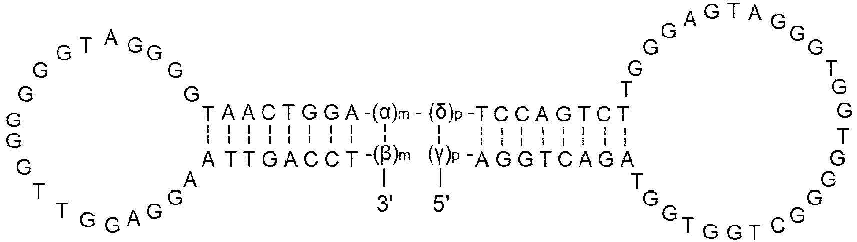
e2-1) 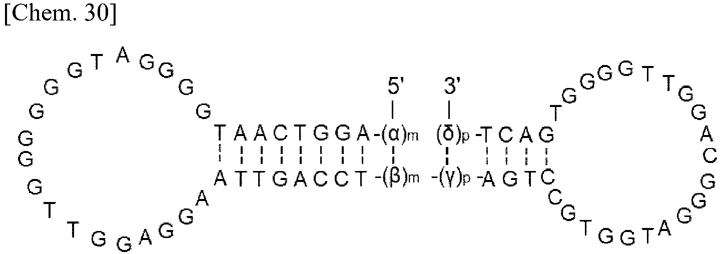
e2-2) 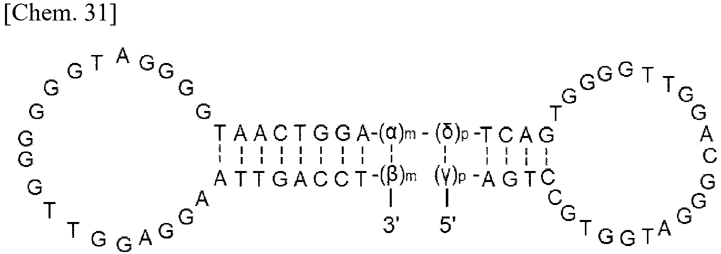
f2) 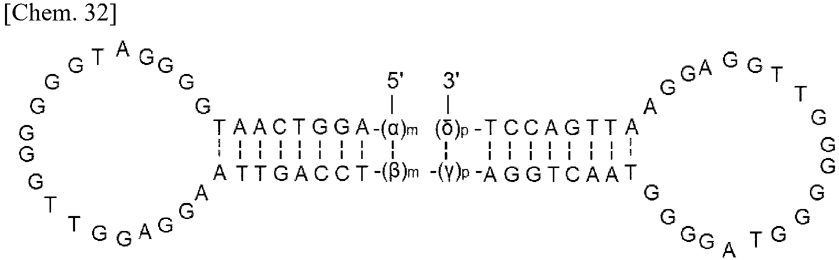 wherein
   m and p represent the numbers of DNA bases;
   m is an integer selected from the group consisting of 1 to 10;
   p is an integer selected from the group consisting of 1 to 10;
   α, β, γ, and δ are bases;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[10] The single-stranded bivalent DNA aptamer according to any one of [1] to [D1] above, wherein
each set of α and β is bases that form a base pair; and/or
each set of γ and δ is bases that form a base pair.

[11] The single-stranded bivalent DNA aptamer according to any one of [1] to [D1] above, wherein m + p equals 5 to 21.
[12] The single-stranded bivalent DNA aptamer according to any one of [1] to [D1] above, wherein m + p equals 5 to 17.
[13] The single-stranded bivalent DNA aptamer according to [0], comprising the sequence of pse (M08s-M03s) (SEQ ID NO: 12).
[14] The single-stranded bivalent DNA aptamer according to [0], comprising the sequence of pse (M08s-TBA29) (SEQ ID NO: 13).
[15] The single-stranded bivalent DNA aptamer according to [0], comprising the sequence of pse (M08s-M08s) (SEQ ID NO: 22).
[E1] The single-stranded bivalent DNA aptamer according to [0], comprising the sequence of M08s-1 (36) (SEQ ID NO: 107) and the sequence of TBA29 (SEQ ID NO: 3).
[E2] The single-stranded bivalent DNA aptamer according to [0], comprising a sequence selected from the group consisting of Pse08-29 ss1-TTACG (70) (SEQ ID NO: 108); Pse08-29 ss1-5A (70) (SEQ ID NO: 109); Pse08-29 ss1-4A (69) (SEQ ID NO: 110); Pse08-29 ss1-2A (67) (SEQ ID NO: 111); Pse08-29 ss1-5T (70) (SEQ ID NO: 112); Pse08-29 ss1-4T (69) (SEQ ID NO: 113); Pse08-29 ss1-2T (67) (SEQ ID NO: 114); Pse08-29 ss1-0A (65) (SEQ ID NO: 115); Pse08-29 ss1-0Aa (65) (SEQ ID NO: 116); Pse08-29 ss1-0Ab (65) (SEQ ID NO: 117); Pse08-29 ss1-0Ac (65) (SEQ ID NO: 118); Pse08-29 ss2-CGTAA (70) (SEQ ID NO: 119); Pse08-29 ss2-5A (70) (SEQ ID NO: 120); Pse08-29 ss2-4A (69) (SEQ ID NO: 121); Pse08-29 ss2-2A (67) (SEQ ID NO: 122); Pse08-29 ss2-5T (70) (SEQ ID NO: 123); Pse08-29 ss2-4T (69) (SEQ ID NO: 124); Pse08-29 ss2-2T (67) (SEQ ID NO: 125); Pse08-29 ss2-0B (65) (SEQ ID NO: 126); Pse08-29 ss2-0Ba (65) (SEQ ID NO: 127); Pse08-29 ss2-0Bb (65) (SEQ ID NO: 127); and Pse08-29 ss2-0Bc (65) (SEQ ID NO: 129).
[E3] The single-stranded bivalent DNA aptamer according to [0], comprising two copies of the sequence of M08s-1 (36) (SEQ ID NO: 107).
[E4] The single-stranded bivalent DNA aptamer according to [0], comprising a sequence selected from the group consisting of Pse08-08 (72) (SEQ ID NO: 130); Pse08-08 (68) (SEQ ID NO: 131); Pse08-08 (64) (SEQ ID NO: 132); Pse08-08 (60) (SEQ ID NO: 133); Pse08-08 (72-GC) (SEQ ID NO: 134); and Pse08-08 (60-GC) (SEQ ID NO: 135).
[1A] The bivalent DNA aptamer according to [0], wherein the bivalent DNA aptamer is in the following double-stranded form and consists of:
an oligonucleotide A comprising the sequence of 5'-(α)ₘ-AGATGATGGGGATGGGGGGTTGGAGGAATGGAT-(β)ₘ-(X)ₙ-3', and
an oligonucleotide B comprising the sequence of 5'-(γ)ₚ-ATGGTGGTCGGGGTGGTGGGATGAGGGTTCTGACCT-(δ)ₚ-(Y)ₙ-3':
   aa) 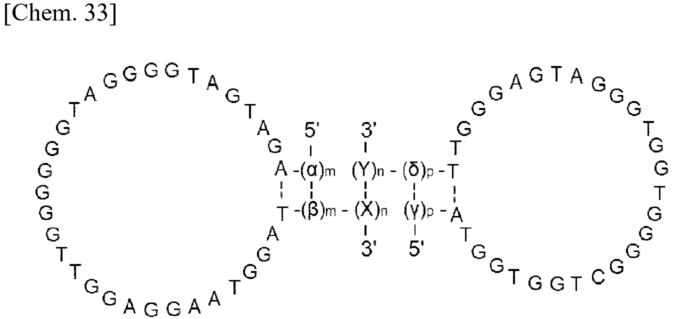 wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 27;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[2A] The bivalent DNA aptamer according to [0], wherein the bivalent DNA aptamer is in the following double-stranded form and consists of:
an oligonucleotide A comprising the sequence of 5'-(α)ₘ-AGGTCAGATGATGGGGATGGGGGGTTGGAGGAATGGATGACCT-(β)ₘ-(X)ₙ-3', and
an oligonucleotide B comprising the sequence of 5'-(γ)ₚ-AGGTCAGATGGTGGTCGGGGTGGTGGGATGAGGGTTCTGACCT-(δ)ₘ-(Y)ₙ-3':
   aa1) 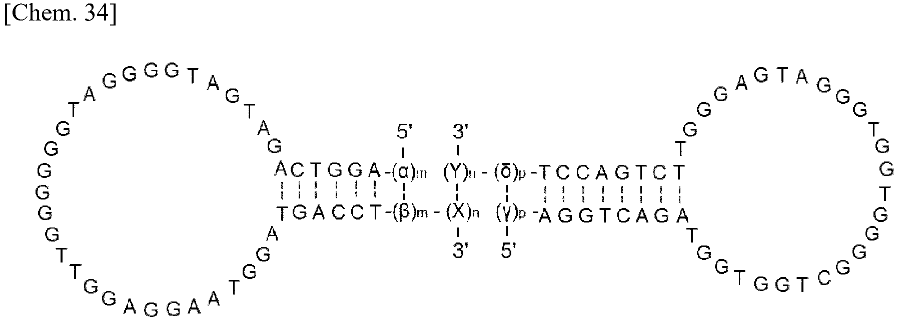 wherein
   m, n, and p represent the numbers of DNA bases;
   m is 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[3A] The bivalent DNA aptamer according to [0], wherein the bivalent DNA aptamer is in the following double-stranded form and consists of:
an oligonucleotide A comprising the sequence of 5'-(Y)ₙ-(α)ₘ-AGATGATGGGGATGGGGGGTTGGAGGAATGGAT-(β)ₘ-3', and
an oligonucleotide B comprising the sequence of 5'-(X)ₙ-(γ)ₚ-ATGGTGGTCGGGGTGGTGGGATGAGGGTT-(δ)ₚ-3':
   bb) 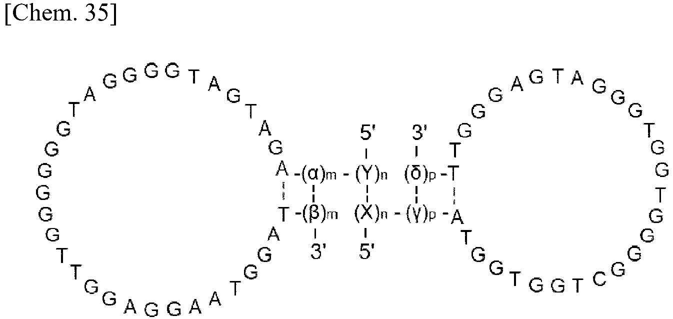 wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 27;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.
[4A]
The bivalent DNA aptamer according to [0], wherein the bivalent DNA aptamer is in the following double-stranded form and consists of:
an oligonucleotide A comprising the sequence of 5'-(Y)ₙ-(α)ₘ-AGGTCAGATGATGGGGATGGGGGGTTGGAGGAATGGATGACCT-(β)ₘ-3', and
an oligonucleotide B comprising the sequence of 5'-(X)ₙ-(γ)ₚ-AGGTCAGATGGTGGTCGGGGTGGTGGGATGAGGGTTCTGACCT-(δ)ₚ-3':
   bb1) 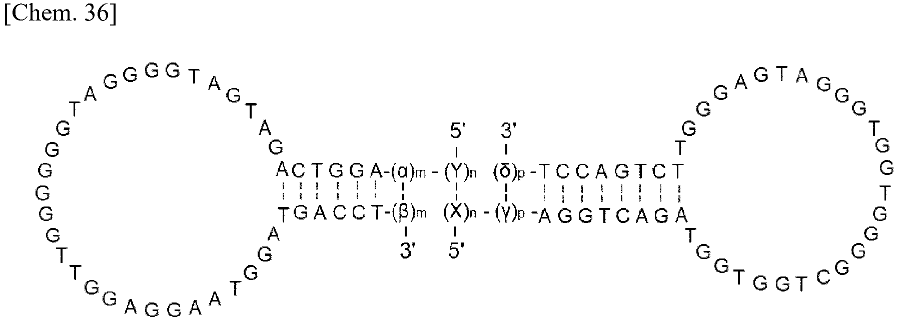 wherein
   m, n, and p represent the numbers of DNA bases;
   m is an integer selected from the group consisting of 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[5A] The bivalent DNA aptamer according to [0], wherein the bivalent DNA aptamer is in the following double-stranded form and consists of:
an oligonucleotide A comprising the sequence of 5'-(α)ₘ-AGATGATGGGGATGGGGGGTTGGAGGAATGGAT-(β)ₘ-(X)ₙ-3', and
an oligonucleotide B comprising the sequence of 5'-(γ)ₚ-CCGTGGTAGGGCAGGTTGGGGTG-(δ)ₘ-(Y)ₙ-3':
   cc) 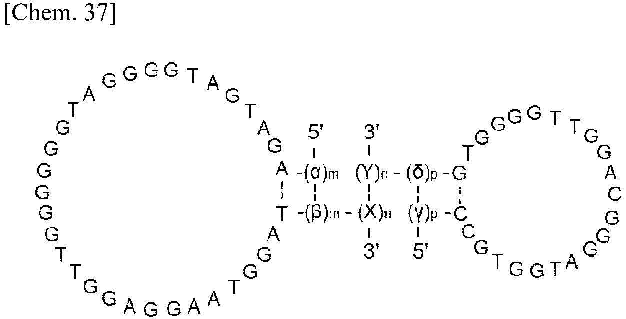 wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 23;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[6A] The bivalent DNA aptamer according to [0], wherein the bivalent DNA aptamer is in the following double-stranded form and consists of:
an oligonucleotide A comprising the sequence of 5'-(α)ₘ-AGGTCAGATGATGGGGATGGGGGGTTGGAGGAATGGATGACCT-(β)ₘ-(X)ₙ-3', and
an oligonucleotide B comprising the sequence of 5'-(γ)ₚ-AGTCCGTGGTAGGGCAGGTTGGGGTGACT-(δ)ₚ-(Y)ₙ-3':
   cc1) 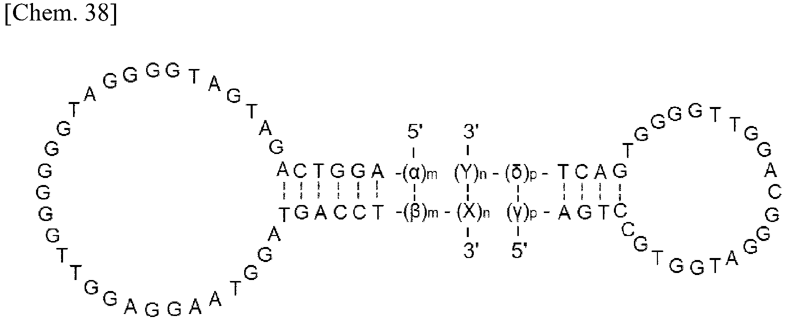 wherein
   m, n, and p represent the numbers of DNA bases;
   m is 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[7A] The bivalent DNA aptamer according to [0], wherein the bivalent DNA aptamer is in the following double-stranded form and consists of:
an oligonucleotide A comprising the sequence of 5'-(Y)ₙ-(α)ₘ-AGGTCAGATGATGGGGATGGGGGGTTGGAGGAATGGATGACCT-(β)ₘ-(X)ₙ-3', and
an oligonucleotide B comprising the sequence of 5'-(X)ₙ-(γ)ₚ-AGTCCGTGGTAGGGCAGGTTGGGGTGACT-(δ)ₚ-3':
   dd) 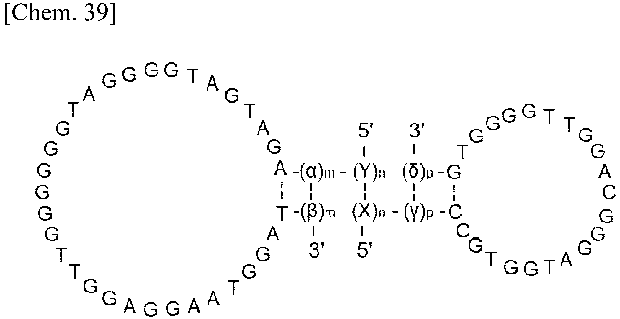 wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 23;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[8A] The bivalent DNA aptamer according to [0], wherein the bivalent DNA aptamer is in the following double-stranded form and consists of:
an oligonucleotide A comprising the sequence of 5'-(Y)ₙ-(α)ₘ-AGGTCAGATGATGGGGATGGGGGGTTGGAGGAATGGATGACCT-(β)ₘ-(X)ₙ-3', and
an oligonucleotide B comprising the sequence of 5'-(X)ₙ-(γ)ₚ-AGTCCGTGGTAGGGCAGGTTGGGGTGACT-(δ)ₚ-3':
   dd1) 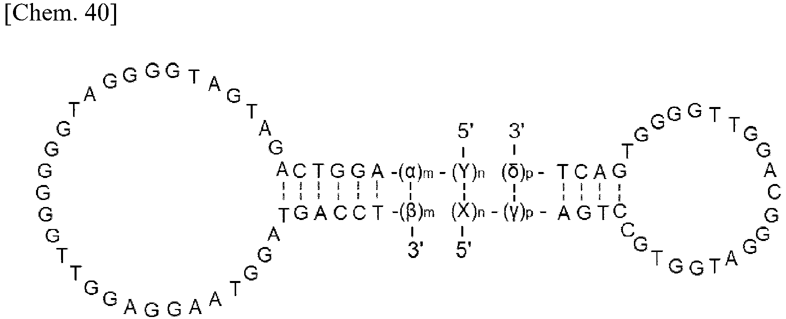 wherein
   m, n, and p represent the numbers of DNA bases;
   m is 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[9A] The bivalent DNA aptamer according to [0], wherein the bivalent DNA aptamer is in the following double-stranded form and consists of:
an oligonucleotide A comprising the sequence of 5'-(α)ₘ-AGATGATGGGGATGGGGGGTTGGAGGAATGGAT-(β)ₘ-(X)ₙ-3', and
an oligonucleotide B comprising the sequence of 5'-(γ)ₚ-AGATGATGGGGATGGGGGGTTGGAGGAATGGAT-(δ)ₚ-(Y)ₙ-3':
   ee) 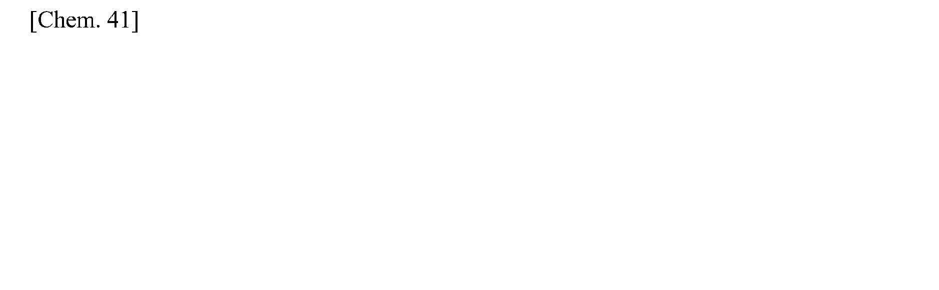 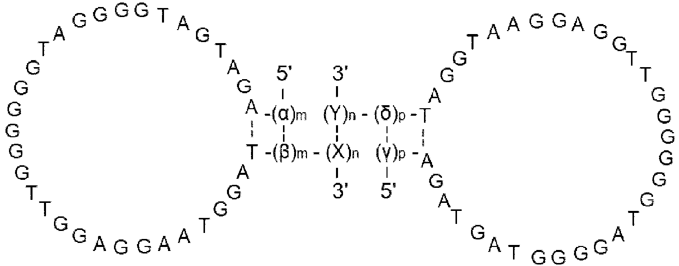 wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 25;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[10A] The bivalent DNA aptamer according to [0], wherein the bivalent DNA aptamer is in the following double-stranded form and consists of:
an oligonucleotide A comprising the sequence of 5'-(α)ₘ-AGGTCAGATGATGGGGATGGGGGGTTGGAGGAATGGATGACCT-(β)ₘ-(X)ₙ-3', and
an oligonucleotide B comprising the sequence of 5'-(γ)ₚ-AGGTCAGATGATGGGGATGGGGGGTTGGAGGAATGGATGACCT-(δ)ₚ-(Y)ₙ-3':
   ee1) 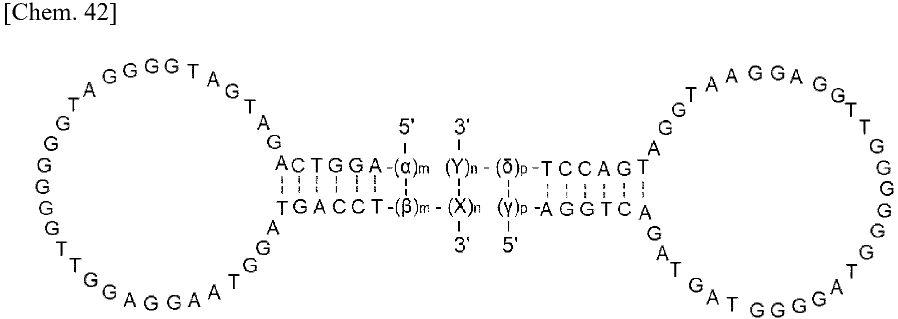 wherein
   m, n, and p represent the numbers of DNA bases;
   m is 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[11A] The bivalent DNA aptamer according to [0], wherein the DNA bivalent aptamer is in the following double-stranded form and consists of:
an oligonucleotide A comprising the sequence of 5'-(Y)ₙ-(α)ₘ-AGATGATGGGGATGGGGGGTTGGAGGAATGGAT-(β)ₘ-3', and
an oligonucleotide B comprising the sequence of 5'-(X)ₙ-(γ)ₚ-AGATGATGGGGATGGGGGGTTGGAGGAATGGAT-(δ)ₚ-3':
   ff) 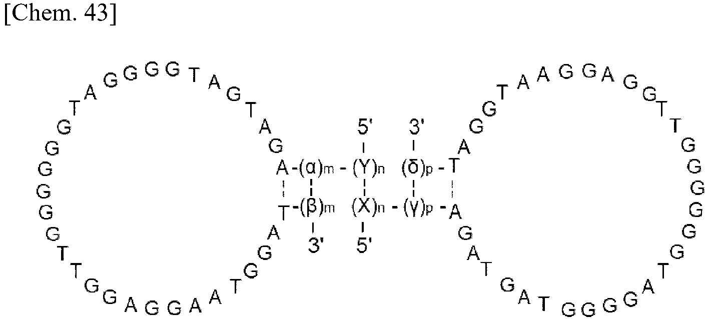 wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 25;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[12A] The bivalent DNA aptamer according to [0], wherein the bivalent DNA aptamer is in the following double-stranded form and consists of:
an oligonucleotide A comprising the sequence of 5'-(Y)ₙ-(α)ₘ-AGGTCAGATGATGGGGATGGGGGGTTGGAGGAATGGATGACCT-(β)ₘ-3', and
an oligonucleotide B comprising the sequence of 5'-(X)ₙ-(γ)ₚ-AGGTCAGATGGTGGTCGGGGTGGTGGGATGAGGGTTCTGACCT-(δ)ₚ-3'.
   ff1) 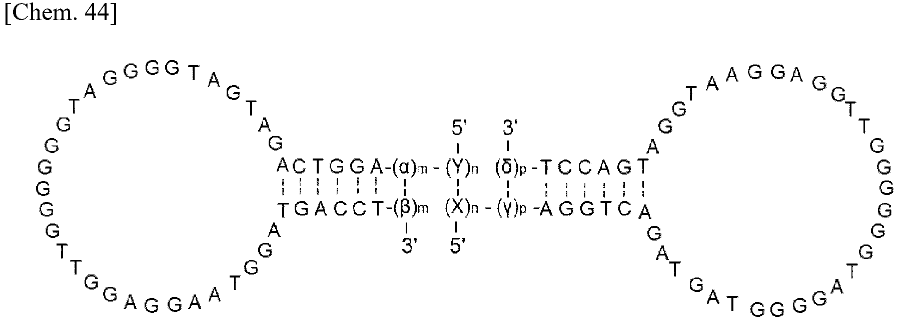 wherein
   m, n, and p represent the numbers of DNA bases;
   m is an integer selected from the group consisting of 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[F1] The bivalent DNA aptamer according to [0], wherein the bivalent DNA aptamer is in the following double-stranded form and consists of:
an oligonucleotide A comprising the sequence of 5'-(α)ₘ-TGGGGATGGGGGGTTGGAGGAA-(β)ₘ-(X)ₙ-3', and
an oligonucleotide B comprising the sequence of 5'-(γ)ₚ-ATGGTGGTCGGGGTGGTGGGATGAGGGTT-(δ)ₚ-(Y)ₙ-3':
   AA) 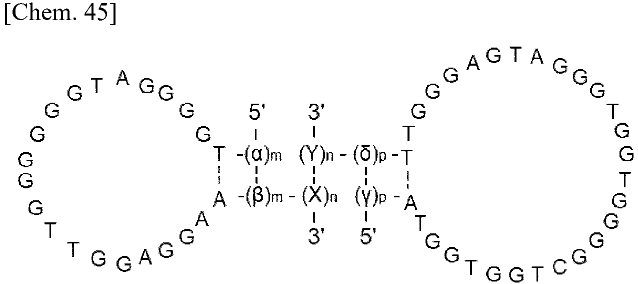 wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 27;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[F2] The bivalent DNA aptamer according to [0], wherein the bivalent DNA aptamer is in the following double-stranded form and consists of:
an oligonucleotide A comprising the sequence of 5'-(α)ₘ-AGGTCAATGGGGATGGGGGGTTGGAGGAATTGACCT-(β)ₘ-(X)ₙ-3', and
an oligonucleotide B comprising the sequence of 5'-(γ)ₚ-AGGTCAGATGGTGGTCGGGGTGGTGGGATGAGGGTTCTGACCT-(δ)ₚ-(Y)ₙ-3':
   AA1) 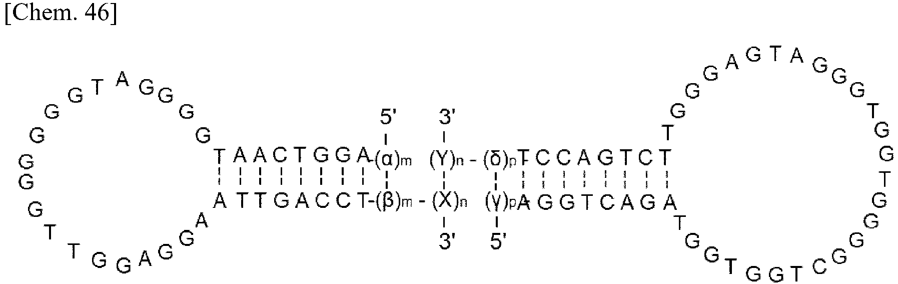 wherein
   m, n, and p represent the numbers of DNA bases;
   m is 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[F3] The bivalent DNA aptamer according to [0], wherein the bivalent DNA aptamer is in the following double-stranded form and consists of:
an oligonucleotide A comprising the sequence of 5'-(Y)ₙ-(α)ₘ-TGGGGATGGGGGGTTGGAGGAA-(β)ₘ-3', and
an oligonucleotide B comprising the sequence of 5'-(X)ₙ-(γ)ₚ-ATGGTGGTCGGGGTGGTGGGATGAGGGTT-(δ)ₚ-3':
   BB) 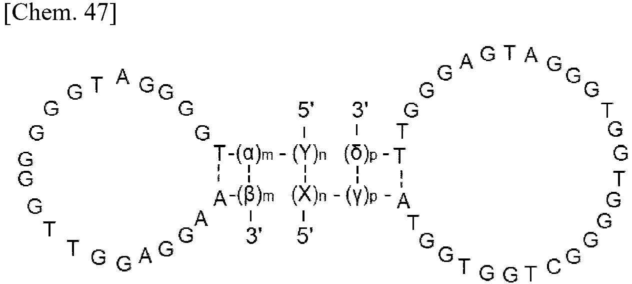 wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 27;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[F4] The bivalent DNA aptamer according to [0], wherein the bivalent DNA aptamer is in the following double-stranded form and consists of:
an oligonucleotide A comprising the sequence of 5'-(Y)ₙ-(α)ₘ-AGGTCAATGGGGATGGGGGGTTGGAGGAATTGACCT-(β)ₘ-3', and
an oligonucleotide B comprising the sequence of 5'-(X)ₙ-(γ)ₚ-AGGTCAGATGGTGGTCGGGGTGGTGGGATGAGGGTTCTGACCT-(δ)ₚ-3':
   BB1) 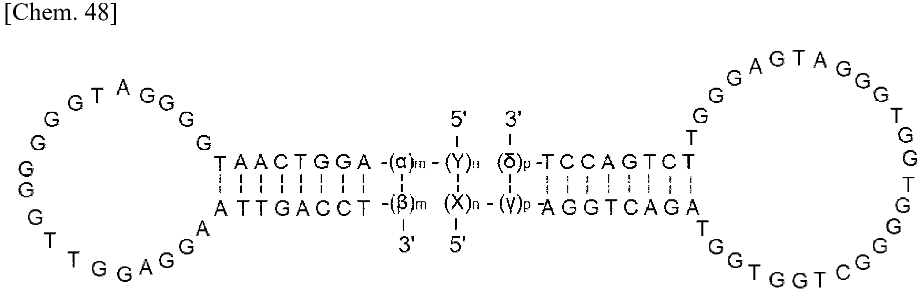 wherein
   m, n, and p represent the numbers of DNA bases;
   m is an integer selected from the group consisting of 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[F5] The bivalent DNA aptamer according to [0], wherein the bivalent DNA aptamer is in the following double-stranded form and consists of:
an oligonucleotide A comprising the sequence of 5'-(α)ₘ-TGGGGATGGGGGGTTGGAGGAA-(β)ₘ-(X)ₙ-3', and
an oligonucleotide B comprising the sequence of 5'-(γ)ₚ-CCGTGGTAGGGCAGGTTGGGGTG-(δ)ₚ-(Y)ₙ-3':
   CC) 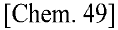 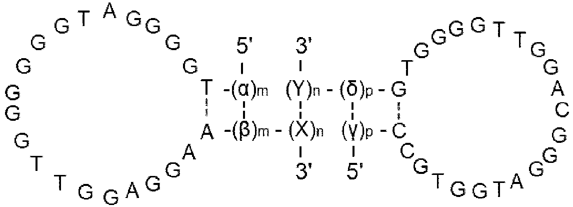 wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 23;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[F6] The bivalent DNA aptamer according to [0], wherein the bivalent DNA aptamer is in the following double-stranded form and consists of:
an oligonucleotide A comprising the sequence of 5'-(α)ₘ-AGGTCAATGGGGATGGGGGGTTGGAGGAATTGACCT-(β)ₘ-(X)ₙ-3', and
an oligonucleotide B comprising the sequence of 5'-(γ)ₚ-AGTCCGTGGTAGGGCAGGTTGGGGTGACT-(δ)ₚ-(Y)ₙ-3':
   CC1) 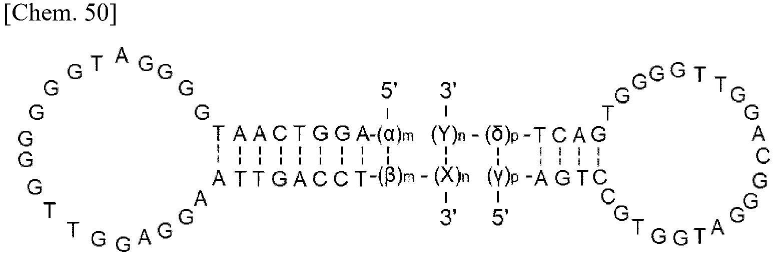 wherein
   m, n, and p represent the numbers of DNA bases;
   m is 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[F7] The bivalent DNA aptamer according to [0], wherein the bivalent DNA aptamer is in the following double-stranded form and consists of:
an oligonucleotide A comprising the sequence of 5'-(Y)ₙ-(α)ₘ-TGGGGATGGGGGGTTGGAGGAA-(β)ₘ-(X)ₙ-3', and
an oligonucleotide B comprising the sequence of 5'-(X)ₙ-(γ)ₚ-CGTGGTAGGGCAGGTTGGGGT-(δ)ₚ-3':
   DD) 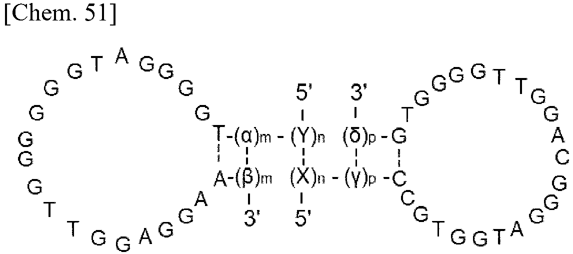 wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 23;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[F8] The bivalent DNA aptamer according to [0], wherein the bivalent DNA aptamer is in the following double-stranded form and consists of:
an oligonucleotide A comprising the sequence of 5'-(Y)ₙ-(α)ₘ-AGGTCAATGGGGATGGGGGGTTGGAGGAATTGACCT-(β)ₘ-(X)ₙ-3', and
an oligonucleotide B comprising the sequence of 5'-(X)ₙ- (γ)ₚ-AGTCCGTGGTAGGGCAGGTTGGGGTGACT-(δ)ₚ-3':
   DD1) 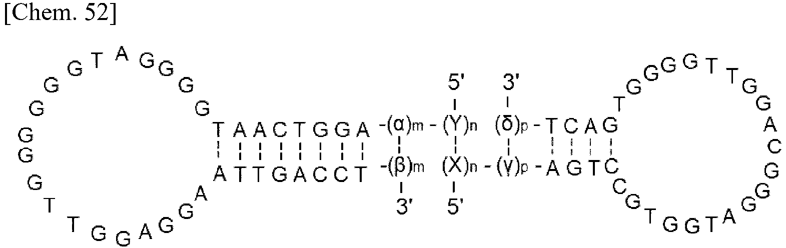 wherein
   m, n, and p represent the numbers of DNA bases;
   m is 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[F9] The bivalent DNA aptamer according to [0], wherein the bivalent DNA aptamer is in the following double-stranded form and consists of:
an oligonucleotide A comprising the sequence of 5'-(α)ₘ-TGGGGATGGGGGGTTGGAGGAA-(β)ₘ-(X)ₙ-3', and
an oligonucleotide B comprising the sequence of 5'-(γ)ₚ-TGGGGATGGGGGGTTGGAGGAA-(δ)ₚ-(Y)ₙ-3':
   EE) 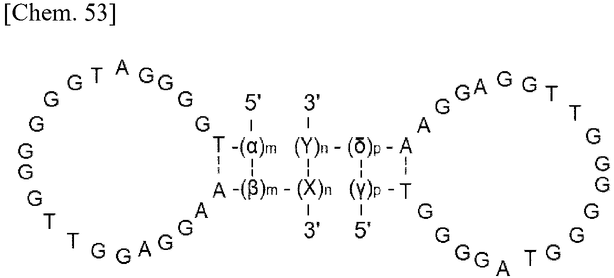 wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 25;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[F10] The bivalent DNA aptamer according to [0], wherein the bivalent DNA aptamer is in the following double-stranded form and consists of:
an oligonucleotide A comprising the sequence of 5'-(α)ₘ-AGGTCAATGGGGATGGGGGGTTGGAGGAATTGACCT-(β)ₘ-(X)ₙ-3', and
an oligonucleotide B comprising the sequence of 5'-(γ)ₚ-AGGTCAATGGGGATGGGGGGTTGGAGGAATTGACCT-(δ)ₚ-(Y)ₙ-3':
   EE1) 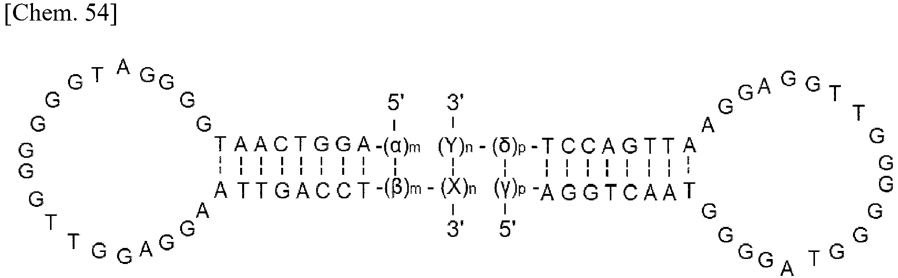 wherein
   m, n, and p represent the numbers of DNA bases;
   m is 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[F11] The bivalent DNA aptamer according to [0], wherein the bivalent DNA aptamer is in the following double-stranded form and consists of:
an oligonucleotide A comprising the sequence of 5'-(Y)ₙ-(α)ₘ-TGGGGATGGGGGGTTGGAGGAA-(β)ₘ-3', and
an oligonucleotide B comprising the sequence of 5'-(X)ₙ-(γ)ₚ-TGGGGATGGGGGGTTGGAGGAA-(δ)ₚ-3':
   FF) 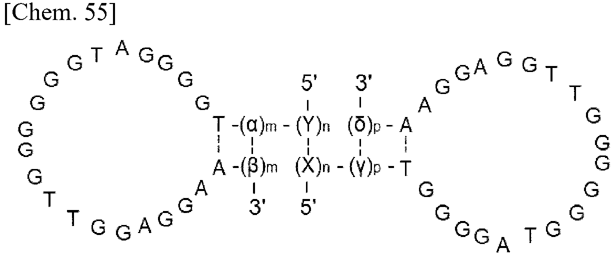 wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 25;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[F12] The bivalent DNA aptamer according to [0], wherein the bivalent DNA aptamer is in the following double-stranded form and consists of:
an oligonucleotide A comprising the sequence of 5'-(Y)ₙ-(α)ₘ-AGGTCAATGGGGATGGGGGGTTGGAGGAATTGACCT-(β)ₘ-3', and
an oligonucleotide B comprising the sequence of 5'-(X)ₙ-(γ)ₚ-AGGTCAATGGGGATGGGGGGTTGGAGGAATTGACCT-(δ)ₚ-3':
   FF1) 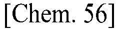 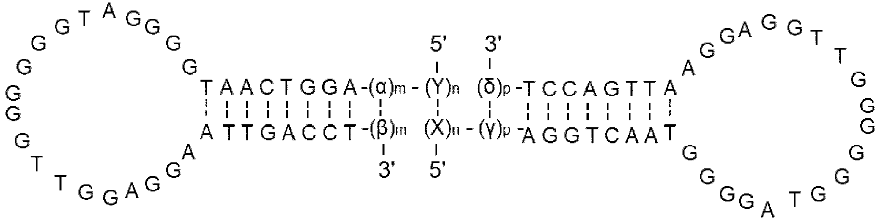 wherein
   m, n, and p represent the numbers of DNA bases;
   m is an integer selected from the group consisting of 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

[13A] The double-stranded bivalent DNA aptamer according to any one of [1A] to [12A] and [F1] to [F12] above, wherein
each set of X and Y is bases that form a base pair,
each set of α and β is bases that form a base pair; and/or
each set of γ and δ is bases that form a base pair.
[14A]
The double-stranded bivalent DNA aptamer according to any one of [1A] to [12A] and [F1] to [F12], wherein m + n + p equals 5 to 21.
[15A]
The double-stranded bivalent DNA aptamer according to any one of [2A], [4A], [6A], [8A], [10A], [12A], [F2], [F4], [F8], [F10], and [F12], wherein the sequence of 5'-(β)ₘ-(X)ₙ-(γ)ₚ-3' is 5'-CTAGTGACTGATTTACG-3', and the sequence of 5'-(δ)ₚ-(Y)ₙ-(α)ₘ-3' is 5'-CGTAAATCAGTCACTAG-3'.
[16A] The double-stranded bivalent DNA aptamer according to [15A], wherein m is 0, n is 17, and p is 0.
[17A] The double-stranded bivalent DNA aptamer according to [16A], wherein the sequence of 5'-(X)ₙ-3' is 5'-CTAGTGACTGATTTACG-3', and the sequence of 5'-(Y)ₙ-3' is 5'-CGTAAATCAGTCACTAG-3'.
[G1] The bivalent DNA aptamer according to [0], wherein the bivalent DNA aptamer is in a double-stranded form and consists of a combination of oligonucleotides selected from the group consisting of the following 1 to 40 combinations of oligonucleotides A and oligonucleotides B:

**[Table 1]**

| Combination | Oligonucleotide A | | Oligonucleotide B | |
|---|---|---|---|---|
| | Name | SEQ ID NO: | Name | SEQ ID NO: |
| 1 | Sticky end20A-1 | 26 | Sticky end20A-2 | 27 |
| 2 | Sticky end20B-1 | 28 | Sticky end20B-2 | 29 |
| 3 | Sticky end20C-1 | 30 | Sticky end20C-2 | 31 |
| 4 | Sticky end20D-1 | 32 | Sticky end20D-2 | 33 |
| 5 | Sticky end20E-1 | 34 | Sticky end20E-2 | 35 |
| 6 | Sticky end19A-1 | 36 | Sticky end19A-2 | 37 |
| 7 | Sticky end19B-1 | 38 | Sticky end19B-2 | 39 |
| 8 | Sticky end19C-1 | 40 | Sticky end19C-2 | 41 |
| 9 | Sticky end19D-1 | 42 | Sticky end19D-2 | 43 |
| 10 | Sticky end19E-1 | 44 | Sticky end19E-2 | 45 |
| 11 | Sticky end18A-1 | 46 | Sticky end18A-2 | 47 |
| 12 | Sticky end18B-1 | 48 | Sticky end18B-2 | 49 |
| 13 | Sticky end18C-1 | 50 | Sticky end18C-2 | 51 |
| 14 | Sticky end18D-1 | 52 | Sticky end18D-2 | 53 |
| 15 | Sticky end18E-1 | 54 | Sticky end18E-2 | 55 |
| 16 | Sticky end17A-1 | 56 | Sticky end17A-2 | 57 |
| 17 | Sticky end17B-1 | 58 | Sticky end17B-2 | 59 |
| 18 | Sticky end17C-1 | 60 | Sticky end17C-2 | 61 |
| 19 | Sticky end17D-1 | 62 | Sticky end17D-2 | 63 |
| 20 | Sticky end17E-1 | 64 | Sticky end17E-2 | 65 |
| 21 | Sticky end16A-1 | 66 | Sticky end16A-2 | 67 |
| 22 | Sticky end16B-1 | 68 | Sticky end16B-2 | 69 |
| 23 | Sticky end16C-1 | 70 | Sticky end16C-2 | 71 |
| 24 | Sticky end16D-1 | 72 | Sticky end16D-2 | 73 |
| 25 | Sticky end16E-1 | 74 | Sticky end16E-2 | 75 |
| 26 | Sticky end15A-1 | 76 | Sticky end15A-2 | 77 |
| 27 | Sticky end15B-1 | 78 | Sticky end15B-2 | 79 |
| 28 | Sticky end15C-1 | 80 | Sticky end15C-2 | 81 |
| 29 | Sticky end15D-1 | 82 | Sticky end15D-2 | 83 |
| 30 | Sticky end15E-1 | 84 | Sticky end15E-2 | 85 |
| 31 | Sticky end14A-1 | 86 | Sticky end14A-2 | 87 |
| 32 | Sticky end14B-1 | 88 | Sticky end14B-2 | 89 |
| 33 | Sticky end14C-1 | 90 | Sticky end14C-2 | 91 |
| 34 | Sticky end14D-1 | 92 | Sticky end14D-2 | 93 |
| 35 | Sticky end14E-1 | 94 | Sticky end14E-2 | 95 |
| 36 | Sticky end13A-1 | 96 | Sticky end13A-2 | 97 |
| 37 | Sticky end13B-1 | 98 | Sticky end13B-2 | 99 |
| 38 | Sticky end13C-1 | 100 | Sticky end13C-2 | 101 |
| 39 | Sticky end13D-1 | 102 | Sticky end13D-2 | 103 |
| 40 | Sticky end13E-1 | 104 | Sticky end13E-2 | 105 |

[H1] A pharmaceutical composition for inhibiting blood coagulation, comprising the DNA aptamer according to any one of [A1] to [A3].
[H2] A method for inhibiting blood coagulation by administering the DNA aptamer according to any one of [A1] to [A3] to a subject, preferably a method for inhibiting blood coagulation activity by causing the DNA aptamer to bind to thrombin in blood.
[H3] The DNA aptamer according to any one of [A1] to [A3] for use in blood coagulation inhibiting treatment.
[H4] Use of the DNA aptamer according to any one of [A1] to [A3] in manufacturing medicine for inhibiting blood coagulation.
[1B] A pharmaceutical composition for inhibiting blood coagulation, comprising the bivalent DNA aptamer according to any one of [0] to [G1] above.
[2B] A method for inhibiting blood coagulation by administering the bivalent DNA aptamer according to any one of [0] to [G1] above to a subject, preferably a method for inhibiting blood coagulation activity by causing the DNA aptamer to bind to thrombin in blood.
[3B] The bivalent DNA aptamer according to any one of [0] to [G1] above for use in blood coagulation inhibiting treatment.
[4B] Use of the bivalent DNA aptamer according to any one of [0] to [G1] above in manufacturing medicine for inhibiting blood coagulation.
[1C] A neutralizing agent for a pharmaceutical composition for inhibiting blood coagulation,
wherein the pharmaceutical composition comprises the bivalent DNA aptamer according to any one of [1] to [G1] above, and
the neutralizing agent comprises a nucleic acid comprising a complementary sequence to 5'-AGGTCAGATGATGGGGATGGG-3' (SEQ ID NO: 23).

[2C] The neutralizing agent for a pharmaceutical composition for inhibiting blood coagulation,
wherein the pharmaceutical composition comprises the bivalent DNA aptamer according to any one of [14] to [16] above, and
the neutralizing agent comprises a nucleic acid comprising a sequence described in [L-M08s]c (SEQ ID NO: 17) or a nucleic acid comprising a sequence described in 08ad (SEQ ID NO: 15).

[I] A neutralizing agent for a pharmaceutical composition for inhibiting blood coagulation,
wherein the pharmaceutical composition comprises a DNA aptamer comprising the sequence of M08s-1 (36) (SEQ ID NO: 107), and
the neutralizing agent comprises a nucleic acid comprising a sequence selected from the group consisting of Ant-M08_36-36 (SEQ ID NO: 142); Ant-M08_36-30 (SEQ ID NO: 143); Ant-M08_36-24 (SEQ ID NO: 144); Ant-M08_36-18 (SEQ ID NO: 145); Ant-M08_36-L18 (SEQ ID NO: 146); Ant-M08_36-R18 (SEQ ID NO: 147); and Ant-M08_36-12 (SEQ ID NO: 148).

[J] A neutralizing agent for a pharmaceutical composition for inhibiting blood coagulation,
wherein the pharmaceutical composition comprises a DNA aptamer comprising the sequence of Pse08-08 (64) (SEQ ID NO: 138), and
the neutralizing agent comprises a nucleic acid comprising the sequence of 2Ant44 _Pse08-08 (64) (SEQ ID NO: 149).

[1D] A method for producing a pharmaceutical composition for inhibiting blood coagulation comprising a bivalent DNA aptamer, comprising the steps of:
1) preparing or providing a test single-stranded oligonucleotide comprising any of the following structures: 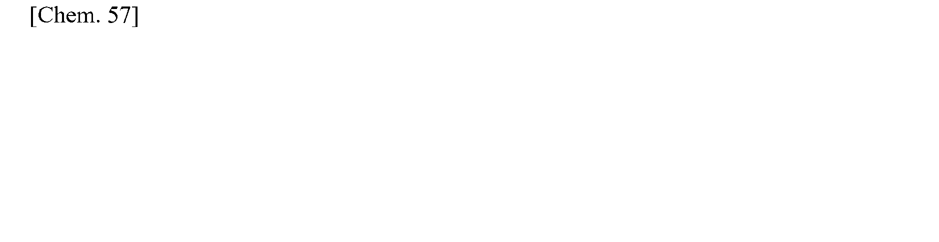 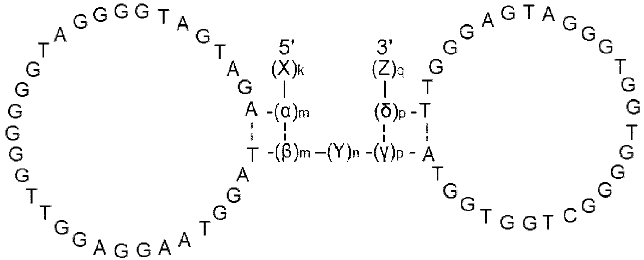 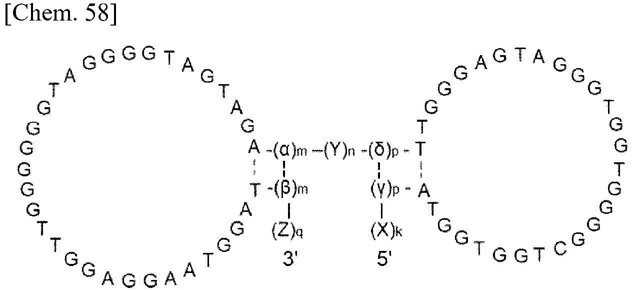 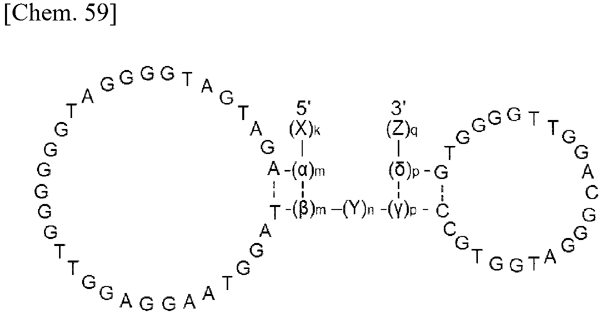 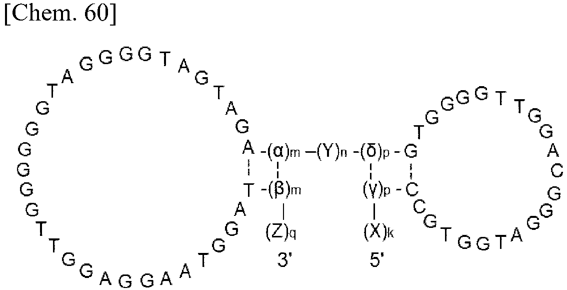 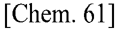 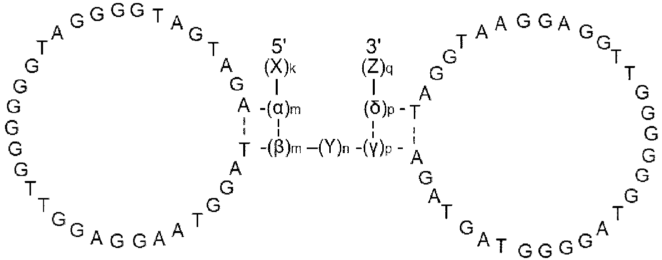 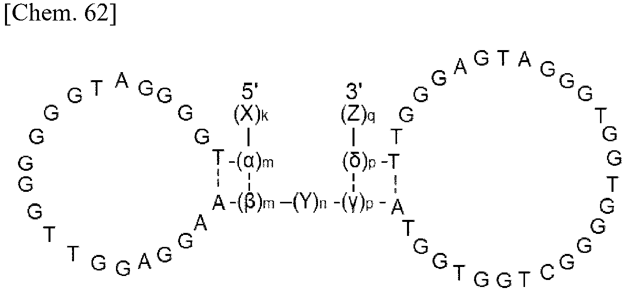 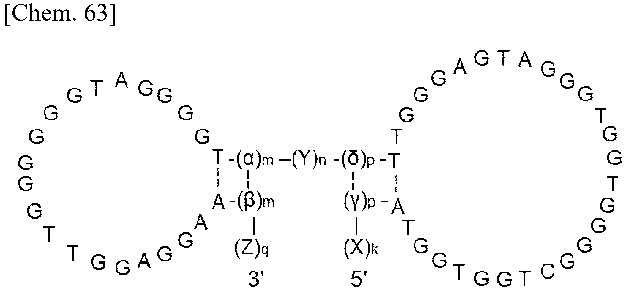 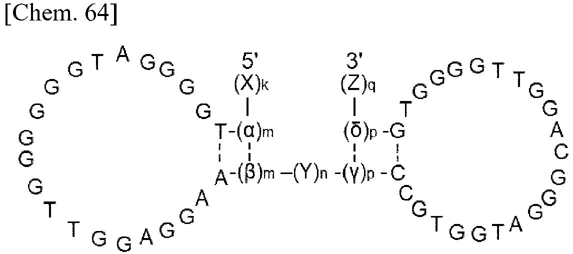 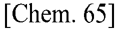 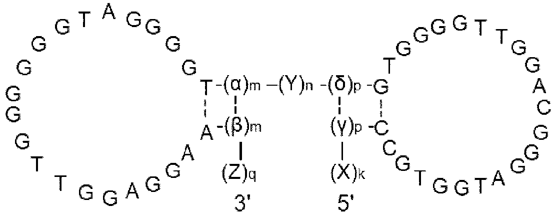 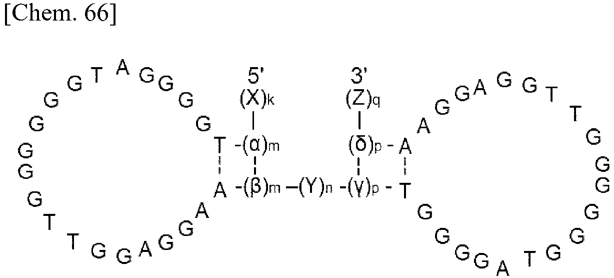 wherein
   k, m, n, p, and q represent the numbers of DNA bases;
   k is 0, 1, 2, or 3;
   m is an integer selected from the group consisting of 1 to 15;
   n is 0, 1, 2, 3, 4, or 5;
   p is an integer selected from the group consisting of 1 to 15;
   q is 0, 1, 2, or 3;
   X, Y, and Z are each independently any bases;
   α, β, γ, and δ are bases;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure;
      and
2) examining an affinity of the test oligonucleotide for thrombin,
wherein
when the affinity of the test oligonucleotide for thrombin is superior to that of the DNA aptamer consisting of the sequence of lin (M08s-TBA29) (SEQ ID NO: 11), the test oligonucleotide is to be contained in the pharmaceutical composition as a bivalent DNA aptamer for inhibiting blood coagulation. [2D]
A method for producing a pharmaceutical composition for inhibiting blood coagulation comprising a bivalent DNA aptamer, comprising the steps of:
1) preparing or providing a test double-stranded oligonucleotide comprising any of the following structures: 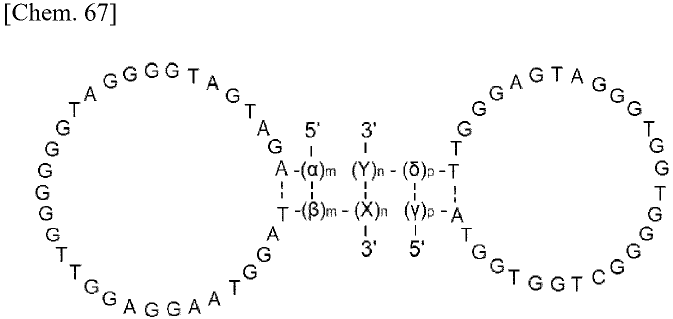 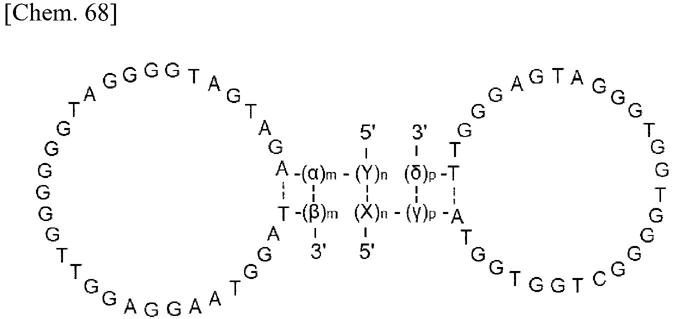 wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 27;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure; 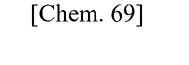 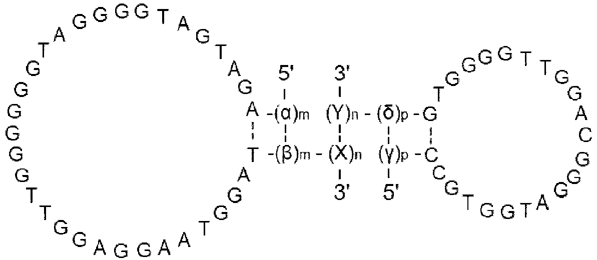 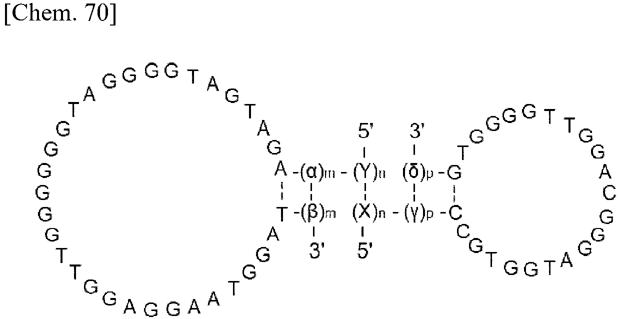
   wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 23;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure; 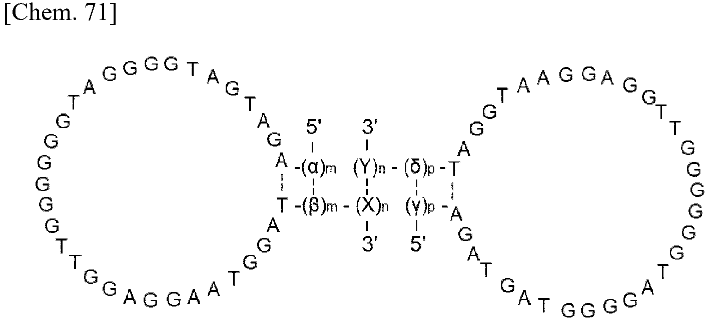 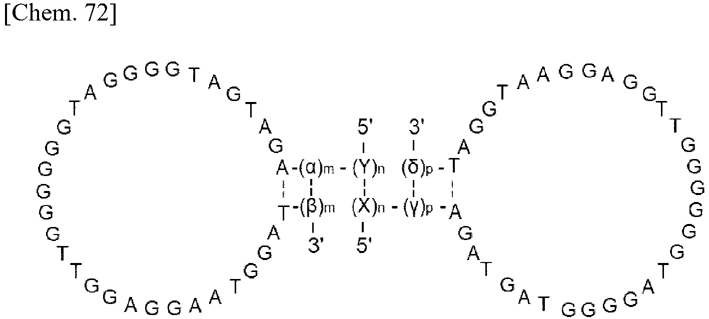
   wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 25;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure,
      and
2) examining the affinity of the test oligonucleotide for thrombin, wherein
   when the affinity of the test oligonucleotide for thrombin is superior to that of the DNA aptamer consisting of the sequence of lin (M08s-TBA29) (SEQ ID NO: 11), the test oligonucleotide is to be contained in the pharmaceutical composition as a bivalent DNA aptamer for inhibiting blood coagulation.

[3D] The production method according to [1D], wherein
each set of α and β is bases that form a base pair; and/or
each set of γ and δ is bases that form a base pair.

[4D] The production method according to [1D], wherein n is 0, and m + p equals 5 to 21.
[5D] The production method according to [2D], wherein
each set of X and Y is bases that form a base pair,
each set of α and β is bases that form a base pair; and/or
each set of γ and δ is bases that form a base pair.

[6D] The production method according to [2D], wherein m + n + p equals 5 to 21.
[7D] The production method according to [6D], wherein
the sequence of 5'-(β)ₘ-(X)ₙ-(γ)ₚ-3' is 5'-CTAGTGACTGATTTACG-3', and the sequence of 5'-(δ)ₚ-(Y)ₙ-(α)ₘ-3' is 5'-CGTAAATCAGTCACTAG-3'.
[8D] The production method according to [6D], wherein m is 0, n is 17, and p is 0.

### Advantageous Effects of Invention

According to the present invention, a monovalent or bivalent aptamer against thrombin can be efficiently produced, the monovalent or bivalent aptamer exerts excellent ability to inhibit blood coagulation, and a neutralizing agent for the monovalent or bivalent aptamer can effectively block the ability of the monovalent or bivalent aptamer to inhibit blood coagulation.

### Brief Description of Drawings

[Fig. 1] Fig. 1 is a schematic diagram showing a binding state of thrombin with an aptamer that binds to exosite I (TAB15 in the figure) and an aptamer that binds to exosite II (TBA29 in the figure) (PDB ID: 5EW1) as an example.
[Fig. 2] Fig. 2 shows the results of examination of the length of a double-stranded linker suitable for the anticoagulation effect by thrombin inhibition. (a) shows the anticoagulation effect when a double-stranded DNA linker is formed of TA base pairs. (b) shows the anticoagulation effect when a double-stranded DNA linker is formed of AT/GC mixed base pairs.
[Fig. 3] Fig. 3 shows predicted secondary structures of the known monovalent aptamers used in Example 2, a bivalent aptamer comprising monovalent aptamers linked via polyT, and, as an example, bivalent aptamers comprising monovalent aptamers linked via double-stranded DNA. (a) NU172, (b) M08s, (c) lin (M08s-TBA29), (d) pse (M08s-M03s), (e) pse (M08s-TBA29), and (f) pse (M08s-M08s).
[Fig. 4] Fig. 4 shows the results of APTT tests using blood samples collected after single administration of each aptamer and low molecular weight argatroban to mice. (a) APTT test at each blood collection time. Weight of mouse: 22 g; age of mouse: 7 weeks. (b) Comparison of the maximum APTT values using blood samples collected 3 minutes after administration. 0.13 µmol/kg. S. D. means with error bars (n=3 or n=5). § Exceeding the measurement limit (APTT > 125 sec). APTT when no aptamer is administered: 22.5 sec.
[Fig. 5] Fig. 5 shows the anticoagulation effect of a single-stranded bivalent aptamer (pse (M08s-M08s)) in human plasma, as an example of the present invention.

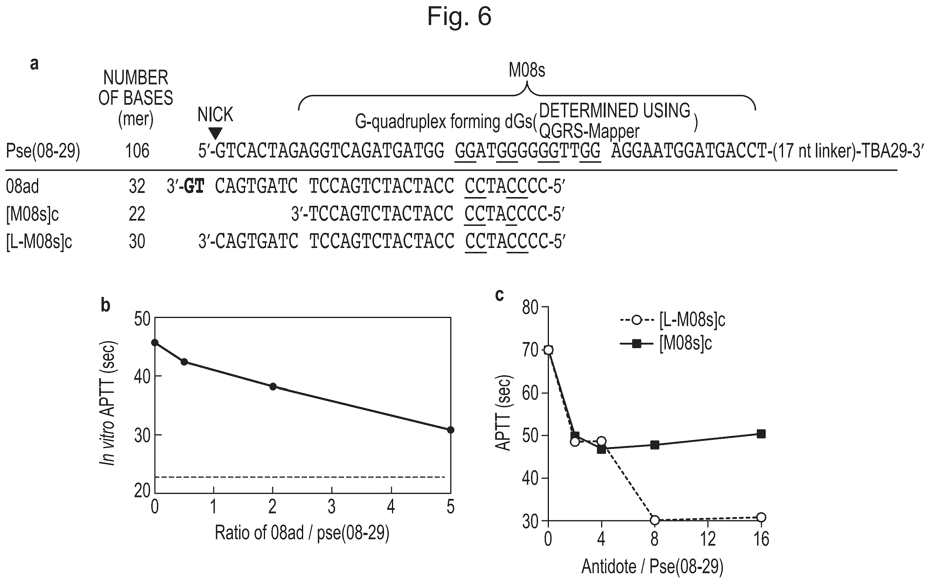

[Fig. 6] Fig. 6 shows the neutralizing effect of adding a complementary strand sequence oligonucleotide to the nucleic acid aptamer, pse (08-29), in human plasma. (a) Correlation diagram between the complementary sequences used as a neutralizing agent and pse (08-29). (b) Neutralizing effect on the anticoagulation effect in a dependent manner of the concentration of the complementary strand, 08ad. pse (08-29): 0.33 µM. Measurement solvent: 32% human plasma in PBS. Dashed line: APTT value of 24.7 sec when a buffer. is used. (c) Neutralizing effects on the anticoagulation effect in a dependent manner of the concentration of the complementary strands, [L-M08s]c and [M08s]c. pse (08-29): 0.33 µM. Measurement solvent: 32% human plasma in PBS. Dashed line: APTT value of 24.7 sec when a buffer is used.
[Fig. 7] Fig. 7 shows the structures of a single-stranded bivalent aptamer (pse (M08s-TBA29)) and one of the corresponding double-stranded bivalent aptamers (pse2 (M08s-TBA29, as an example of the present invention.
[Fig. 8] Fig. 8 shows the results of simulating the stability of the secondary structure of the double-stranded bivalent aptamer, as an example of the present invention.
[Fig. 9A] Fig. 9A shows structural schematic diagrams of the double-stranded bivalent aptamers used in Example 4 (M08s-TBA29: 17 to 20 linkers). % indicates predicted rates of complementary strand formation calculated by using NUPACK.
[Fig. 9B] Fig. 9B shows the results of an APTT test on the double-stranded bivalent aptamers in Example 4 (M08s-TBA29: 17 to 20 linkers).
[Fig. 10A] Fig. 10A shows structural schematic diagrams of the double-stranded bivalent aptamers used in Example 4 (M08s-TBA29: 13 to 16 linkers). % indicates predicted rates of complementary strand formation calculated by using NUPACK.
[Fig. 10B] Fig. 10B shows the results of an APTT test on the double-stranded bivalent aptamers in Example 4 (M08s-TBA29: 13 to 16 linkers).
[Fig. 11A] Fig. 11A shows the difference between M08s (43-mer) and M08s (36-mer) used in Example 5. The underlined bases in M08s (43-mer) are bases deleted in M08s (36-mer).
[Fig. 11B] Fig. 11B shows the anticoagulation effects of M08s (43-mer) and M08s (36-mer) in Example 5.
[Fig. 12A] Fig. 12A shows structural schematic diagrams of the single-stranded bivalent aptamers used in Example 6 (M08s (36)-TBA29).
[Fig. 12B] Fig. 12B shows the results of an APTT test on the single-stranded bivalent aptamers in Example 6 (M08s (36)-TBA29).
[Fig. 13A] Fig. 13A shows a structural schematic diagram of the single-stranded bivalent aptamers used in Example 7 (M08s (36)-M08s (36)).
[Fig. 13B] Fig. 13B shows the results of an APTT test on the single-stranded bivalent aptamers in Example 7 (M08s (36)-M08s (36)).
[Fig. 14A] Fig. 14A shows complementary strand sequence oligonucleotides to M08s (36) and Pse08-08 (64) used in Example 8. The underlines indicate sequences that form stem structures.
[Fig. 14B] Fig. 14B shows neutralizing effects of complementary strand sequence oligonucleotides to a) Pse08-29 ss1-4A (69); b) Pse08-29 ss1-2A (67); and c) Pse08-08 (64) in Example 8.

### Description of Embodiments

As used herein, "comprising" encompasses a form of "substantially comprising", a form of "essentially comprising", a form of "consisting essentially of", and a form of "consisting of".

The present invention includes a bivalent nucleic acid aptamer comprising nucleic acid aptamers for thrombin, two nucleic acid aptamers that bind to exosite I of thrombin, two nucleic acid aptamers that bind to exosite II, or a nucleic acid aptamer that binds to exosite I of thrombin and a nucleic acid aptamer that binds to exosite II, linked to each other.

The nucleic acid aptamer used in the present invention refers to a nucleic acid molecule which has a high affinity for a predetermined target molecule and can specifically bind to it. The nucleic acid molecule having such properties is called a nucleic acid aptamer, and not limited by the nucleotide sequence, the molecular size, the molecular conformation, or the like, unless otherwise stated. Preferably, the nucleic acid aptamer is DNA.

Accordingly, an embodiment of the present invention is a bivalent DNA aptamer comprising DNA aptamers for thrombin; two DNA aptamers that bind to exosite I of thrombin (hereinafter, also referred to as I-1 and I-2); two DNA aptamers that bind to exosite II (hereinafter, also referred to as II-1 and II-2); or a DNA aptamer that binds to exosite I of thrombin and a DNA aptamer that binds to exosite II, linked to each other.

Thrombin (also referred to as factor IIa) is a multifunctional serine protease and an enzyme important in a hemostatic network (the EC No. is EC 3.4.21.5). Thrombin has a function of catalyzing the reaction of converting fibrinogen to fibrin, and in humans, it is encoded by the F2 gene present at p11-q12 of chromosome 11.

Exosite I of thrombin is a site to which a substrate such as fibrinogen, factor X, factor XIII, and protease-activated receptor binds.

Exosite II of thrombin is a site to which a substrate such as factor V, Factor VIII, and antithrombin III binds.

The nucleic acid aptamer that binds to exosite I or exosite II of thrombin of the present invention may be subjected to a pharmacologically acceptable modification (e.g., PEGylation or labelling with a fluorescent substance or the like; substitution with an artificial nucleic acid (e.g., fluorinated pyrimidine); or partial or complete conversion of a phosphate bond between bases to a phosphorothioate bond or a phosphorodithioate bond) as long as the nucleic acid aptamer maintains the ability to bind to exosite I or exosite II of thrombin. In addition, the nucleic acid aptamer may have 1 to several, for example, 1, 2, or 3 bases substituted, deleted, inserted, or the like in its sequence.

Without specific limitation, examples of the aptamer that binds to exosite I include, but are not limited to, TBA15, NU172, M08s, and M08s (36), which is described later, and examples of the aptamer that binds to exosite II include, but are not limited to, TBA29 and M03s.

In an embodiment of the present invention, two DNA aptamers that bind to exosite I of thrombin, two DNA aptamers that bind to exosite II, or a DNA aptamer that binds to exosite I of thrombin and a DNA aptamer that binds to exosite II, may be linked via a duplex nucleic acid to each other. In the above aspect, the two DNA aptamers that bind to exosite I of thrombin (I-1 and 1-2) may be the same (sequence) molecules or different (sequence) molecules. Similarly, the two DNA aptamers that bind to exosite II of thrombin (II-1 and II-2) may be the same (sequence) molecules or different (sequence) molecules.

The duplex nucleic acid means a nucleic acid in which two polydeoxyribonucleotides or polyribonucleotides, or their complexes are oriented in antiparallel directions, and their constituent bases are linked via a hydrogen bond.

Without specific limitation, such a duplex nucleic acid is preferably DNA. Accordingly, an embodiment of the present invention is a bivalent DNA aptamer comprising two DNA aptamers that bind to exosite I of thrombin, two DNA aptamers that bind to exosite II, or a DNA aptamer that binds to exosite I of thrombin and a DNA aptamer that binds to exosite II, which are linked via duplex DNA to each other.

An embodiment of the present invention is a single-stranded bivalent DNA aptamer comprising two DNA aptamers that bind to exosite I of thrombin, two DNA aptamers that bind to exosite II, or a DNA aptamer that binds to exosite I of thrombin and a DNA aptamer that binds to exosite II, which are linked via duplex DNA to each other.

Such duplex DNA may be formed:
(1) in such a way that the complementarity between the DNA sequence structure (hereinafter, also referred to as an α sequence structure) bound to the 5'-end of the DNA aptamer that binds to exosite I of thrombin (or I-1 or I-2), and the DNA sequence structure (hereinafter, also referred to as a β sequence structure) bound to the 3'-end of the DNA aptamer that binds to exosite I of thrombin (or I-1 or I-2) is maintained with a certain degree;
(2) in such a way that the complementarity between the DNA sequence structure (hereinafter, also referred to as a γ sequence structure) bound to the 5'-end of the DNA aptamer that binds to exosite II of thrombin (or II-1 or II-2), and the DNA sequence structure (hereinafter, also referred to as a δ sequence structure) bound to the 3'-end of the DNA aptamer that binds to exosite II of thrombin (or II-1 or II-2) is maintained with a certain degree; or
(3) in both ways of (1) and (2).

The above complementarity may be made by a so-called Watson-Crick base pair, or may be made by a mismatched base pair in which the thermodynamic stability is decreased to some extent. It is preferable for base pairs to be formed for hydrogen bonds between bases. Here, the Watson-Crick base pair includes, but not particularly limited to, A and T, and C and G; and the mismatched base pair includes 8 types: G-G, G-A, G-T, A-A, A-C, C-T, C-C, and T-T.

Although not particularly limited,
(1) a) 5'-α sequence structure-DNA aptamer that binds to exosite I of thrombin (or I-1 or I-2)-β sequence structure-3', and b) a DNA aptamer that binds to exosite II of thrombin (or I-2, II-2) may be included in this order or in the reverse order;
(2) a) 5'-y sequence structure-DNA aptamer that binds to exosite II of thrombin (or II-1 or II-2)-δ sequence structure-3', and b) a DNA aptamer that binds to exosite I of thrombin (or I-1, II-1) may be included in this order or in the reverse order; or
(3) a) 5'-α sequence structure-DNA aptamer that binds to exosite I of thrombin (or I-1 or I-2)-β sequence structure-3', and b) 5'-γ sequence structure-DNA aptamer that binds to exosite II of thrombin (or II-1 or II-2)-δ sequence structure-3' may be included in this order or in the reverse order.

Note that, although not particularly limited, a sequence of 0, 1, 2, or 3 bases that does not form duplex DNA (hereinafter, also referred to as a Y sequence structure) may be included between a) and b) of the above (1) to (3).

In addition, at the 5'-end and-3'-end of the single-stranded bivalent DNA aptamer, sequences that do not contribute to the duplex DNA and do not contribute to the binding capacity of the aptamer (hereinafter, also referred to as X sequence structure and Z sequence structure, respectively) may be contained.

The α sequence structure and the β sequence structure; and the γ sequence structure and the δ sequence structure may contain modified bases (e.g., methylcytosine, deoxyuracil, inosine Ds and Px, and NaM and TPT3) that can maintain the complementarity in addition to the normal Watson-Crick complementary bases (i.e., A and T; and G and C). Alternatively, the α sequence structure and the β sequence structure; and the γ sequence structure and the δ sequence structure may contain mismatched complementary bases (e.g., G-G, G-A, and G-T).

The present invention includes a DNA aptamer that contains the sequence of SEQ ID NO: 106. As long as it functions as a DNA aptamer that binds to exosite I of thrombin, the total number of bases is not limited, and it may contain any sequence that functions as another aptamer (for thrombin or the like).

In an embodiment of the present invention, the above DNA aptamer has the following structure: 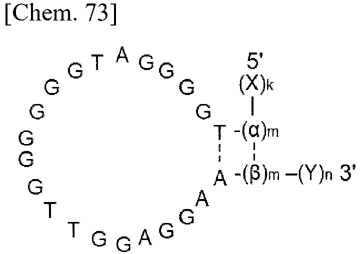 wherein k, m, and n represent the numbers of DNA bases;
k is 0, 1, 2, or 3;
m is an integer selected from the group consisting of 1 to 15;
n is 0, 1, 2, or 3;
X and Y are each independently any bases;
α and β are bases; and
5'-(α)ₘ-3' and 5'-(β)ₘ-3' form an antiparallel double-stranded structure.

In an embodiment of the present invention, the above DNA aptamer has the following structure:

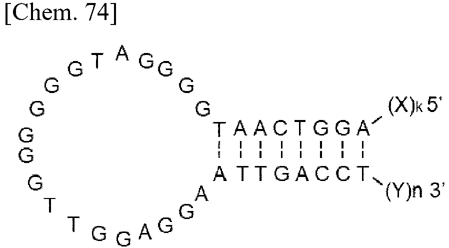

In addition, the above DNA aptamer may contain the sequence of M08s (36) (SEQ ID NO: 107; hereinafter, also referred to as M08s-1 (36)).

An embodiment of the present invention is
a single-stranded bivalent DNA aptamer comprising any of the following primary structures a-1) to f):
a-1) 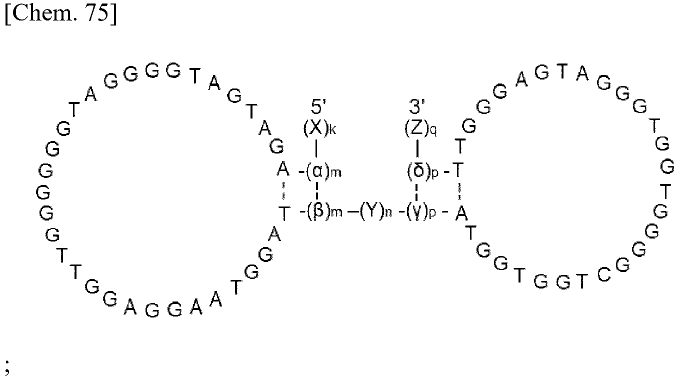
a-2) 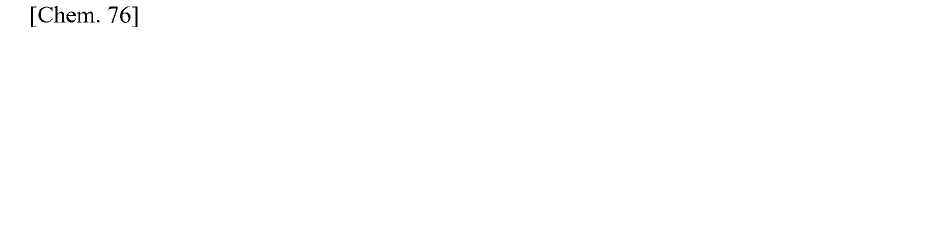 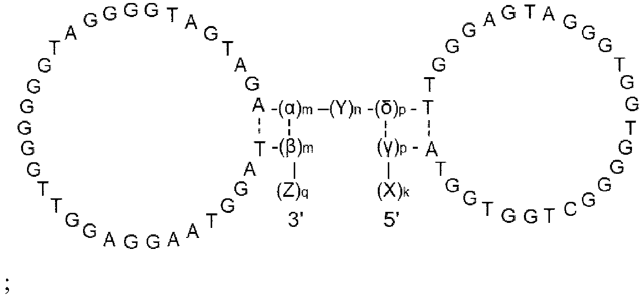
b-1) 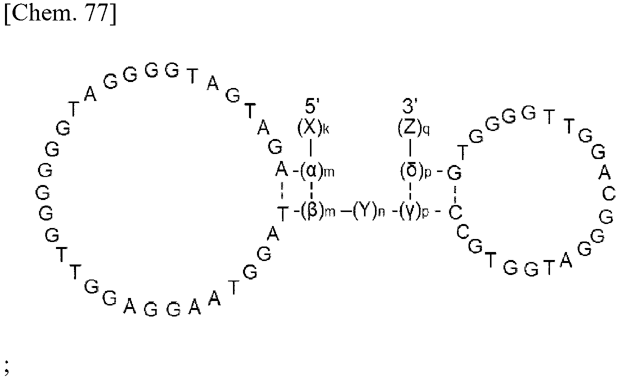
b-2) 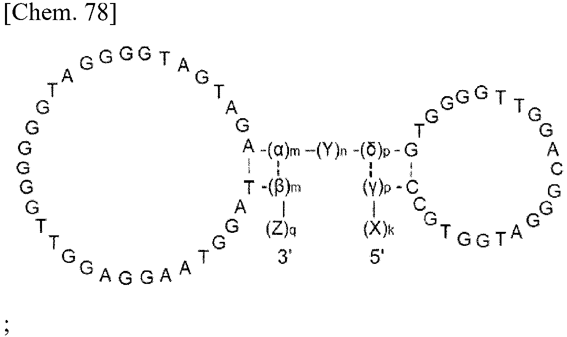
c) 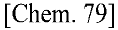 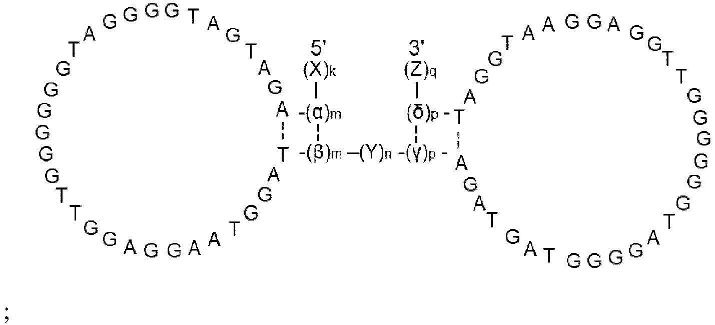
d-1) 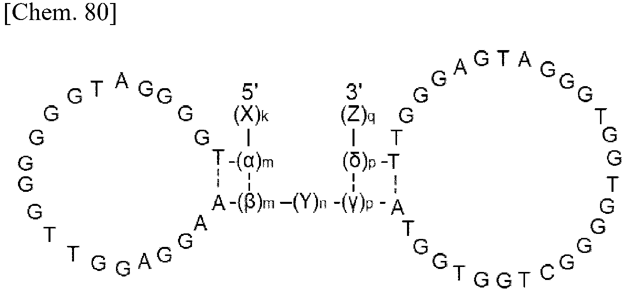
d-2) 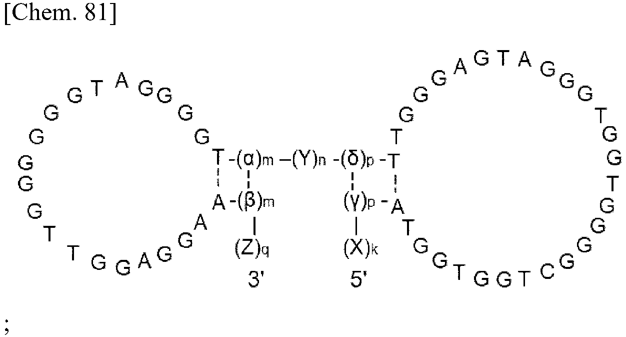
e-1) 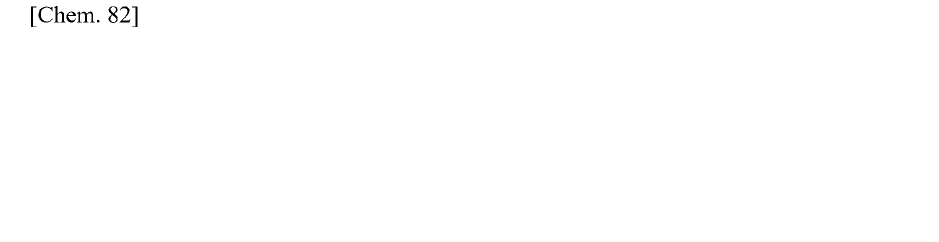 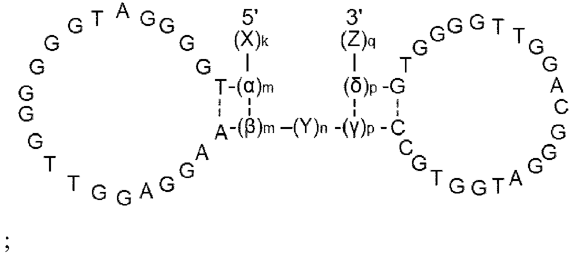
e-2) 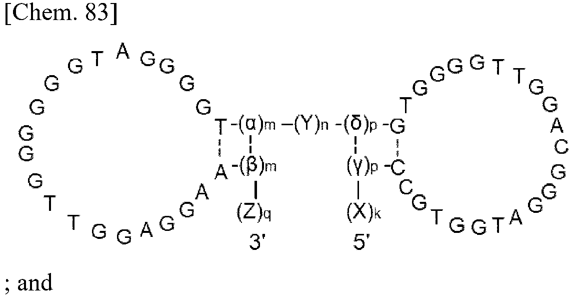
f) 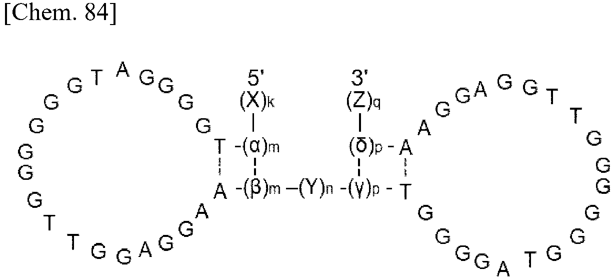 wherein
   k, m, n, p, and q represent the numbers of DNA bases;
   k is 0, 1, 2, or 3;
   m is an integer selected from the group consisting of 1, 2, 3, 4, 5, 6, 7, 8, 9, 10, 11, 12, 13, 14, and 15;
   n is 0, 1, 2, 3, 4, or 5;
   p is an integer selected from the group consisting of 1, 2, 3, 4, 5, 6, 7, 8, 9, 10, 11, 12, 13, 14, 15, 16, and 17;
   q is 0, 1, 2, or 3;
   X, Y, and Z are each independently any bases;
   α, β, γ, and δ are bases;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

Here, (X)ₖ, (Y)ₙ, and (Z)ₚ correspond to the above X sequence structure, the Y sequence structure, and the Z sequence structure, respectively, 5'-(α)ₘ-3' and 5'-(β)ₘ-3' correspond to the α sequence structure and the β sequence structure that form an antiparallel base pair (hereinafter, dashed lines may refer to hydrogen bonds, and covalent bonds may be indicated by solid lines), and 5'-(γ)ₚ-3' and 5'-(δ)ₚ-3' correspond to the γ sequence structure and the δ sequence structure that form an antiparallel base pair.

The above single-stranded bivalent DNA aptamer includes:
a1-1) 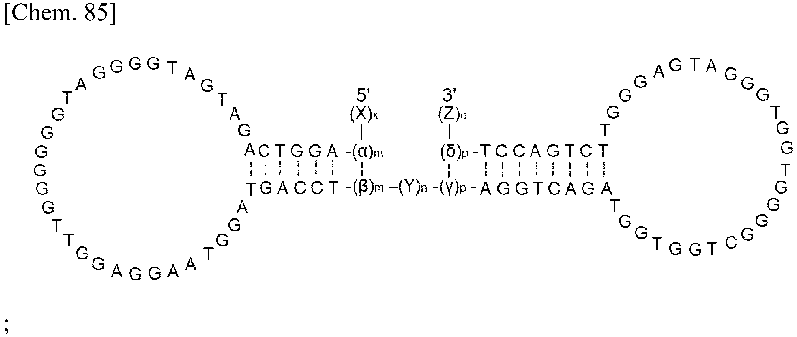
a1-2) 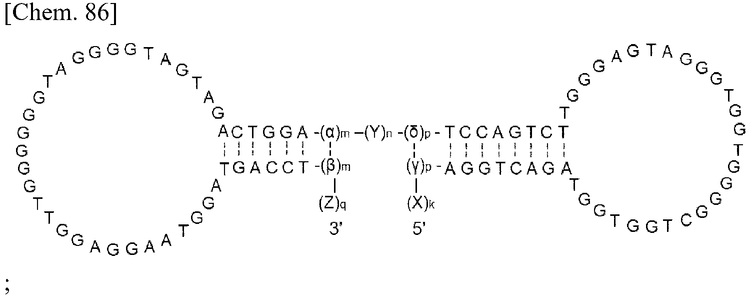
b1-1) 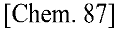 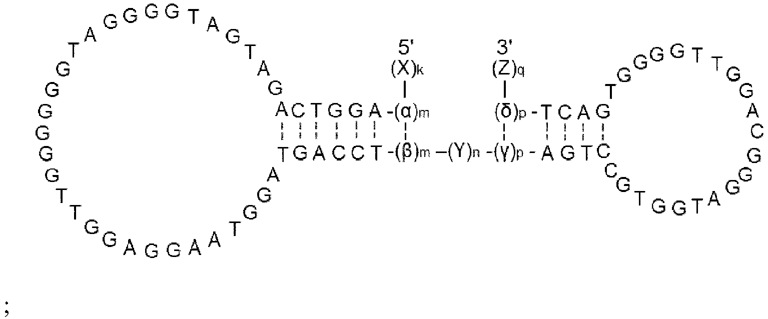
b1-2) 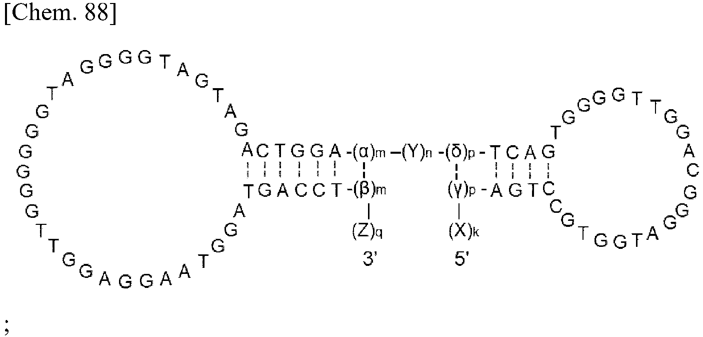
c1) 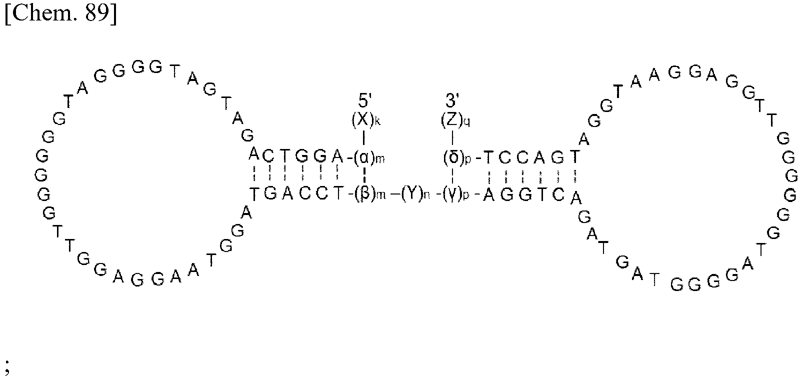
d1-1) 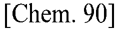 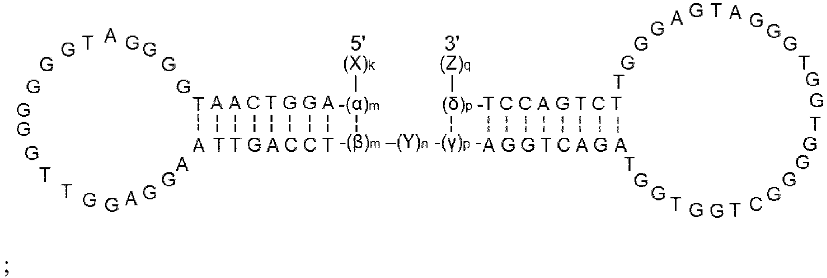
d1-2) 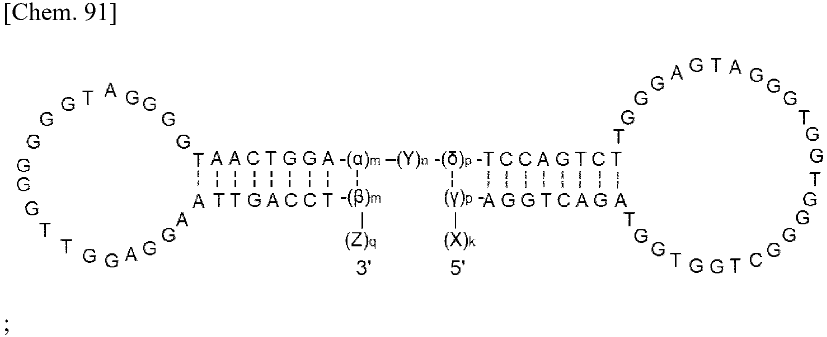
e1-1) 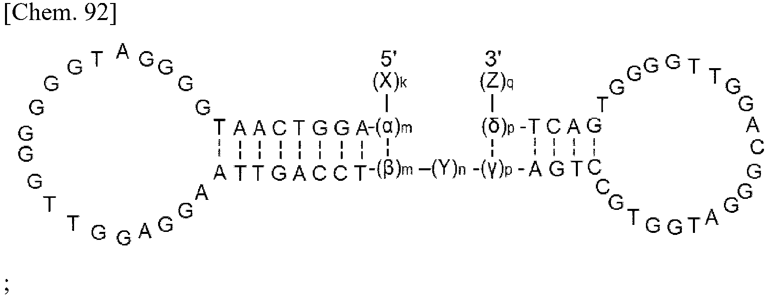
e1-2) 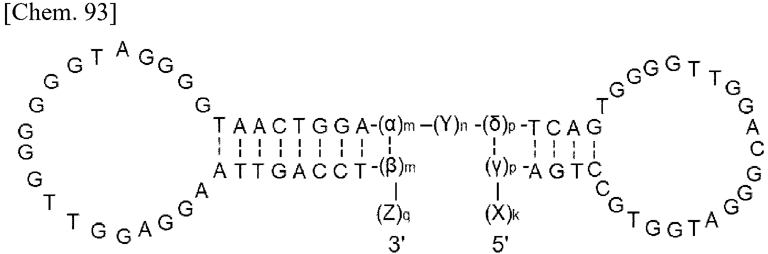 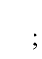 and
f1) 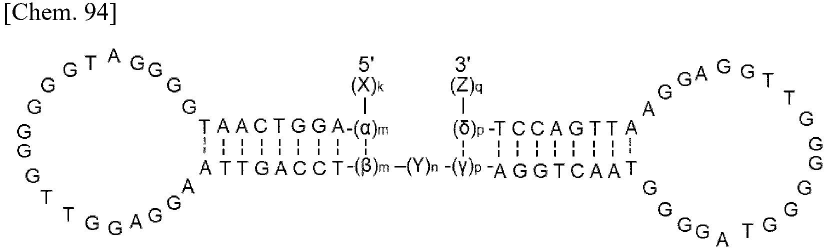 in other words, in a-1) to f), a part of the 3'-end side of 5'-(α)ₘ-3' is AGGTC or AGGTCAA, and a part of the 5'-end side of 5'-(β)ₘ-3' is GACCT or TTGACCT; in a-1) to f), a part of the 3'-end side of 5'-(γ)ₚ-3' is AGGTCAG, AGT, AGGTC, or AGGTCAA, and a part of the 5'-end side of 5'-(δ)ₚ-3' is CTGACCT, ACT, GACCT, or TTGACCT.

The above single-stranded bivalent DNA aptamer includes:
a2-1) 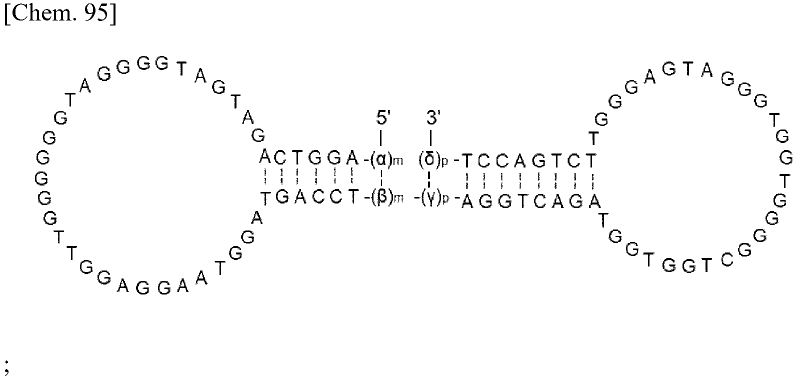
a2-2) 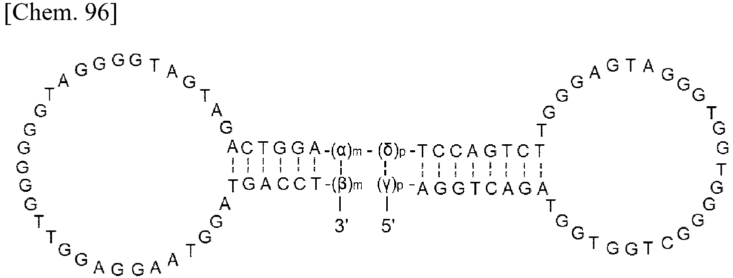 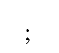
b2-1) 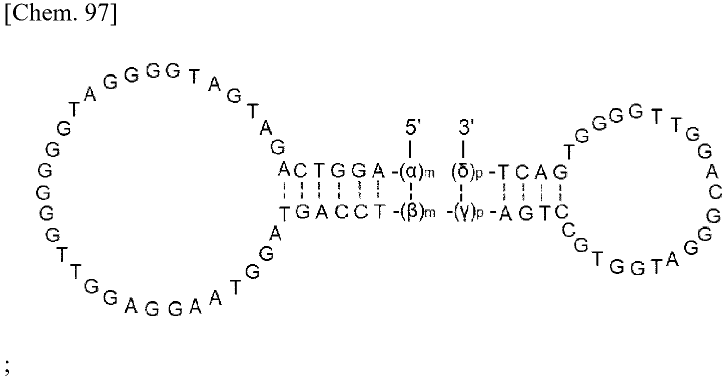
b2-2) 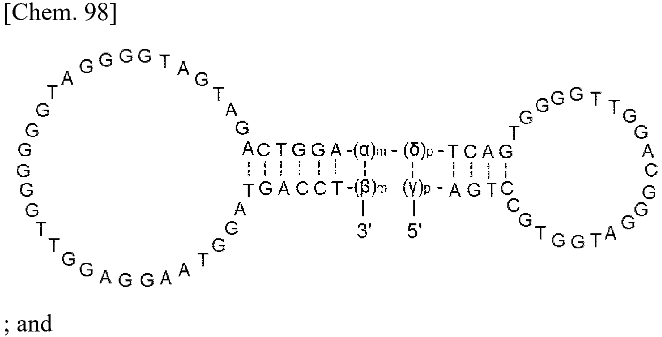
c2) 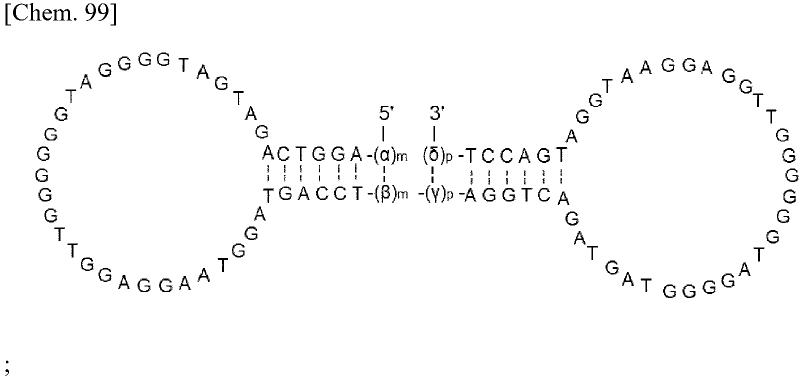
d2-1) 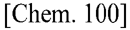 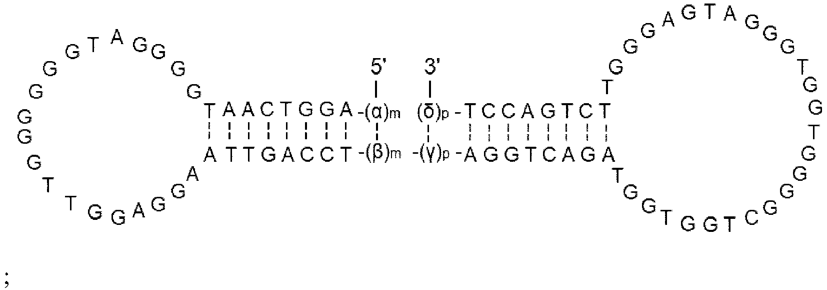
d2-2) 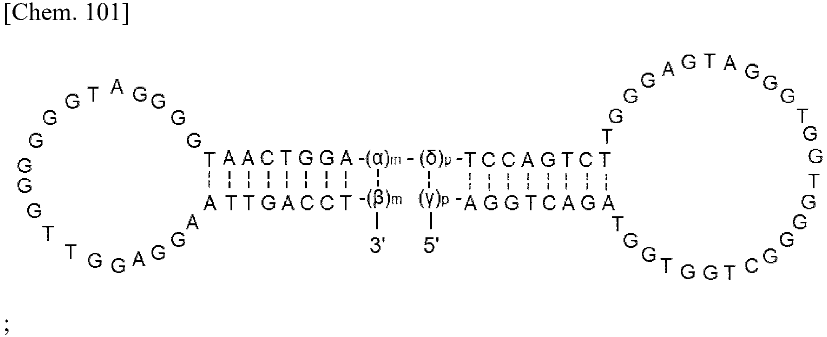
e2-1) 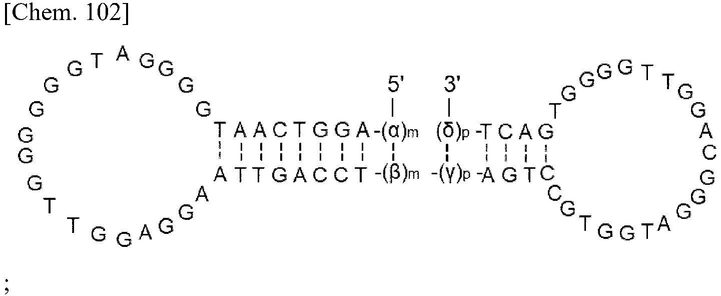
e2-2) 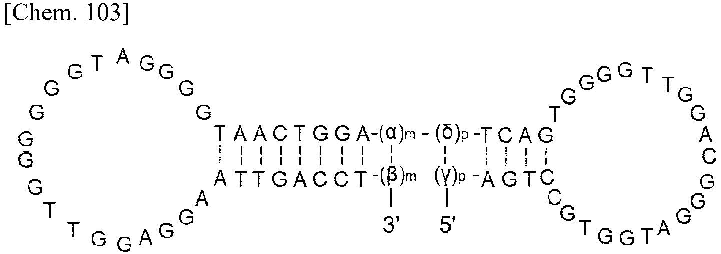 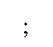 f2) 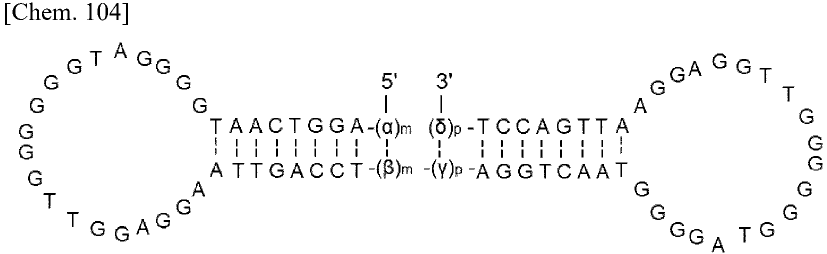 in other words, the X sequence structure, the Y sequence structure, and the Z sequence structure are not comprised.

Although not particularly limited, the length of the duplex DNA moiety (i.e., m + p) is preferably 1, 2, 3, 4, 5, 6, 7, 8, 9, 10, 11, 12, 13, 14, 15, 16, 17, 18, 19, 20, or 21, more preferably 5 to 21, and further preferably 5 to 17.

The above single-stranded bivalent DNA aptamer may include:
the sequence of pse (M08s-M03s) (SEQ ID NO: 12);
the sequence of pse (M08s-TBA29) (SEQ ID NO: 13);
the sequence of pse (M08s-M08s) (SEQ ID NO: 22);
the sequence of M08s-1 (36) (SEQ ID NO: 107) and the sequence of TBA29 (SEQ ID NO: 3); or
two sequences of M08s-1 (36) (SEQ ID NO: 107).

Alternatively, the single-stranded bivalent DNA aptamer may include a sequence selected from the group consisting of:
Pse08-29 ss1-TTACG (70) (SEQ ID NO: 108);
Pse08-29 ss1-5A (70) (SEQ ID NO: 109);
Pse08-29 ss1-4A (69) (SEQ ID NO: 110);
Pse08-29 ss1-2A (67) (SEQ ID NO: 111);
Pse08-29 ss1-5T (70) (SEQ ID NO: 112);
Pse08-29 ss1-4T (69) (SEQ ID NO: 113);
Pse08-29 ss1-2T (67) (SEQ ID NO: 114);
Pse08-29 ss1-0A (65) (SEQ ID NO: 115);
Pse08-29 ss1-0Aa (65) (SEQ ID NO: 116);
Pse08-29 ss1-0Ab (65) (SEQ ID NO: 117);
Pse08-29 ss1-0Ac (65) (SEQ ID NO: 118);
Pse08-29 ss2-CGTAA (70) (SEQ ID NO: 119);
Pse08-29 ss2-5A (70) (SEQ ID NO: 120);
Pse08-29 ss2-4A (69) (SEQ ID NO: 121);
Pse08-29 ss2-2A (67) (SEQ ID NO: 122);
Pse08-29 ss2-5T (70) (SEQ ID NO: 123);
Pse08-29 ss2-4T (69) (SEQ ID NO: 124);
Pse08-29 ss2-2T (67) (SEQ ID NO: 125);
Pse08-29 ss2-0B (65) (SEQ ID NO: 126);
Pse08-29 ss2-0Ba (65) (SEQ ID NO: 127);
Pse08-29 ss2-0Bb (65) (SEQ ID NO: 127); and
Pse08-29 ss2-0Bc (65) (SEQ ID NO: 129).

Alternatively, the single-stranded bivalent DNA aptamer may include a sequence selected from the group consisting of:
Pse08-08 (72) (SEQ ID NO: 130);
Pse08-08 (68) (SEQ ID NO: 131);
Pse08-08 (64) (SEQ ID NO: 132);
Pse08-08 (60) (SEQ ID NO: 133);
Pse08-08 (72-GC) (SEQ ID NO: 134); and
Pse08-08 (60-GC) (SEQ ID NO: 135).

An embodiment of the present invention is a bivalent DNA aptamer comprising two DNA aptamers that bind to exosite I of thrombin; two DNA aptamers that bind to exosite II; or a DNA aptamer that binds to exosite I of thrombin and a DNA aptamer that binds to exosite II, which are linked via duplex DNA to each other.

The DNA aptamer that binds to exosite I of thrombin (or I-1, I-2) and one side of the sequence that forms duplex DNA (hereinafter, also referred to as the X sequence structure) are bound to each other (hereinafter, the structure may be referred to as oligonucleotide A), and the DNA aptamer that binds to exosite II of thrombin (or II-1, II-2) and the other side of the sequence that forms duplex DNA (hereinafter, also referred to as the Y sequence structure) are bound to each other (hereinafter, the structure may be referred to as oligonucleotide B). Using the complementarity, a bivalent aptamer linked via a hydrogen bond may be formed by forming a double-stranded structure.

Or conversely, the Y sequence structure and the DNA aptamer that binds to exosite I of thrombin (or I-1, I-2) are bound to each other, and the X sequence structure and the DNA aptamer that binds to exosite II of thrombin (or I-1, II-2) are bound to each other. Using the complementarity, a bivalent aptamer linked via a hydrogen bond may be formed by forming a double-stranded structure.

The above complementarity may be made by a so-called Watson-Crick base pair, or may be made by a mismatched base pair. It is preferable for base pairs to be formed for hydrogen bonds between bases. Here, the Watson-Crick base pair includes, but not particularly limited to, A and T, and C and G; and the mismatched base pair includes 8 types: G-G, G-A, G-T, A-A, A-C, C-T, C-C, and T-T.

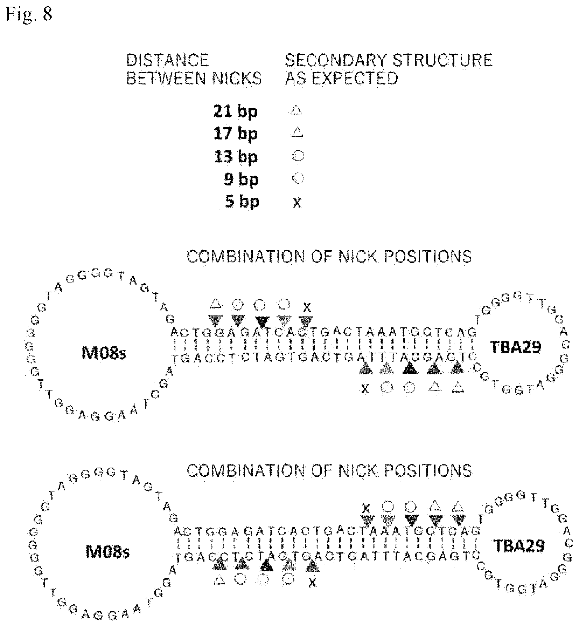

Whether the double-stranded structure is maintained in the living body (or in a similar environment thereof) can be simulated using a nucleic acid secondary structure prediction software, from the complementarity between the X sequence structure and the Y sequence structure, (e.g., NUPACK: Non Patent Literature 3) (Fig. 8). According to this, the length of the X sequence structure and the Y sequence structure is preferably 6 bases long or more, 7 bases long or more, 8 bases long or more, 9 bases long or more, 10 bases long or more, 11 bases long or more, 12 bases long or more, 13 bases long or more, 14 bases long or more, 15 bases long or more, 16 bases long or more, or 17 bases long or more, and preferably 25 bases long or less, 24 bases long or less, 23 bases long or less, 22 bases long or less, 21 bases long or less, 20, or 19 bases long or less.

Sequence structures having the complementarity (hereinafter, also referred to as an α sequence structure and a β sequence structure) may be linked to the 5'-end and the 3'-end of the DNA aptamer that binds to exosite I of thrombin (or I-1, I-2). The DNA aptamer that binds to exosite I (or I-1, I-2) may bind to the X sequence structure via the β sequence structure to form oligonucleotide A, or may bind to the Y sequence structure via the α sequence structure to form oligonucleotide A.

Sequence structures having the complementarity (hereinafter, also referred to as an γ sequence structure and a δ sequence structure) may be linked to the 5'-end and the 3'-end of the DNA aptamer that binds to exosite II of thrombin (or II-1, II-2). The DNA aptamer that binds to exosite II (or II-1, II-2) may bind to the X sequence structure via the γ sequence structure to form oligonucleotide B, or may bind to the Y sequence structure via the δ sequence structure to form oligonucleotide B.

Accordingly, in the double-stranded bivalent aptamer, the duplex DNA may be formed of:
"the X sequence structure and the Y sequence structure" only;
"the α sequence structure and the β sequence structure", and "the X sequence structure and the Y sequence structure";
"the X sequence structure and the Y sequence structure" and "the γ sequence structure and the δ sequence structure"; or
"the α sequence structure and the β sequence structure", "the X sequence structure and the Y sequence structure" and "the γ sequence structure and the δ sequence structure".

"The X sequence structure and the Y sequence structure", "the α sequence structure and the β sequence structure", and "the γ sequence structure and the δ sequence structure" may contain modified bases (e.g., methylcytosine, deoxyuracil, inosine Ds and Px, and NaM and TPT3) that can maintain the complementarity in addition to the normal Watson-Crick complementary bases (i.e., A and T; and G and C). Alternatively, the α sequence structure and the β sequence structure; and the γ sequence structure and the δ sequence structure may contain mismatched complementary bases (e.g., G-G, G-A, and G-T).

The above double-stranded bivalent DNA aptamer includes:
the following double-stranded bivalent DNA aptamer using a 3'-end overhang,
consisting of:
   an oligonucleotide A comprising the sequence of 5'-(α)ₘ-AGATGATGGGGATGGGGGGTTGGAGGAATGGAT (SEQ ID NO: 18)-(β)ₘ-(X)ₙ-3', and
   an oligonucleotide B comprising the sequence of 5'-(γ)ₚ-ATGGTGGTCGGGGTGGTGGGATGAGGGTTCTGACCT (SEQ ID NO: 19)-(δ)ₚ-(Y)ₙ-3';
   aa) 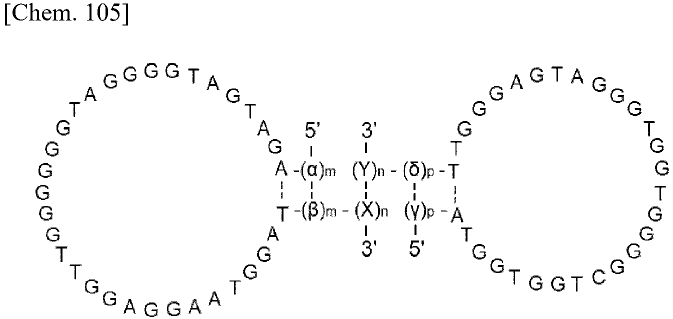 and
   the following double-stranded bivalent DNA aptamer using a 5'-end overhang, consisting of an oligonucleotide A comprising the sequence of 5'-(Y)ₙ-(α)ₘ-AGATGATGGGGATGGGGGGTTGGAGGAATGGAT-(β)ₘ-3', and
   an oligonucleotide B comprising the sequence of 5'-(X)ₙ-(γ)ₚ-ATGGTGGTCGGGGTGGTGGGATGAGGGTT-(δ)ₚ-3';
   bb) 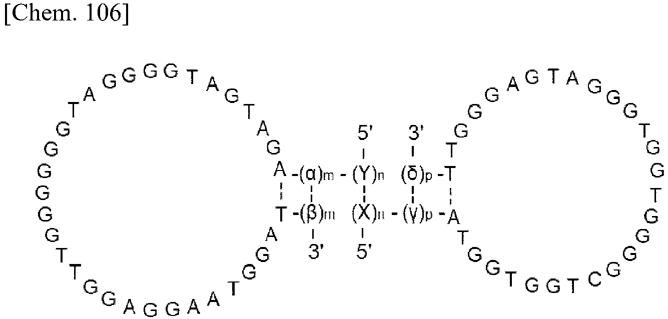
   wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 27;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and 5'-(γ)ₚ-3' and 5'-(δ)ₚ-3' form an antiparallel double-stranded structure.

The above double-stranded bivalent DNA aptamer includes:
the following double-stranded bivalent DNA aptamer using a 3'-end overhang,
consisting of:
   an oligonucleotide A comprising the sequence of 5'-(α)ₘ-AGGTCAGATGATGGGGATGGGGGGTTGGAGGAATGGATGACCT (SEQ ID NO: 14)-(β)ₘ-(X)ₙ-3', and
   an oligonucleotide B comprising the sequence of 5'-(γ)ₚ-AGGTCAGATGGTGGTCGGGGTGGTGGGATGAGGGTTCTGACCT (SEQ ID NO: 21)-(δ)ₚ-(Y)ₙ-3';
   aa1) 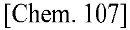 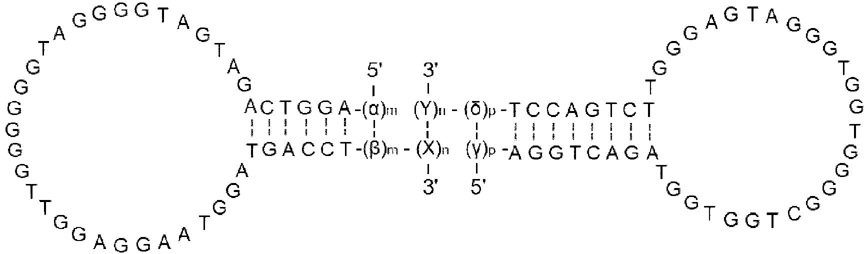 and
   the following double-stranded bivalent DNA aptamer using a 5'-end overhang,
   consisting of an oligonucleotide A comprising the sequence of 5'-(Y)ₙ-(α)ₘ-AGGTCAGATGATGGGGATGGGGGGTTGGAGGAATGGATGACCT-(β)ₘ-3', and
   an oligonucleotide B comprising the sequence of 5'-(X)ₙ-(γ)ₚ-AGGTCAGATGGTGGTCGGGGTGGTGGGATGAGGGTTCTGACCT-(δ)ₚ-3';
   bb1) 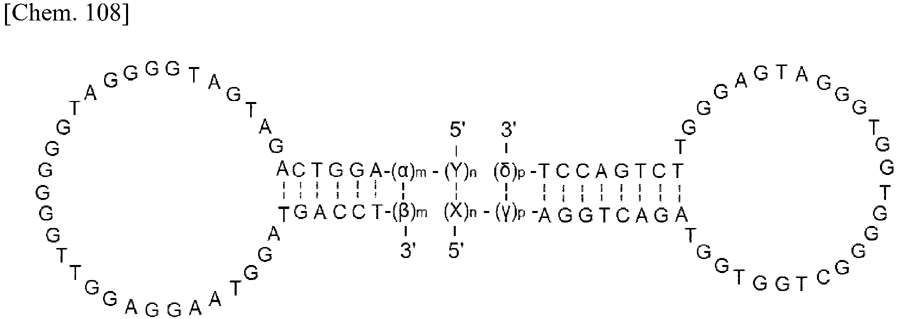
   wherein
   m, n, and p represent the numbers of DNA bases;
   m is 0 to 20;
   n is an integer selected from the group consisting of 1 to 20;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure, in other words, in c) and d), a part of the 3'-end side of 5'-(α)ₘ-3' is AGGTC, and a part of the 5'-end side of 5'-(β)ₘ-3' is GACCT;
   in c) and d), a part of the 3'-end side of 5'-(γ)ₚ-3' is AGGTCAG, and a part of the 5'-end side of 5'-(δ)ₚ-3' is CTGACCT.

The above double-stranded bivalent DNA aptamer includes:
the following double-stranded bivalent DNA aptamer using a 3'-end overhang,
consisting of:
   an oligonucleotide A comprising the sequence of 5'-(α)ₘ-AGATGATGGGGATGGGGGGTTGGAGGAATGGAT (SEQ ID NO: 18)-(β)ₘ-(X)ₙ-3', and
   an oligonucleotide B comprising the sequence of 5'-(γ)ₚ-CCGTGGTAGGGCAGGTTGGGGTG (SEQ ID NO: 20)-(δ)ₚ-(Y)ₙ-3';
   cc) 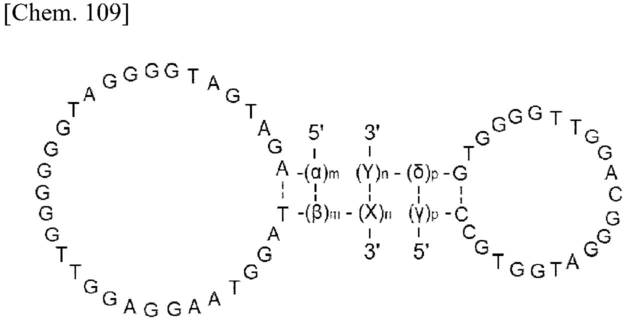 and
   the following double-stranded bivalent DNA aptamer using a 5'-end overhang,
   consisting of an oligonucleotide A comprising the sequence of 5'-(Y)ₙ-(α)ₘ-AGGTCAGATGATGGGGATGGGGGGTTGGAGGAATGGATGACCT-(β)ₘ-3', and
   an oligonucleotide B comprising the sequence of 5'-(X)ₙ-(γ)ₚ-CCGTGGTAGGGCAGGTTGGGGTG-(δ)ₚ-3';
   dd) 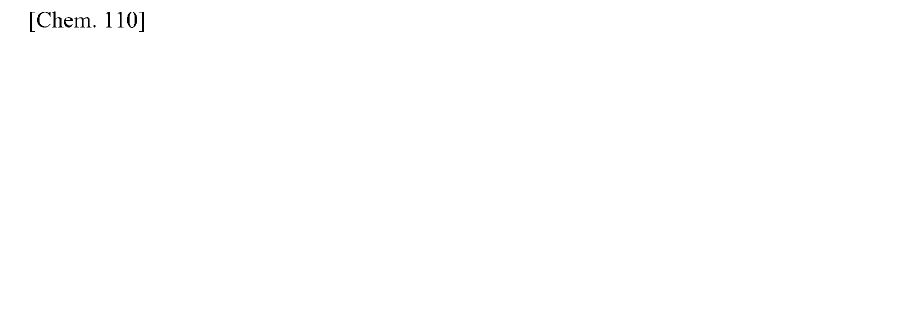 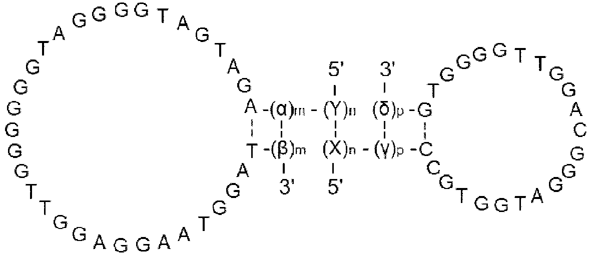
   wherein
   m, n, and p represent the numbers of DNA bases;
   m is an integer selected from the group consisting of 0, 1, 2, 3, 4, 5, 6, 7, 8, 9, 10, 11, 12, 13, 14, 15, 16, 17, 18, 19, 20, 21, 22, 22, 23, 24, and 25;
   n is an integer selected from the group consisting of 0, 1, 2, 3, 4, 5, 6, 7, 8, 9, 10, 11, 12, 13, 14, 15, 16, 17, 18, 19, 20, 21, 22, 22, 23, 24, and 25;
   p is an integer selected from the group consisting of 0, 1, 2, 3, 4, 5, 6, 7, 8, 9, 10, 11, 12, 13, 14, 15, 16, 17, 18, 19, 20, 21, 22, 22, and 23;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

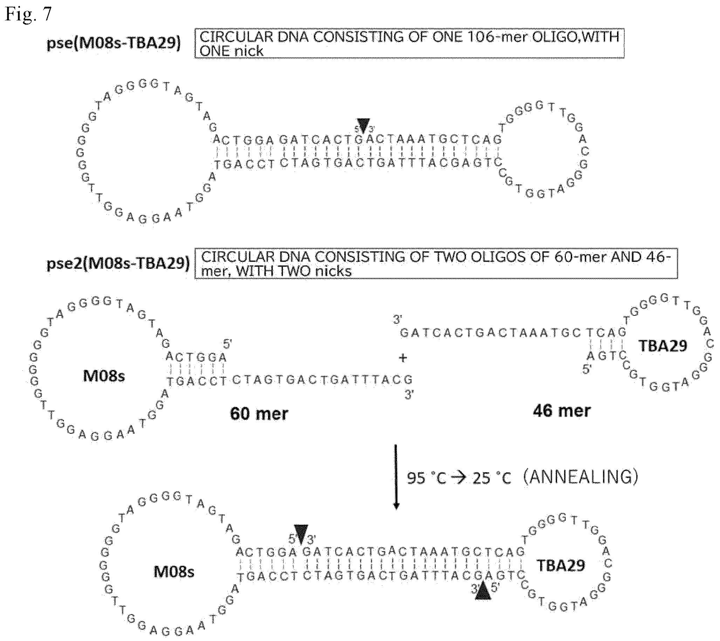

Fig. 7 shows a double-stranded pse2 (M08s-TBA29) corresponding to the single-stranded pse (M08s-TBA29), as one example of e.

The above double-stranded bivalent DNA aptamer includes:
the following double-stranded bivalent DNA aptamer using a 3'-end overhang,
consisting of:
   an oligonucleotide A comprising the sequence of 5'-(α)ₘ-AGGTCAGATGATGGGGATGGGGGGTTGGAGGAATGGATGACCT (SEQ ID NO: 15)-(β)ₘ-(X)ₙ-3', and
   an oligonucleotide B comprising the sequence of 5'-(γ)ₚ-AGTCCGTGGTAGGGCAGGTTGGGGTGACT (SEQ ID NO: 3)-(δ)ₚ-(Y)ₙ-3';
   cc1) 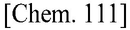 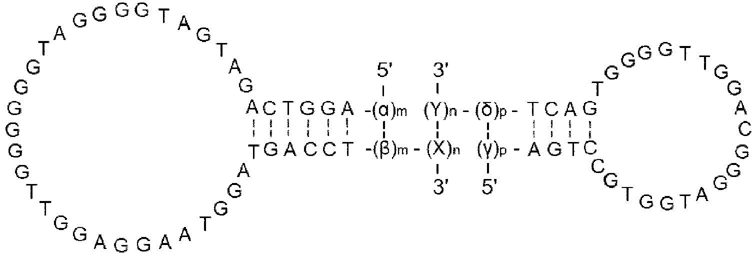 and
   the following double-stranded bivalent DNA aptamer using a 5'-end overhang,
   consisting of an oligonucleotide A comprising the sequence of 5'-(Y)ₙ-(α)ₘ-AGGTCAGATGATGGGGATGGGGGGTTGGAGGAATGGATGACCT-(β)ₘ -3', and
   an oligonucleotide B comprising the sequence of 5'-(X)ₙ-(γ)ₚ-AGTCCGTGGTAGGGCAGGTTGGGGTGACT-(δ)ₚ-3';
   dd1) 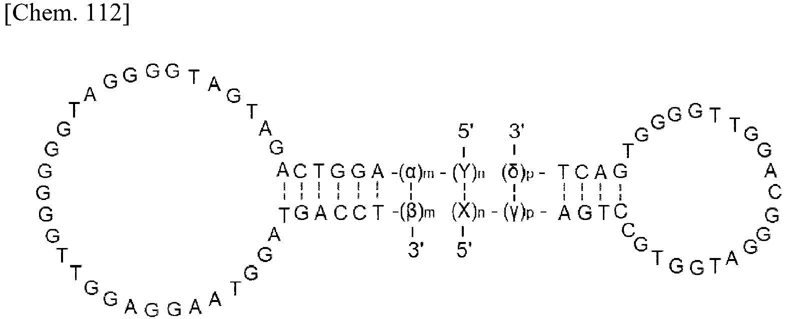
   wherein
   m, n, and p represent the numbers of DNA bases;
   m is an integer selected from the group consisting of 0, 1, 2, 3, 4, 5, 6, 7, 8, 9, 10, 11, 12, 13, 14, 15, 16, 17, 18, 19, and 20;
   n is an integer selected from the group consisting of 6, 7, 8, 9, 10, 11, 12, 13, 14, 15, 16, 17, 18, 19, 20, 21, 22, 22, 23, 24, and 25;
   p is an integer selected from the group consisting of 0, 1, 2, 3, 4, 5, 6, 7, 8, 9, 10, 11, 12, 13, 14, 15, 16, 17, 18, 19, and 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure;

The above double-stranded bivalent DNA aptamer includes:
the following double-stranded bivalent DNA aptamer using a 3'-end overhang,
consisting of:
   an oligonucleotide A comprising the sequence of 5'-(α)ₘ-AGATGATGGGGATGGGGGGTTGGAGGAATGGAT (SEQ ID NO: 18)-(β)ₘ-(X)ₙ-3', and
   an oligonucleotide B comprising the sequence of 5'-(γ)ₚ-AGATGATGGGGATGGGGGGTTGGAGGAATGGAT (SEQ ID NO: 18)-(δ)ₚ-(Y)ₙ-3';
   ee) 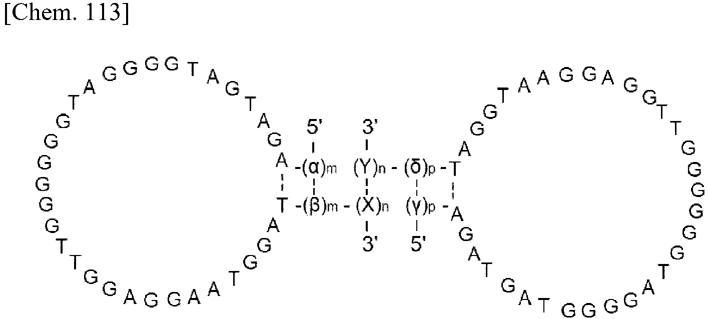 and
   the following double-stranded bivalent DNA aptamer using a 5'-end overhang, consisting of
   an oligonucleotide A comprising the sequence of 5'-(Y)ₙ-(α)ₘ-AGATGATGGGGATGGGGGGTTGGAGGAATGGAT-(β)ₘ-3', and
   an oligonucleotide B comprising the sequence of 5'-(X)ₙ-(γ)ₚ-AGATGATGGGGATGGGGGGTTGGAGGAATGGAT-(δ)ₚ-3';
   ff) 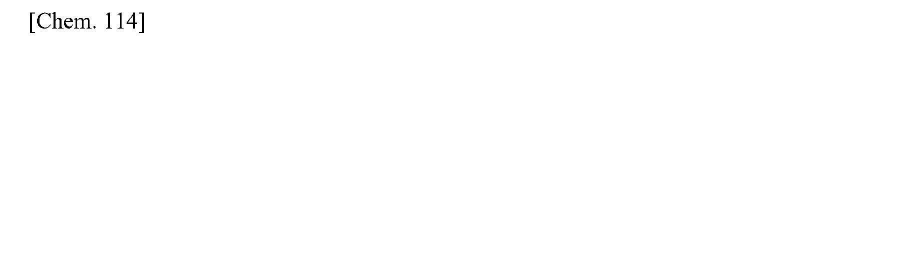 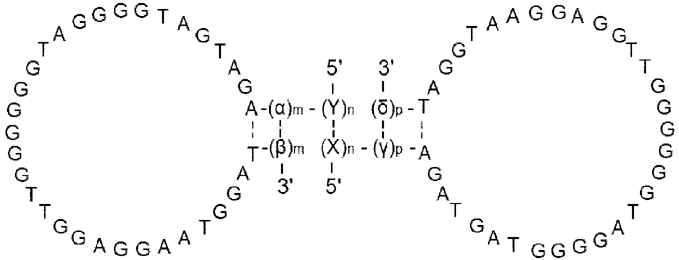
   wherein
   m, n, and p represent the numbers of DNA bases;
   m is an integer selected from the group consisting of 0, 1, 2, 3, 4, 5, 6, 7, 8, 9, 10, 11, 12, 13, 14, 15, 16, 17, 18, 19, 20, 21, 22, 22, 23, 24, and 25;
   n is an integer selected from the group consisting of 6, 7, 8, 9, 10, 11, 12, 13, 14, 15, 16, 17, 18, 19, 20, 21, 22, 22, 23, 24, and 25;
   p is an integer selected from the group consisting of 0, 1, 2, 3, 4, 5, 6, 7, 8, 9, 10, 11, 12, 13, 14, 15, 16, 17, 18, 19, 20, 21, 22, 22, 23, 24, and 25;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

The above double-stranded bivalent DNA aptamer includes:
the following double-stranded bivalent DNA aptamer using a 3'-end overhang,
consisting of:
   an oligonucleotide A comprising the sequence of 5'-(α)ₘ-AGGTCAGATGATGGGGATGGGGGGTTGGAGGAATGGATGACCT (SEQ ID NO: 14)-(β)ₘ-(X)ₙ-3', and
   an oligonucleotide B comprising the sequence of 5'-(γ)ₚ-AGGTCAGATGATGGGGATGGGGGGTTGGAGGAATGGATGACCT (SEQ ID NO: 14)-(δ)ₘ-(Y)ₙ-3';
   ff1) 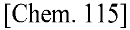 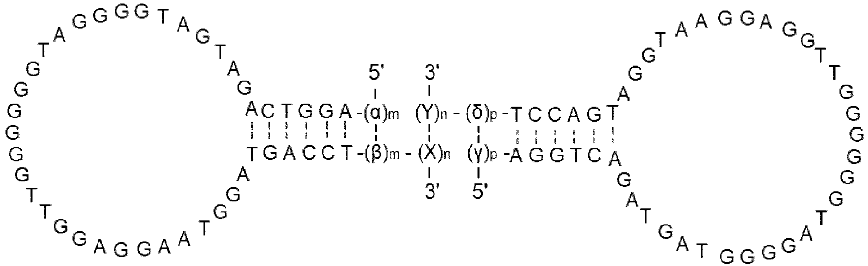 and
   the following double-stranded bivalent DNA aptamer using a 5'-end overhang,
   consisting of
   an oligonucleotide A comprising the sequence of 5'-(Y)ₙ-(α)ₘ-AGGTCAGATGATGGGGATGGGGGGTTGGAGGAATGGATGACCT-(β)ₘ-3', and
   an oligonucleotide B comprising the sequence of 5'-(X)ₙ-(γ)ₚ-AGGTCAGATGGTGGTCGGGGTGGTGGGATGAGGGTTCTGACCT-(δ)ₚ-3';
   ff1) 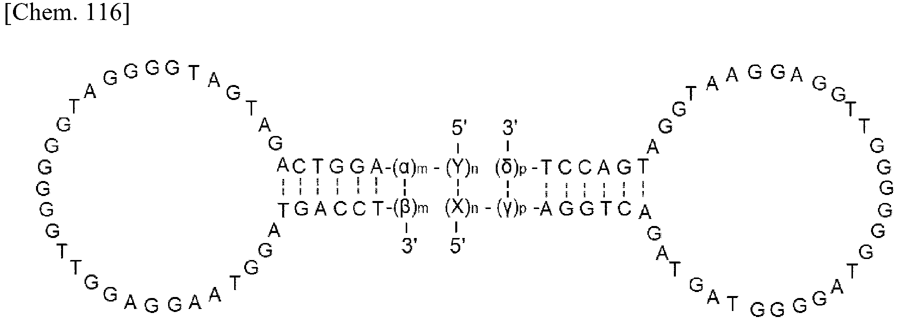
   wherein
   m, n, and p represent the numbers of DNA bases;
   m is 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

The above double-stranded bivalent DNA aptamer includes:
the following double-stranded bivalent DNA aptamer using a 3'-end overhang,
consisting of:
   an oligonucleotide A comprising the sequence of 5'-(α)ₘ-TGGGGATGGGGGGTTGGAGGAA-(β)ₘ-(X)ₙ-3', and
   an oligonucleotide B comprising the sequence of 5'-(γ)ₚ-ATGGTGGTCGGGGTGGTGGGATGAGGGTT-(δ)ₚ-(Y)ₙ-3';
   AA) 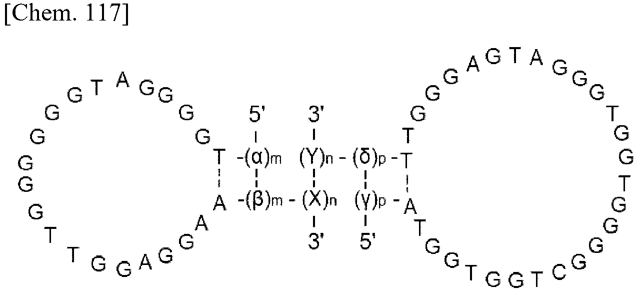 and
   the following double-stranded bivalent DNA aptamer using a 5'-end overhang,
   consisting of
   an oligonucleotide A comprising the sequence of 5'-(Y)ₙ-(α)ₘ-TGGGGATGGGGGGTTGGAGGAA-(β)ₘ-3', and
   an oligonucleotide B comprising the sequence of 5'-(X)ₙ-(γ)ₚ-ATGGTGGTCGGGGTGGTGGGATGAGGGTT-(δ)ₚ-3';
   BB) 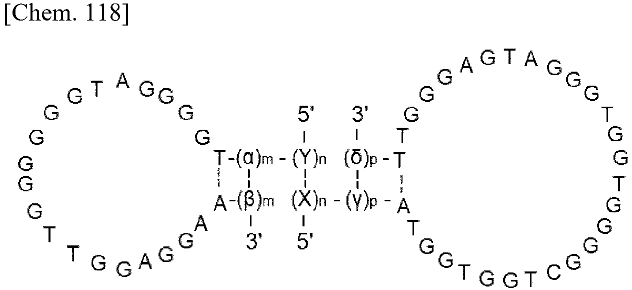
   wherein m, n, and p represent the numbers of DNA bases;
   m is an integer selected from the group consisting of 0 to 25;
   n is an integer selected from the group consisting of 6 to 25;
   p is an integer selected from the group consisting of 0 to 27;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

The above double-stranded bivalent DNA aptamer includes:
the following double-stranded bivalent DNA aptamer using a 3'-end overhang,
consisting of:
   an oligonucleotide A comprising the sequence of 5'-(α)ₘ-AGGTCAATGGGGATGGGGGGTTGGAGGAATTGACCT-(β)ₘ-(X)ₙ-3', and
   an oligonucleotide B comprising the sequence of 5'-(γ)ₚ-AGGTCAGATGGTGGTCGGGGTGGTGGGATGAGGGTTCTGACCT-(δ)ₚ-(Y)ₙ-3';
   AA1) 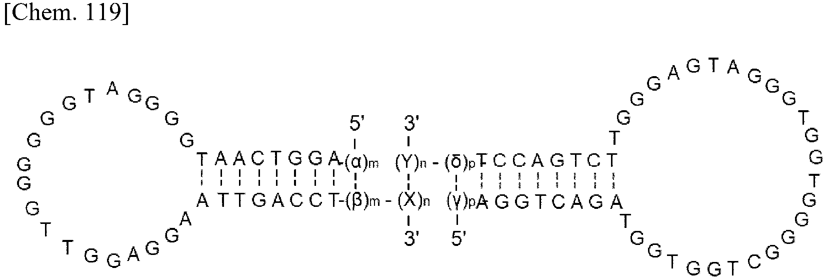 and
   the following double-stranded bivalent DNA aptamer using a 5'-end overhang, consisting of
   an oligonucleotide A comprising the sequence of 5'-(Y)ₙ-(α)ₘ-AGGTCAATGGGGATGGGGGGTTGGAGGAATTGACCT-(β)ₘ-3', and
   an oligonucleotide B comprising the sequence of 5'-(X)ₙ-(γ)ₚ-AGGTCAGATGGTGGTCGGGGTGGTGGGATGAGGGTTCTGACCT-(δ)ₚ-3';
   BB1) 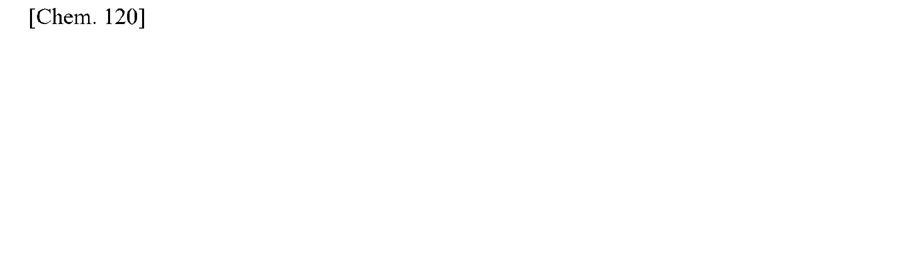 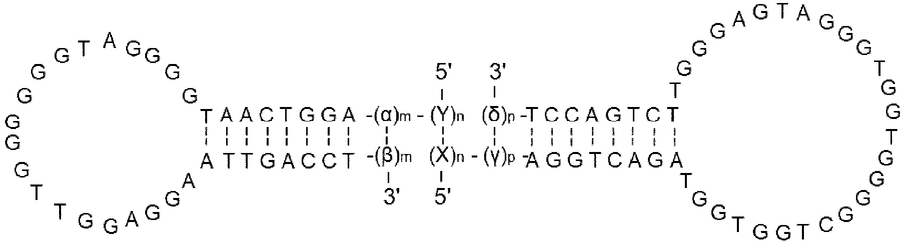
   wherein
   m, n, and p represent the numbers of DNA bases;
   m is an integer selected from the group consisting of 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

The above double-stranded bivalent DNA aptamer includes:
the following double-stranded bivalent DNA aptamer using a 3'-end overhang,
consisting of:
   an oligonucleotide A comprising the sequence of 5'-(α)ₘ-TGGGGATGGGGGGTTGGAGGAA-(β)ₘ-(X)ₙ-3', and
   an oligonucleotide B comprising the sequence of 5'-(γ)ₚ-CCGTGGTAGGGCAGGTTGGGGTG-(δ)ₚ-(Y)ₙ-3';
   CC) 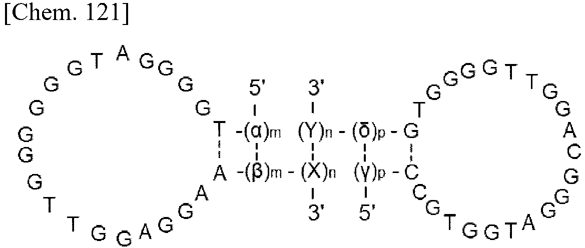 and
   the following double-stranded bivalent DNA aptamer using a 5'-end overhang,
   consisting of
   an oligonucleotide A comprising the sequence of 5'-(Y)ₙ-(α)ₘ-TGGGGATGGGGGGTTGGAGGAA-(β)ₘ-(X)ₙ-3', and
   an oligonucleotide B comprising the sequence of 5'-(X)ₙ-(γ)ₚ-CGTGGTAGGGCAGGTTGGGGT-(δ)ₚ-3';
   DD) 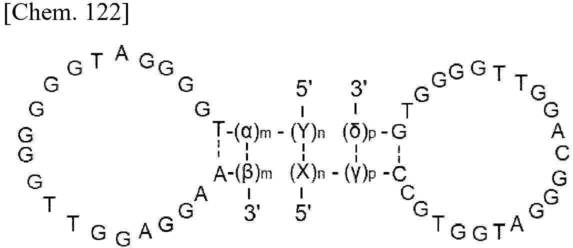
   wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 23;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

The above double-stranded bivalent DNA aptamer includes:
the following double-stranded bivalent DNA aptamer using a 3'-end overhang,
consisting of:
   an oligonucleotide A comprising the sequence of 5'-(α)ₘ-AGGTCAATGGGGATGGGGGGTTGGAGGAATTGACCT-(β)ₘ-(X)ₙ-3', and
   an oligonucleotide B comprising the sequence of 5'-(γ)ₚ-AGTCCGTGGTAGGGCAGGTTGGGGTGACT-(δ)ₚ-(Y)ₙ-3';
   CCl) 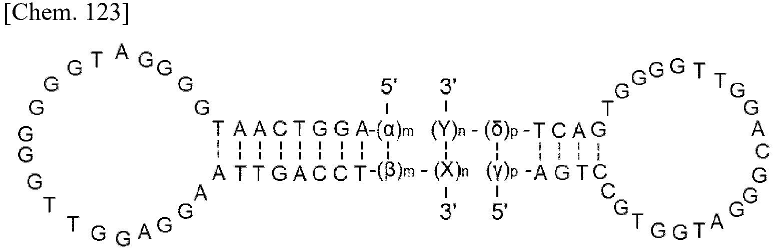 and
   the following double-stranded bivalent DNA aptamer using a 5'-end overhang,
   consisting of
   an oligonucleotide A comprising the sequence of 5'-(Y)ₙ-(α)ₘ-AGGTCAATGGGGATGGGGGGTTGGAGGAATTGACCT-(β)ₘ-(X)ₙ-3', and
   an oligonucleotide B comprising the sequence of 5'-(X)ₙ-(γ)ₚ-AGTCCGTGGTAGGGCAGGTTGGGGTGACT-(δ)ₚ-3';
   DD1) 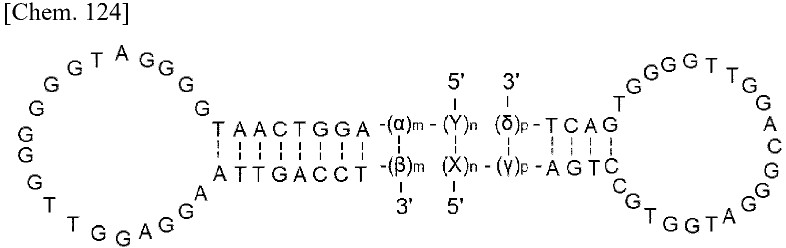
   wherein
   m, n, and p represent the numbers of DNA bases;
   m is 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

The above double-stranded bivalent DNA aptamer includes:
the following double-stranded bivalent DNA aptamer using a 3'-end overhang,
consisting of:
   an oligonucleotide A comprising the sequence of 5'-(α)ₘ-TGGGGATGGGGGGTTGGAGGAA-(β)ₘ-(X)ₙ-3', and
   an oligonucleotide B comprising the sequence of 5'-(γ)ₚ-TGGGGATGGGGGGTTGGAGGAA-(δ)ₚ-(Y)ₙ-3';
   EE) 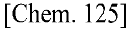 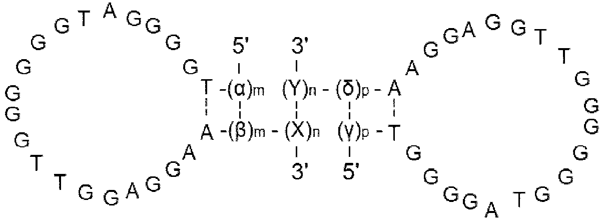 and
   the following double-stranded bivalent DNA aptamer using a 5'-end overhang,
   consisting of
   an oligonucleotide A comprising the sequence of 5'-(Y)ₙ-(α)ₘ-TGGGGATGGGGGGTTGGAGGAA-(β)ₘ-3', and
   an oligonucleotide B comprising the sequence of 5'-(X)ₙ-(γ)ₚ-TGGGGATGGGGGGTTGGAGGAA-(δ)ₚ-3';
   FF) 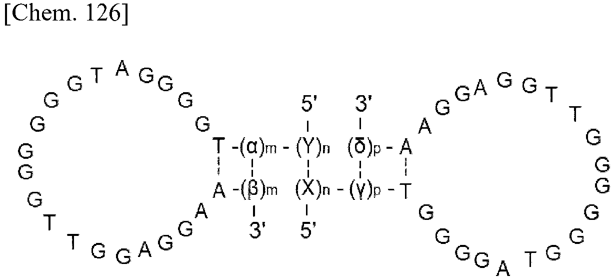
   wherein
   m, n, and p represent the numbers of DNA bases;
   m is an integer selected from the group consisting of 0 to 25;
   n is an integer selected from the group consisting of 6 to 25;
   p is an integer selected from the group consisting of 0 to 25;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

The above double-stranded bivalent DNA aptamer includes:
the following double-stranded bivalent DNA aptamer using a 3'-end overhang,
consisting of:
   an oligonucleotide A comprising the sequence of 5'-(α)ₘ-AGGTCAATGGGGATGGGGGGTTGGAGGAATTGACCT-(β)ₘ-(X)ₙ-3', and
   an oligonucleotide B comprising the sequence of 5'-(γ)ₚ-AGGTCAATGGGGATGGGGGGTTGGAGGAATTGACCT-(δ)ₚ-(Y)ₙ-3';
   EE1) 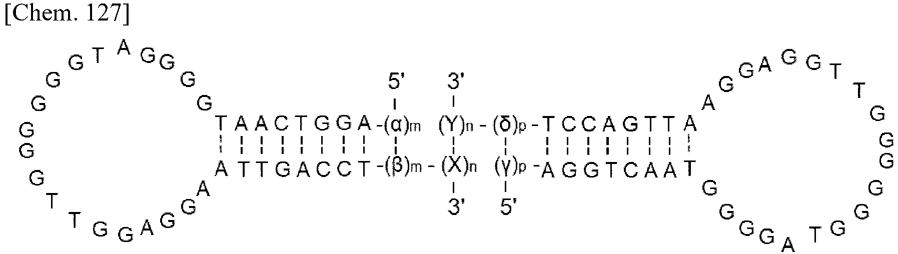 and
   the following double-stranded bivalent DNA aptamer using a 5'-end overhang,
   consisting of
   an oligonucleotide A comprising the sequence of 5'-(Y)ₙ-(α)ₘ-AGGTCAATGGGGATGGGGGGTTGGAGGAATTGACCT-(β)ₘ-3', and
   an oligonucleotide B comprising the sequence of 5'-(X)ₙ-(γ)ₚ-AGGTCAATGGGGATGGGGGGTTGGAGGAATTGACCT-(δ)ₚ-3';
   FF1) 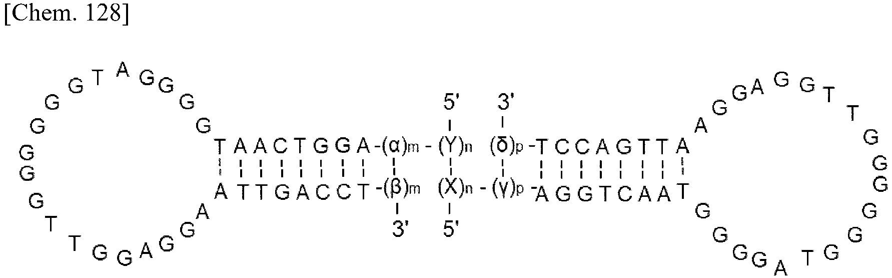
   wherein
   m, n, and p represent the numbers of DNA bases;
   m is an integer selected from the group consisting of 0 to 20;
   n is an integer selected from the group consisting of 6 to 25;
   p is an integer selected from the group consisting of 0 to 20;
   X, Y, α, β, γ, and δ are bases;
   5'-(X)ₙ-3' and 5'-(Y)ₙ-3' form an antiparallel double-stranded structure;
   5'-(α)ₘ-3' and 5'-(β)ₘ-3' form an antiparallel double-stranded structure; and
   5'-(γ)ₚ-3' and 5'-(δ)ₚ-3' form an antiparallel double-stranded structure.

In the double-stranded bivalent DNA aptamer, 5'-(X)ₙ-3' and 5'-(Y)ₙ-3' correspond to the above X sequence structure and the Y sequence structure that form an antiparallel base pair, respectively, 5'-(α)ₘ-3' and 5'-(β)ₘ-3' correspond to the α sequence structure and the β sequence structure that form an antiparallel base pair (dashed lines may refer to hydrogen bonds, and covalent bonds may be indicated by solid lines), and 5'-(γ)ₚ-3' and 5'-(δ)ₚ-3' correspond to the γ sequence structure and the δ sequence structure that form a base pair.

Although not particularly limited, the length of the duplex DNA moiety (i.e., m + n + p) is preferably 1, 2, 3, 4, 5, 6, 7, 8, 9, 10, 11, 12, 13, 14, 15, 16, 17, 18, 19, 20, 21, 22, 23, 24, or 25, more preferably 5 to 25, and further preferably 5 to 17.

The aspects of the above aa1), bb1), cc1), dd1), ee1), and ff1); as well as AA1), BB1), CC1), DD1), EE1), and FF1) may include an aspect in which the sequence of 5'-(β)ₘ-(X)ₙ-(γ)ₚ-3' is 5'-CTAGTGACTGATTTACG-3' (SEQ ID NO: 24), and the sequence of 5'-(δ)ₚ-(Y)ₙ-(α)ₘ-3' is 5'-CGTAAATCAGTCACTAG-3' (SEQ ID NO: 25).

The aspects of the above aa1), bb1), cel), dd1), ee1), and ff1); as well as AA1), BB1), CC1), DD1), EE1), and FF1) may include an aspect in which m is 0, n is 17, and p is 0.

When the aspect is such that the sequence of 5'-(β)ₘ-(X)ₙ-(γ)ₚ-3' is 5'-CTAGTGACTGATTTACG-3', and the sequence of 5'-(δ)ₚ-(Y)ₙ-(α)ₘ-3' is 5'-CGTAAATCAGTCACTAG-3', and m is 0, n is 17, and p is 0, then the sequence of 5'-(X)ₙ-3' is 5'-CTAGTGACTGATTTACG-3', and the sequence of 5'-(Y)ₙ-3' is 5'-CGTAAATCAGTCACTAG-3'.

The above double-stranded bivalent DNA aptamer includes
the DNA aptamer in a double-stranded form and consisting of a combination of oligonucleotides selected from the group consisting of the following 1 to 40 combinations of oligonucleotides A and oligonucleotides B:

**[Table 2]**

| Combination | Oligonucleotide A | | Oligonucleotide B | |
|---|---|---|---|---|
| | Name | SEQ ID NO: | Name | SEQ ID NO: |
| 1 | Sticky end20A-1 | 26 | Sticky end20A-2 | 27 |
| 2 | Sticky end20B-1 | 28 | Sticky end20B-2 | 29 |
| 3 | Sticky end20C-1 | 30 | Sticky end20C-2 | 31 |
| 4 | Sticky end20D-1 | 32 | Sticky end20D-2 | 33 |
| 5 | Sticky end20E-1 | 34 | Sticky end20E-2 | 35 |
| 6 | Sticky end19A-1 | 36 | Sticky end19A-2 | 37 |
| 7 | Sticky end19B-1 | 38 | Sticky end19B-2 | 39 |
| 8 | Sticky end19C-1 | 40 | Sticky end19C-2 | 41 |
| 9 | Sticky end19D-1 | 42 | Sticky end19D-2 | 43 |
| 10 | Sticky end19E-1 | 44 | Sticky end19E-2 | 45 |
| 11 | Sticky end18A-1 | 46 | Sticky end18A-2 | 47 |
| 12 | Sticky end18B-1 | 48 | Sticky end18B-2 | 49 |
| 13 | Sticky end18C-1 | 50 | Sticky end18C-2 | 51 |
| 14 | Sticky end18D-1 | 52 | Sticky end18D-2 | 53 |
| 15 | Sticky end18E-1 | 54 | Sticky end18E-2 | 55 |
| 16 | Sticky end17A-1 | 56 | Sticky end17A-2 | 57 |
| 17 | Sticky end17B-1 | 58 | Sticky end17B-2 | 59 |
| 18 | Sticky end17C-1 | 60 | Sticky end17C-2 | 61 |
| 19 | Sticky end17D-1 | 62 | Sticky end17D-2 | 63 |
| 20 | Sticky end17E-1 | 64 | Sticky end17E-2 | 65 |
| 21 | Sticky end16A-1 | 66 | Sticky end16A-2 | 67 |
| 22 | Sticky end16B-1 | 68 | Sticky end16B-2 | 69 |
| 23 | Sticky end16C-1 | 70 | Sticky end16C-2 | 71 |
| 24 | Sticky end16D-1 | 72 | Sticky end16D-2 | 73 |
| 25 | Sticky end16E-1 | 74 | Sticky end16E-2 | 75 |
| 26 | Sticky end15A-1 | 76 | Sticky end15A-2 | 77 |
| 27 | Sticky end15B-1 | 78 | Sticky end15B-2 | 79 |
| 28 | Sticky end15C-1 | 80 | Sticky end15C-2 | 81 |
| 29 | Sticky end15D-1 | 82 | Sticky end15D-2 | 83 |
| 30 | Sticky end15E-1 | 84 | Sticky end15E-2 | 85 |
| 31 | Sticky end14A-1 | 86 | Sticky end14A-2 | 87 |
| 32 | Sticky end14B-1 | 88 | Sticky end14B-2 | 89 |
| 33 | Sticky end14C-1 | 90 | Sticky end14C-2 | 91 |
| 34 | Sticky end14D-1 | 92 | Sticky end14D-2 | 93 |
| 35 | Sticky end14E-1 | 94 | Sticky end14E-2 | 95 |
| 36 | Sticky end13A-1 | 96 | Sticky end13A-2 | 97 |
| 37 | Sticky end13B-1 | 98 | Sticky end13B-2 | 99 |
| 38 | Sticky end13C-1 | 100 | Sticky end13C-2 | 101 |
| 39 | Sticky end13D-1 | 102 | Sticky end13D-2 | 103 |
| 40 | Sticky end13E-1 | 104 | Sticky end13E-2 | 105 |

The present invention includes pharmaceutical use of the above aptamers.

The aptamer of the present invention can be formulated as a pharmaceutical composition comprising a therapeutically effective amount thereof and a pharmaceutically acceptable carrier. Examples of the pharmaceutically acceptable carrier can include binders (e.g., hydroxypropyl cellulose, polyvinyl alcohol, polyvinylpyrrolidone, and polyethylene glycol), excipients (e.g., lactose, sucrose, mannitol, sorbitol, corn starch, potato starch, crystalline cellulose, and calcium carbonate), lubricants (e.g., magnesium stearate, calcium stearate, and talc), disintegrants (e.g., low-substituted hydroxypropyl cellulose and cross-linked carboxymethyl cellulose), and humectants (e.g., sodium lauryl sulfate).

The pharmaceutical composition of the present invention can be parenterally administered, and can be used as an appropriate pharmaceutical preparation. Examples of the appropriate pharmaceutical preparation for parenteral administration can include suppositories, injection or infusion using injectable distilled water, physiological saline, aqueous dextrose solution, or the like, or inhalants.

The dosage of the pharmaceutical composition of the present invention can vary depending on the mode of administration, and the age, body weight, and the condition of the patient, and the daily dosage is 0.001 to 100 mg/kg, preferably 0.1 to 30 mg/kg, and more preferably 0.1 to 10 mg/kg, which can be administered once or divided in 2 to 4 times.

The pharmaceutical composition of the present invention can be expected to use for diseases related to blood coagulation, such as cerebral infarction, congenital antithrombin III deficiency, and heparin-induced thrombocytopenia. It can also be used as an anticoagulant during coronary artery bypass surgery or extracorporeal blood circulation. In addition, it can be the first selective drug or auxiliary agent for alternative anticoagulants when bleeding tendency is caused by excessive intake of antithrombotic drugs required not only during the perioperative period but also after bypass, stent, and artificial valve surgery, renal failure, drug interactions, and increased blood concentration due to dehydration, and the like.

Symptoms caused by a blood clot, known as thrombus in a blood vessel are called thrombosis in its broad sense (hereinafter, when simply written as "thrombosis", it refers to thrombosis in the broad sense), and pathological conditions caused by a thrombus are classified into thrombosis in the narrow sense and embolism. The thrombosis in the narrow sense is a symptom caused by a thrombus partially or completely blocking blood flow at the site of its formation; whereas the embolism refers to a pathological condition caused by a thrombus breaking free from its formation site and traveling through blood flow, and then partially or completely blocking blood flow at another site. Such thrombosis can induce various diseases depending on the location of the blood vessel where the thrombus formed. When it occurs in a cerebral blood vessel or a cardiac blood vessel, among others, it can cause a pathological condition such as cerebral infarction and myocardial infarction, which have high mortality rates, and even after survival, it can lead to irreversible impairment such as hemiplegia and the onset of functional decline. Currently, research and development have been conducted mainly on antithrombotic drugs that suppress the formation of thrombi, and thrombolytic agents that dissolve the formed thrombi in order to solve thrombosis.

The present invention includes a neutralizing agent for the above pharmaceutical composition.

Such a neutralizing agent contains, as a main component, a nucleic acid (including DNA, RNA, PNA and a composite thereof) that has full or partial complementarity to the sequence of the DNA aptamer that binds to exosite I of thrombin or the DNA aptamer that binds to exosite II.

In an embodiment of the present invention, the neutralizing agent contains a part or full of the DNA aptamer that binds to exosite I of thrombin of the bivalent aptamer of the present invention (or I-1, II-1, I-2, II-2); and a nucleic acid (including DNA, RNA, PNA and a composite thereof) that has the complementarity to a part or full of the duplex DNA.

In an embodiment of the present invention, the neutralizing agent contains a nucleic acid comprising a complementary sequence to 5'-AGGTCAGATGATGGGGATGGG-3' (SEQ ID NO: 21).

In an embodiment of the present invention, the neutralizing agent contains a nucleic acid comprising a sequence described in [L-M08s]c (SEQ ID NO: 17) or 08ad (SEQ ID NO: 15).

In an embodiment of the present invention, the neutralizing agent for the DNA aptamer comprising the sequence of M08s-1 (36) (SEQ ID NO: 107) is a nucleic acid comprising a part or full of the complementary sequence to M08s-1 (36) (SEQ ID NO: 107).

In an embodiment of the present invention, the neutralizing agent for the DNA aptamer comprising the sequence of M08s-1 (36) (SEQ ID NO: 107) may contain a nucleic acid comprising a sequence selected from the group consisting of:
Ant-M08_36-36 (SEQ ID NO: 142);
Ant-M08_36-30 (SEQ ID NO: 143);
Ant-M08_36-24 (SEQ ID NO: 144);
Ant-M08_36-18 (SEQ ID NO: 145);
Ant-M08_36-L18 (SEQ ID NO: 146);
Ant-M08_36-R18 (SEQ ID NO: 147); and
Ant-M08_36-12 (SEQ ID NO: 148).

By administering such a neutralizing agent, the ability to inhibit blood coagulation due to the administration of the bivalent aptamer according to the present invention is suppressed and inhibited, and the blood coagulation reaction in the living body is promoted. The neutralizing agent may be administered locally to body tissue where blood coagulation inhibition is not desired to occur.

The present invention includes a method for producing a bivalent aptamer excellent in the ability to inhibit blood coagulation.

Test oligonucleotides having the above structures (e.g., a) to f) for single-stranded types; aa) to ff) and AA) to FF) for double-stranded types) are prepared or provided;
the ability to inhibit blood coagulation is measured by a method such as that described in the following Examples (e.g., APTT measurement), and
it is compared to the ability of a reference aptamer or a reagent to inhibit blood coagulation, thereby the one with a higher effect can be used as an excellent bivalent aptamer for formulation.

The reference aptamer is preferably an aptamer excellent in the ability to inhibit blood coagulation. Although not particularly limited, a polyA linker-bound bivalent aptamer (e.g., M08a-A15-TBA29 or M08a-A5-M03a described in Patent Literature 1, and HD1-15dA-60.29 described in Non Patent Literature 1), a polyT linker-bound bivalent aptamer (such as Lin(M08s-TBA29) in the present specification), and the like can be used as the reference aptamer. Since there is a report that the polyT linker-bound bivalent aptamer is in the ability to inhibit blood coagulation superior to the polyA linker-bound bivalent aptamer (Non Patent Literature 4), the polyT linker-bound bivalent aptamer is more preferable as the reference aptamer.

Examples of the direct thrombin inhibitor in clinical use can include lowmolecular weight, argatroban, dabigatran, and bivalirudin, which is a peptide.

### Examples

Hereinafter, the present invention will be described in more detail using Examples; however, the Examples are not intended to limit the scope of the present invention in any way.

### <Example 1> Examination of Length of Double-Stranded Linker Suitable for Anticoagulation Effect by Thrombin Inhibition

### 1. Synthesis of Aptamer

A single-stranded bivalent aptamers comprising M08s, an exosite I-binding aptamer, and TBA29 an exosite II-binding aptamer, linked by duplex DNA of various lengths were synthesized (SEQ ID NOs: 4 to 10).

**[Table 3]**

| Sequence of DNA aptamer | |
|---|---|
| Length of duplex DNA | Sequence |
| 0bp | AGGTCAGATGATGGGGATGGGGGGTTGGAGGAATGGATGACCT AGTCCGTGGTAGGGCAGGTTGGGGTGACT |
| TA-5bp | TAAGGTCAGATGATGGGGATGGGGGGTTGGAGGAATGGATGAC CTTATATAGTCCGTGGTAGGGCAGGTTGGGGTGACTATA |
| TA-17bp | TATATATAAGGTCAGATGATGGGGATGGGGGGTTGGAGGAATG GATGACCTTATATATATATATATATAGTCCGTGGTAGGGCAGG TTGGGGTGACTATATATATA |
| TA-21bp | TATATATATAAGGTCAGATGATGGGGATGGGGGGTTGGAGGAA TGGATGACCTTATATATATATATATATATATAGTCCGTGGTAG GGCAGGTTGGGGTGACTATATATATATA |
| ATGC-5bp | AAAGGTCAGATGATGGGGATGGGGGGTTGGAGGAATGGATGAC CTTTACGAGTCCGTGGTAGGGCAGGTTGGGGTGACTCGT |
| ATGC-17bp | GTCACTAGAGGTCAGATGATGGGGATGGGGGGTTGGAGGAATG GATGACCTCTAGTGACTGATTTACGAGTCCGTGGTAGGGCAGG TTGGGGTGACTCGTAAATCA |
| ATGC-21bp | CACTAGACTGAGGTCAGATGATGGGGATGGGGGGTTGGAGGAA TGGATGACCTCAGTCTAGTGACTGATTTACGAGTCCGTGGTAG GGCAGGTTGGGGTGACTCGTAAATCAGT |

### 2. Measurement of Thrombin Coagulation Inhibition Ability

Thrombin was dissolved in PBS, and its concentration was adjusted to 1 µM. Each aptamer was adjusted to 5 µM using PBS followed by heating at 95°C for 3 minutes using a dry bath incubator (Major Science Co., Ltd. Taiwan), and then immediately cooled in a heat block stored at 25°C in advance to perform annealing. To the mixture, 100 µL of 12.5 nM aptamer and 100 µL of 6.25 nM thrombin were added and incubated at room temperature for 15 minutes. To a well of Costar Assay Plate 96 well (Corning Incorporated, NY, USA), 80 µL each of the mixed solution of aptamer and thrombin was added, and then, 20 µL of fibrinogen solution was added using an 8-channel pipetter. Immediately after that, under the following conditions:
Aptamer : 5.0 nM
Human thrombin : 2.5 nM
Human fibrinogen : 1200 nM
PBS (Vehicle)
Temperature : 25°C
Mean value of two measurements
the absorbance was measured at 350 nm using a UV-Vis microplate reader (Viento nano, Biotek, Vermont, USA).

### 3. Results

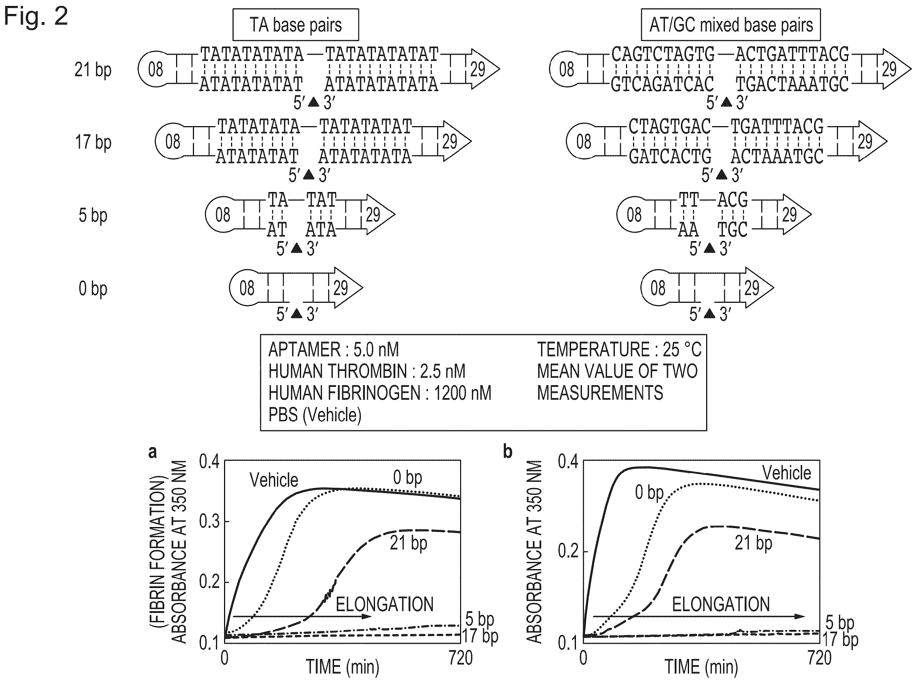

The results are shown in Fig. 2. The ability to inhibit thrombin coagulation significantly varied depending on the length of duplex DNA. Fig. 2 indicates that the ability to inhibit thrombin coagulation decreases when the length is too long or too short, and that the length of the duplex DNA has a critical importance.

### <Example 2> Evaluation Results of Anticoagulation Effect in Human Plasma or in Plasma After Administration of Compound to Animal by Activated Partial Thromboplastin Time (hereinafter, also referred to as APTT) Test

### 1. Synthesis of Aptamer

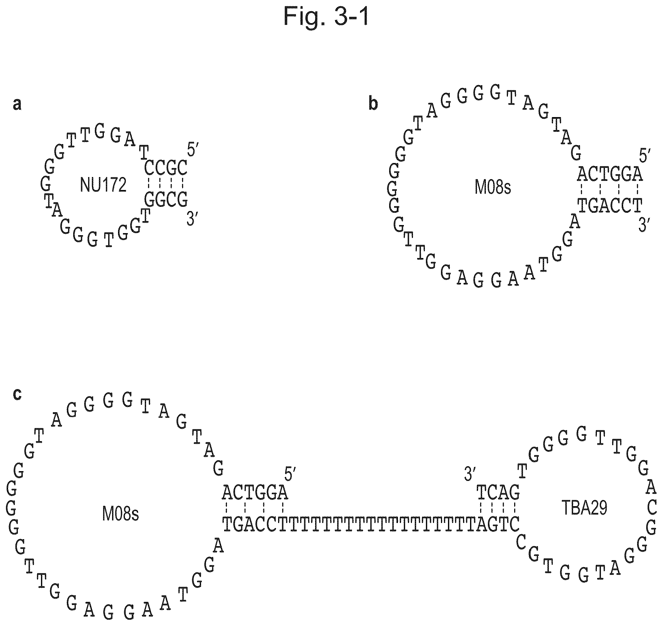

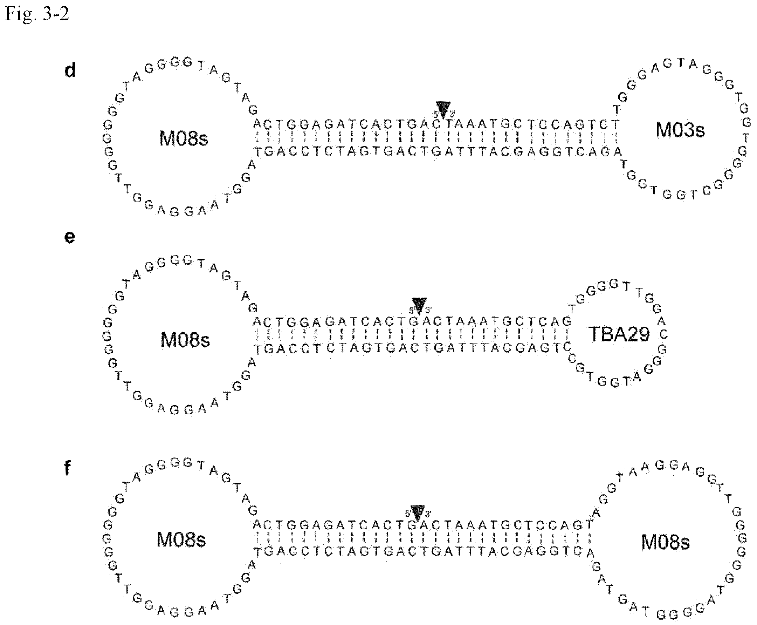

Monovalent aptamers or bivalent aptamers described in Fig. 3 and the following Table 2 were synthesized (SEQ ID NOs: 11 to 13, 22, 2, and 14).

**[Table 4]**

| Aptamer | Sequence information |
|---|---|
| lin (M08s-TBA29) | AGGTCAGATGATGGGGATGGGGGGTTGGAGGAAT GGATGACCTTTTTTTTTTTTTTTTTTAGTCCGTG GTAGGGCAGGTTGGGGTGACT |
| pse (M08s-M03s) | CAGTCACTAGAGGTCAGATGATGGGGATGGGGGG TTGGAGGAATGGATGACCTCTAGTGACTGATTTA CGAGGTCAGATGGTGGTCGGGGTGGTGGGATGAG GGTTCTGACCTCGTAAAT |
| pse (M08s-TBA29) | GTCACTAGAGGTCAGATGATGGGGATGGGGGGTT GGAGGAATGGATGACCTCTAGTGACTGATTTACG AGTCCGTGGTAGGGCAGGTTGGGGTGACTCGTAA ATCA |
| pse (M08s-M08s) | GTCACTAGAGGTCAGATGATGGGGATGGGGGGTT GGAGGAATGGATGACCTCTAGTGACTGATTTACG AGGTCAGATGATGGGGATGGGGGGTTGGAGGAAT GGATGACCTCGTAAATCA |
| NU172 | CGCCTAGGTTGGGTAGGGTGGTGGCG |
| M08s | AGGTCAGATGATGGGATGGGGGTTGGAGGAAT GGATGACCT |

### 2. In vitro APTT Test of Aptamers in Human Plasma

The concentration of aptamers was adjusted to 150 µM using PBS followed by heating at 95°C for 3 minutes using a dry bath incubator (Major Science Co., Ltd. Taiwan), and then immediately cooled in a heat block that had been left still at 25°C in advance to perform annealing. To a sample cup (Sysmex Co., Ltd., Japan), 50 µL of normal human plasma (George King Bio-Medical Inc., Kansas, USA) was added to incubate at 37°C for 1 minute. Next, 50 µL of an APTT reagent (Sysmex Co., Ltd., Japan) was added thereto to incubate at 37°C for 2 minutes, and then 50 µL of 0.2 M calcium chloride was added, and the APTT was immediately measured by tracking the time course of the scattered light intensity at 660 nm using an automated blood coagulation analyzer (Sysmex Co., Ltd., Japan).

### 3. APTT Tests Using Blood Samples Collected After Single Administration of Various Aptamers and Low Molecular Weight Argatroban to Mice

Each aptamer was adjusted to 150 µM using PBS followed by heating at 95°C for 3 minutes using a dry bath incubator (Major Science Co., Ltd. Taiwan), and then immediately cooled in a heat block that had been left still at 25°C in advance to perform annealing. Each aptamer was administered to the jugular vein of normal mice (22 g, 7-week old) anesthetized in advance at 0.04 µmol/kg or 0.13 µmol/kg, and blood was collected from the inferior vena cava after 3, 10, 30, or 60 minutes of standing. The collected blood was mixed with 3.8% sodium citrate (blood: 3.8% sodium citrate = 9:1, v/v). The blood was centrifuged at 1,500 G for 15 minutes with a centrifuge (Thermo Fisher Scientific, Inc., Massachusetts, USA), and then the plasma layer was collected. To a sample cup (Sysmex Co., Ltd., Japan), 50 µL of the plasma was added to incubate at 37°C for 1 minute. Next, 50 µL of an APTT reagent (Sysmex Co., Ltd., Japan) was mixed to incubate at 37°C for 2 minutes, and then, 50 µL of 0.2 M calcium chloride was added, and the APTT was immediately measured by tracking the time course of the scattered light intensity at 660 nm using an automated blood coagulation analyzer (Sysmex Co., Ltd., Japan).

### 4. Results

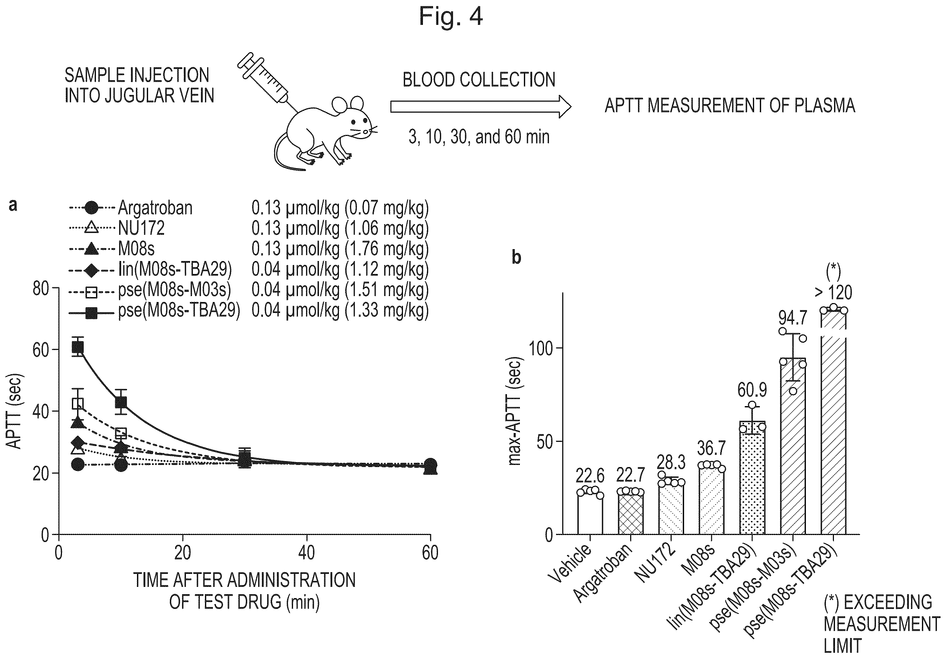

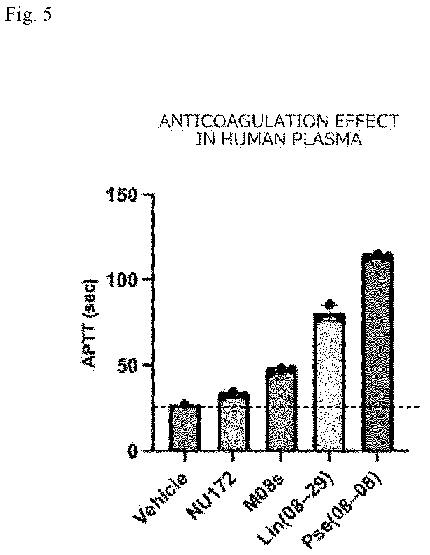

The results are shown in Figs. 4 and 5. The bivalent DNA aptamers according to the present invention (pse (M08s-M03s) (Fig. 4), pse (P08s-TBA29) (Fig. 4), and pse (M08s-M08s) (described as pse (08-08) in Fig. 5)) exhibited a higher anticoagulation effect than the bivalent DNA aptamer linked with the single-stranded DNA (lin (M08s-TBA29) (described as Lin(08-29) in Fig. 5)).

### <Example 3> Neutralizing Effect of Adding Oligonucleotide With Complementary Strand Sequence to Nucleic Acid Aptamer, pse (M08s-TBA29), in Human Plasma

### 1. APTT Tests Using pse (M-08s-TBA29) and Complementary Strand Sequence Oligonucleotide

The concentrations of the anti-coagulation apter pse (M08s-TBS29) and the complementary strand sequence oligonucleotides (08d (SEQ ID NO: 15), [M08s]c (SEQ ID NO: 16), and [L-08s]c (SEQ ID NO: 17) were each adjusted to 150 µM, and 1000 µM using PBS followed by heating at 95°C for 3 minutes using a dry bath incubator (Major Science Co., Ltd. Taiwan), and then immediately cooled in a heat block that had been left still at 25°C in advance to perform annealing. To a sample cup (Sysmex Co., Ltd., Japan), 145 µL of normal human plasma (George King Bio-Medical Inc., Kansas, USA), 60 µM of Pse (08-29), or 2.5 µL of 120, 240, 480 or 960 µM of the complementary strand sequence were added to incubate at 37°C for 1 minute. Next, 50 µL of an APTT reagent (Sysmex Co., Ltd., Japan) was mixed to incubate at 37°C for 2 minutes, and then, 50 µL of 0.2 M calcium chloride was added, and the APTT was immediately measured by tracking the time course of the scattered light intensity at 660 nm using an automated blood coagulation analyzer (Sysmex Co., Ltd., Japan) under the following conditions:
pse (M08s-TBA29) : 0.33 µM (1 eqv.)
Complementary strand sequence oligonucleotide : 2, 4, 8, or 16 eqv.
Normal human plasma : 32 v/v%
Temperature : 37°C
One measurement.

**[Table 5]**

| Complementary strand sequence oligonucleotide | Sequence information |
|---|---|
| 08ad | CCCCATCCCCATCATCTGACCTCTAGTGACTG |
| [M08s] c | CCCCATCCCCATCATCTGACCT |
| [L-M08s] c | CCCCATCCCCATCATCTGACCTCTAGTGAC |

### 2. Results

The results are shown in Fig. 6. Upon the addition of 08ad, the anticoagulation effect of pse(M08s-TBA29) was suppressed (Fig. 6b). [L-M08s]c, which lacks 2 bases at the 3'-end of 08ad, showed neutralizing effect similar to 08ad. Conversely, when a complementary strand is not present up to the double-stranded linker region, the drug effects could not completely be neutralized ([M08s]c, Fig. 6c).

This indicates that the neutralizing agent (complementary strand) sequence requires a complementary strand not only in the region of M08s, which exhibits the inhibitory effect, but also in the double-stranded structure region.

### <Reference Example 1> Dimerization Simulation by NUPACK

The secondary structure stability of the double-stranded bivalent aptamer was simulated by NUPACK.

Specifically, using the conditions of the oligonucleotide concentration of 10 µM, the NaCl concentration of 150 mM, and MgCl₂ concentration of 0.5 mM, temperature was lowered from 95°C to 5°C by 5°C, and the hybridization strength of the double strand at 35°C was examined. However, the sequence stabilization by the formation of G-quartet structure formed by M08s and TBA29 was not taken in consideration in the simulation.

The results are shown in Fig. 8. When the distance between nicks was set to 21 bp, where the aptamer regions was violated, the stem structure of the aptamer was distorted on the sides of M08s and TBA29 (△). When the distance between nicks was set to 17 bp, the stem structure of the aptamer was distorted on the side of TBA29 (△). When the distance between nicks was set to 13 bp or 9 bp, the expected dimer was predicted to be stably formed (∘). On the other hand, when the distance between nicks was set to 5 bp or less, it was found that the monomer formation was overwhelmingly dominant (×). Since the sequence stabilization by the formation of G-quartet structure formed by M08s and TBA29 was not taken in consideration in the simulation, even if the simulation result was △, it is quite possible that the folding occurs without any problems at an experimental level, and that the anticoagulation effect is exhibited.

### <Example 4> Evaluation of Anticoagulation Effect of Double-Stranded Bivalent Aptamer (M08s-TBA29: 13 to 20 liners) in Plasma

The following 1 to 40 combinations of oligonucleotides A and oligonucleotides B were used.

**[Table 6]**

| Combination | Oligonucleotide A | | Oligonucleotide B | |
|---|---|---|---|---|
| | Name | SEQ ID NO: | Name | SEQ ID NO: |
| 1 | Sticky end20A-1 | 26 | Sticky end20A-2 | 27 |
| 2 | Sticky end20B-1 | 28 | Sticky end20B-2 | 29 |
| 3 | Sticky end20C-1 | 30 | Sticky end20C-2 | 31 |
| 4 | Sticky end20D-1 | 32 | Sticky end20D-2 | 33 |
| 5 | Sticky end20E-1 | 34 | Sticky end20E-2 | 35 |
| 6 | Sticky end19A-1 | 36 | Sticky end19A-2 | 37 |
| 7 | Sticky end19B-1 | 38 | Sticky end19B-2 | 39 |
| 8 | Sticky end19C-1 | 40 | Sticky end19C-2 | 41 |
| 9 | Sticky end19D-1 | 42 | Sticky end19D-2 | 43 |
| 10 | Sticky end19E-1 | 44 | Sticky end19E-2 | 45 |
| 11 | Sticky end18A-1 | 46 | Sticky end18A-2 | 47 |
| 12 | Sticky end18B-1 | 48 | Sticky end18B-2 | 49 |
| 13 | Sticky end18C-1 | 50 | Sticky end18C-2 | 51 |
| 14 | Sticky end18D-1 | 52 | Sticky end18D-2 | 53 |
| 15 | Sticky end18E-1 | 54 | Sticky end18E-2 | 55 |
| 16 | Sticky end17A-1 | 56 | Sticky end17A-2 | 57 |
| 17 | Sticky end17B-1 | 58 | Sticky end17B-2 | 59 |
| 18 | Sticky end17C-1 | 60 | Sticky end17C-2 | 61 |
| 19 | Sticky end17D-1 | 62 | Sticky end17D-2 | 63 |
| 20 | Sticky end17E-1 | 64 | Sticky end17E-2 | 65 |
| 21 | Sticky end16A-1 | 66 | Sticky end16A-2 | 67 |
| 22 | Sticky end16B-1 | 68 | Sticky end16B-2 | 69 |
| 23 | Sticky end16C-1 | 70 | Sticky end16C-2 | 71 |
| 24 | Sticky end16D-1 | 72 | Sticky end16D-2 | 73 |
| 25 | Sticky end16E-1 | 74 | Sticky end16E-2 | 75 |
| 26 | Sticky end15A-1 | 76 | Sticky end15A-2 | 77 |
| 27 | Sticky end15B-1 | 78 | Sticky end15B-2 | 79 |
| 28 | Sticky end15C-1 | 80 | Sticky end15C-2 | 81 |
| 29 | Sticky end15D-1 | 82 | Sticky end15D-2 | 83 |
| 30 | Sticky end15E-1 | 84 | Sticky end15E-2 | 85 |
| 31 | Sticky end14A-1 | 86 | Sticky end14A-2 | 87 |
| 32 | Sticky end14B-1 | 88 | Sticky end14B-2 | 89 |
| 33 | Sticky end14C-1 | 90 | Sticky end14C-2 | 91 |
| 34 | Sticky end14D-1 | 92 | Sticky end14D-2 | 93 |
| 35 | Sticky end14E-1 | 94 | Sticky end14E-2 | 95 |
| 36 | Sticky end13A-1 | 96 | Sticky end13A-2 | 97 |
| 37 | Sticky end13B-1 | 98 | Sticky end13B-2 | 99 |
| 38 | Sticky end13C-1 | 100 | Sticky end13C-2 | 101 |
| 39 | Sticky endl3D-1 | 102 | Sticky end13D-2 | 103 |
| 40 | Sticky end13E-1 | 104 | Sticky end13E-2 | 105 |

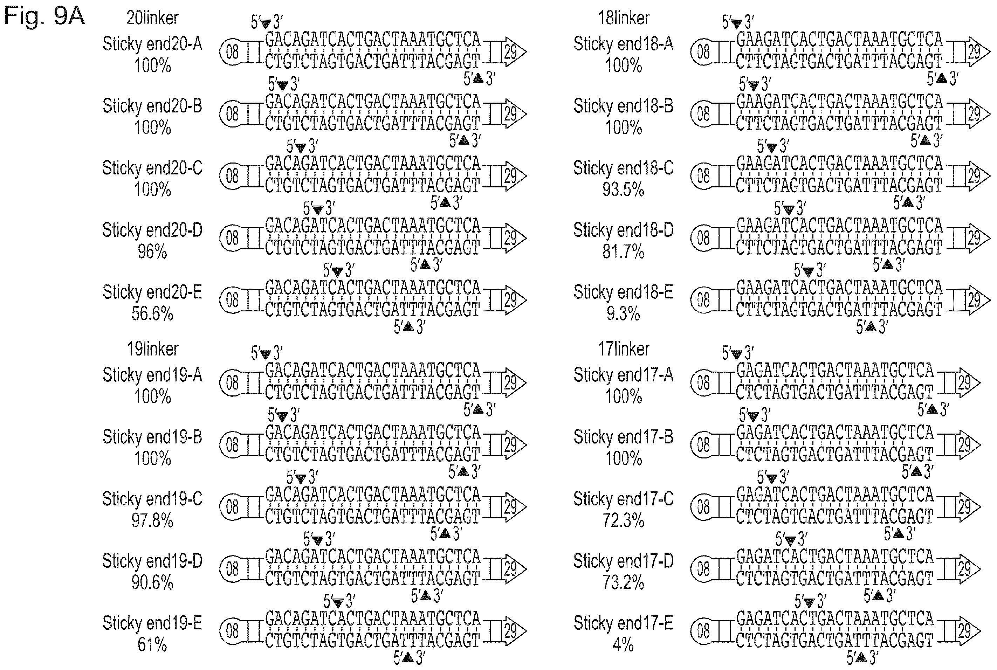

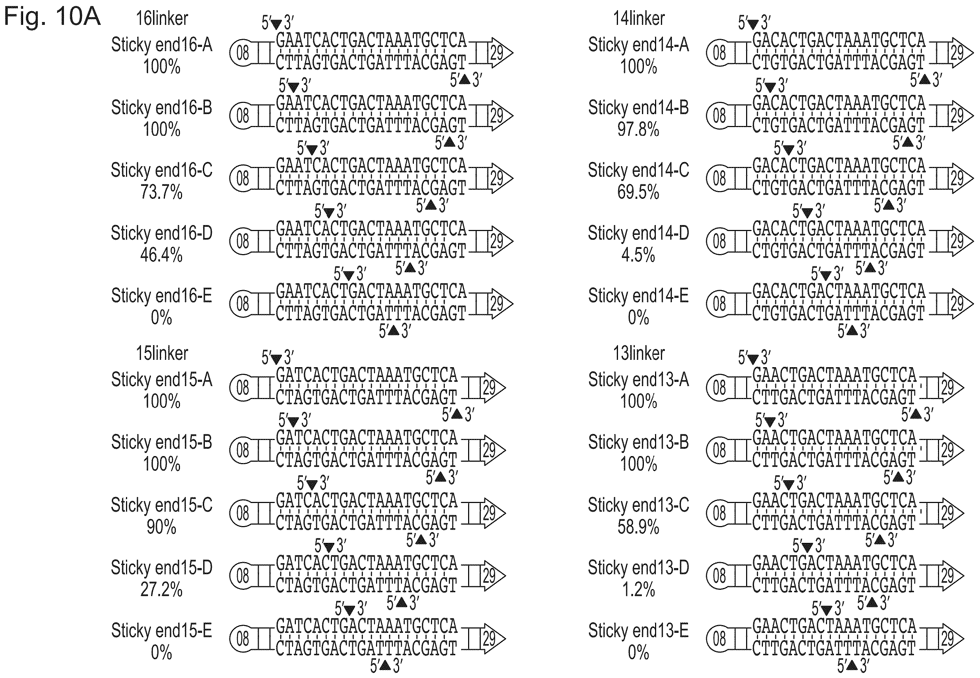

The concentration of each combination of oligonucleotides was adjusted to 150 µM using PBS followed by heating at 95°C for 3 minutes using a dry bath incubator (Major Science Co., Ltd. Taiwan), and then immediately cooled in a heat block that had been left still at 25°C in advance to perform annealing. The predicted schematic diagrams of the secondary structure are shown in Figs. 9A and 10A.

To a sample cup (Sysmex Co., Ltd., Japan), 50 µL of normal human plasma (George King Bio-Medical Inc., Kansas, USA) was added to incubate at 37°C for 1 minute. Next, 50 µL of an APTT reagent (Sysmex Co., Ltd., Japan) was added thereto to incubate at 37°C for 2 minutes, and then 50 µL of 0.2 M calcium chloride was added, and the APTT was immediately measured by tracking the time course of the scattered light intensity at 660 nm using an automated blood coagulation analyzer (Sysmex Co., Ltd., Japan) (final concentration of oligonucleotide: 1 µM). PBS and pse (M08s-TBA29) (SEQ ID NO: 13) were used as a negative control and a positive control, respectively.

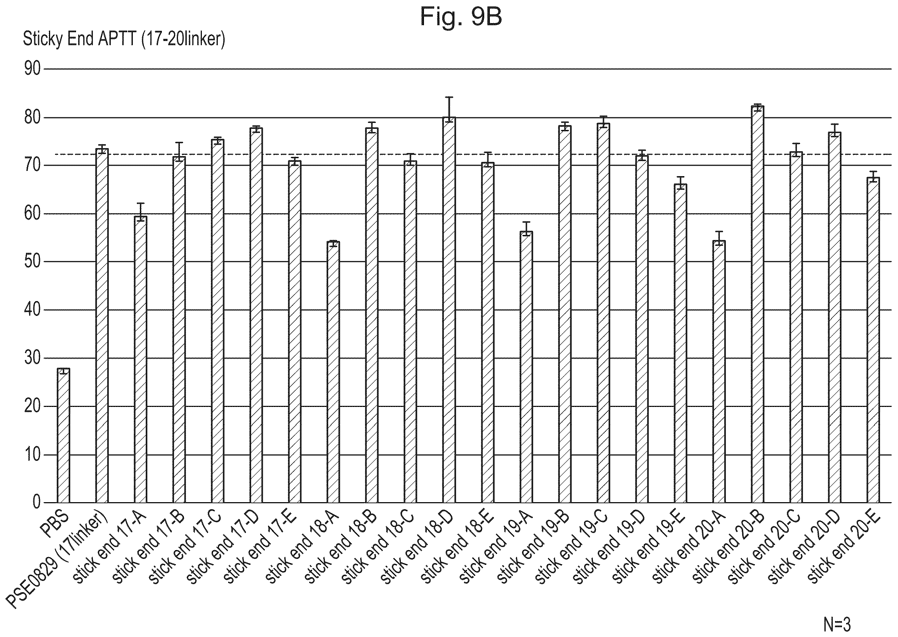

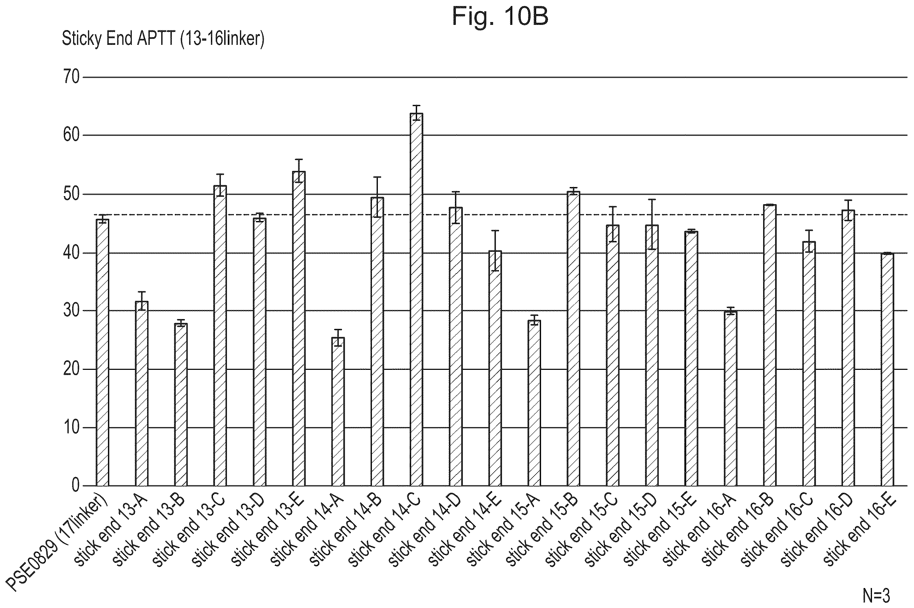

The results are shown in Figs. 9B and 10B. Regardless of the length of the linker, the coagulation inhibitory activity decreased when the overhanging portion (i.e., (Y)ₙ :(X)ₙ) for annealing oligonucleotide A and oligonucleotide B to form a double strand was too long (A) or too short (E). If the base length of the complementary strand forming site is long, a thermodynamically stable double strand is formed; however, if the position of the nick in the double strand approaches the nucleotide sequence that directly interacts with thrombin, the binding affinity of the aptamer for thrombin decreases, so B, C, D, and the like, which have a good balance between both effects, tended to show a relatively high anticoagulation effect.

### <Example 5> Evaluation of Anticoagulation Effect of Monovalent Aptamer (M08s (36); SEQ ID NO: 107)

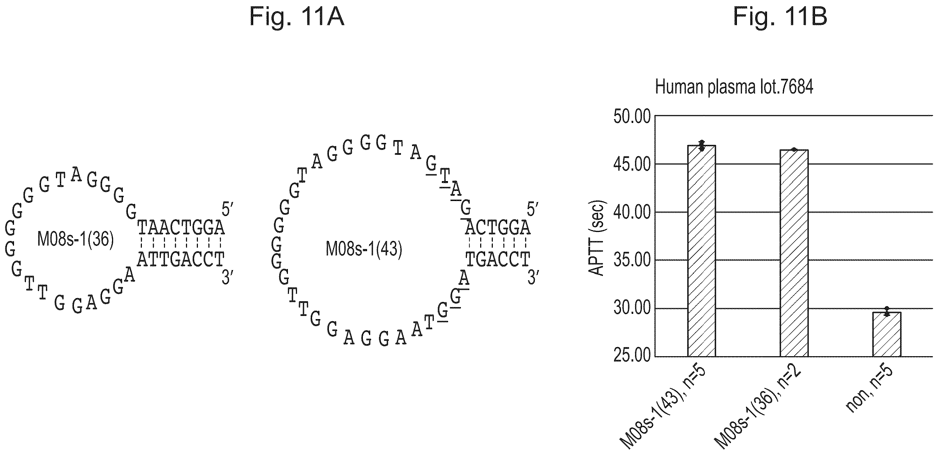

The concentrations of M08s (43) and M08s (36) were adjusted to 150 µM using PBS followed by heating at 95°C for 3 minutes using a dry bath incubator (Major Science Co., Ltd. Taiwan), and then immediately cooled in a heat block that had been left still at 25°C in advance to perform annealing. Fig. 11A shows a comparison of the sequences of M08s (43) and M08s (36). To a sample cup (Sysmex Co., Ltd., Japan), 50 µL of normal human plasma (George King Bio-Medical Inc., Kansas, USA) was added to incubate at 37°C for 1 minute. Next, 50 µL of an APTT reagent (Sysmex Co., Ltd., Japan) was added thereto to incubate at 37°C for 2 minutes, and then 50 µL of 0.2 M calcium chloride was added, and the APTT was immediately measured by tracking the time course of the scattered light intensity at 660 nm using an automated blood coagulation analyzer (Sysmex Co., Ltd., Japan) (final concentration of each oligonucleotide: 0.33 µM). PBS and pse(M08s-TBA29) (SEQ ID NO: 13) were used as a negative control and a positive control, respectively.

The results are shown in Fig. 11B. M08s (36) was observed to have an anticoagulation effect at almost the same level as M08s (43).

### <Example 6> Evaluation of Anticoagulation Effect of Single-Stranded Heterobivalent Aptamer (M08s (36)-TBA29) in Plasma

Oligonucleotides in which M08s (36) and TBA29 were linked by a linker (5'-M08s (36)-linker-TBA29-3': Pse08-29 ss1-XX); 5'-TBA29-linker-M08s (36)-3': Pse08-29 ss2-XX), and oligonucleotides in which M08s (36) and TBA29 were linked without a linker and the 5'-end and the 3'-end were designed to be the stem regions of TBA29 (Pse08-29 ss1-0Ax; Pse08-29 ss2-0Bx) were synthesized and each concentration thereof was adjusted to 150 µM using PBS followed by heating at 95°C for 3 minutes using a dry bath incubator (Major Science Co., Ltd. Taiwan), and then immediately cooled in a heat block that had been left still at 25°C in advance to perform annealing.

To a sample cup (Sysmex Co., Ltd., Japan), 50 µL of normal human plasma (George King Bio-Medical Inc., Kansas, USA) was added to incubate at 37°C for 1 minute. Next, 50 µL of an APTT reagent (Sysmex Co., Ltd., Japan) was added thereto to incubate at 37°C for 2 minutes, and then 50 µL of 0.2 M calcium chloride was added, and the APTT was immediately measured by tracking the time course of the scattered light intensity at 660 nm using an automated blood coagulation analyzer (Sysmex Co., Ltd., Japan) (final concentration of oligonucleotide: 0.33 µM). PBS and pse(M08s-TBA29) (SEQ ID NO: 13) were used as a negative control and a positive control, respectively.

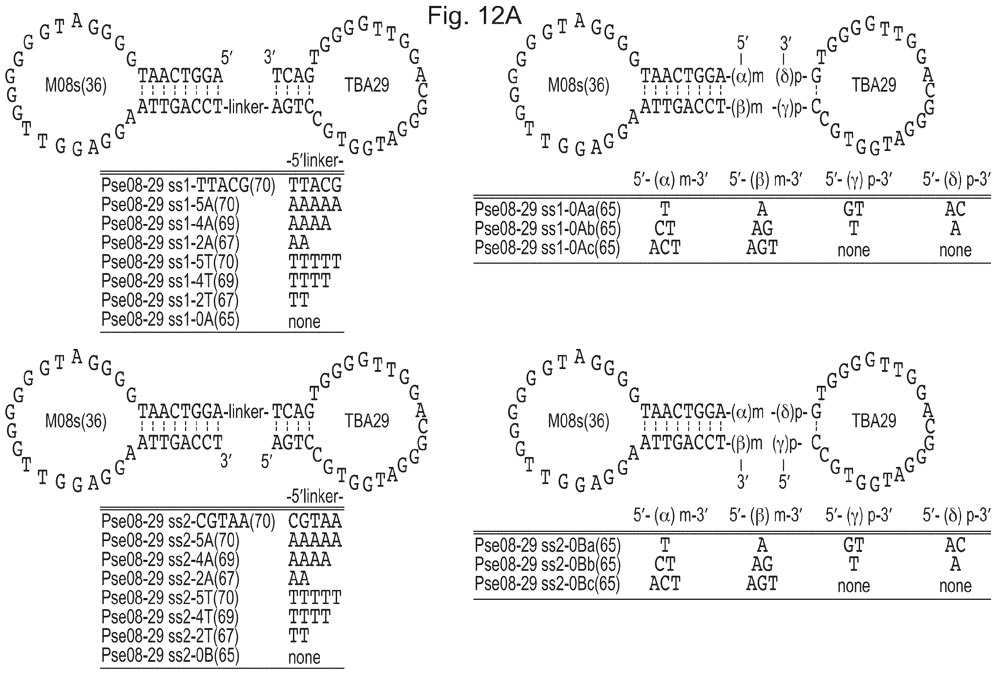

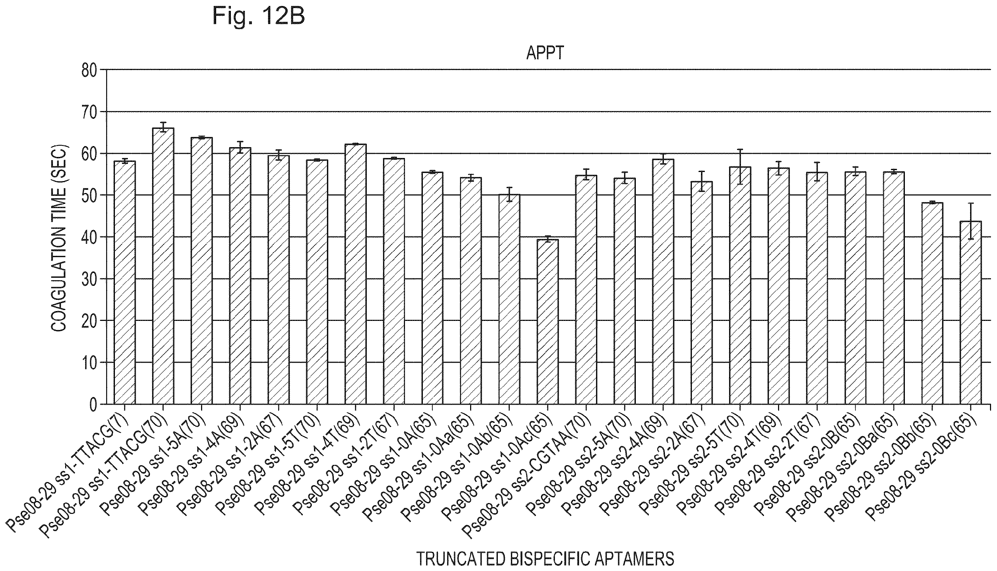

The results are shown in Fig. 12B. The anticoagulation effect was maintained even without a linker; however, when the 5'-end and the 3'-end were designed to be the stem regions of TBA29, the anticoagulation effect was reduced. This indicates that the position of TBA29 (the structure of the stem region) in the bivalent aptamer and the orientation of the two thrombin binding sites on the aptamer contribute to the anticoagulation effect.

### <Example 7> Evaluation of Anticoagulation Effect of Single-Stranded Homobivalent Aptamer (M08s (36)-M08s (36)) in Plasma

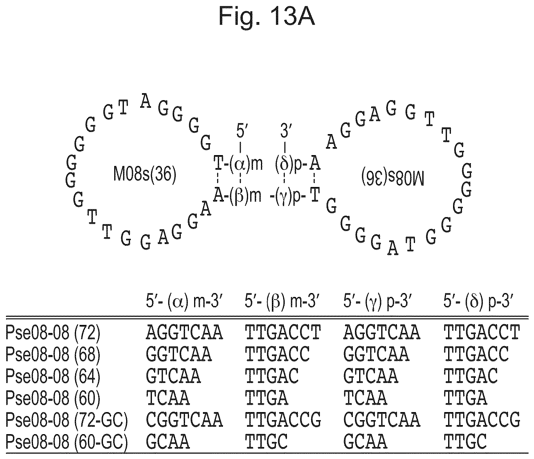

Oligonucleotides in which M08s (36) and M08s (36) were linked and the length of the stem region and the amino acid sequences thereof were modified (Pse08-08(xx)) were synthesized, each concentration thereof was adjusted to 150 µM using PBS followed by heating at 95°C for 3 minutes using a dry bath incubator (Major Science Co., Ltd. Taiwan), and then immediately cooled in a heat block that had been left still at 25°C in advance to perform annealing. The schematic diagram of the secondary structure is shown in Fig. 13A.

To a sample cup (Sysmex Co., Ltd., Japan), 50 µL of normal human plasma (George King Bio-Medical Inc., Kansas, USA) was added to incubate at 37°C for 1 minute. Next, 50 µL of an APTT reagent (Sysmex Co., Ltd., Japan) was added thereto to incubate at 37°C for 2 minutes, and then 50 µL of 0.2 M calcium chloride was added, and the APTT was immediately measured by tracking the time course of the scattered light intensity at 660 nm using an automated blood coagulation analyzer (Sysmex Co., Ltd., Japan) (final concentration of oligonucleotide: 0.33 µM). PBS and pse(M08s-TBA29) (SEQ ID NO: 13) were used as a negative control and a positive control, respectively.

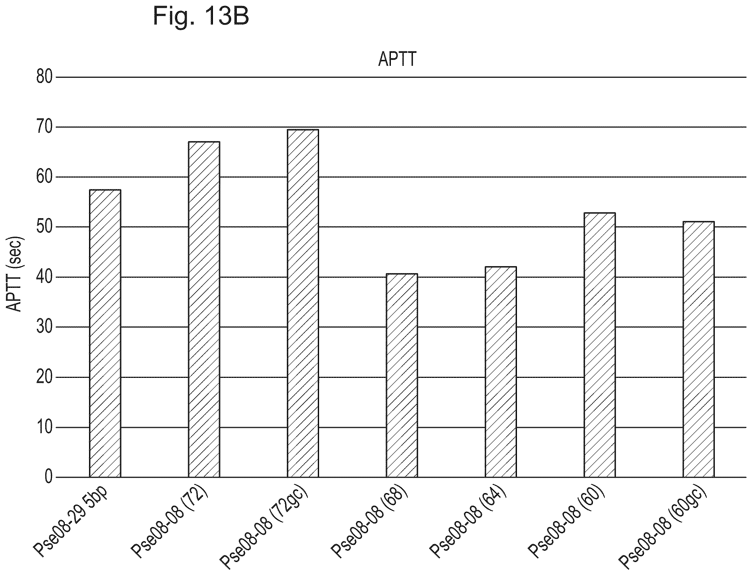

The results are shown in Fig. 13B. This indicates that the orientation of the two thrombin binding sites on the bivalent aptamer and the like contribute to the anticoagulation effect.

### <Example 8> Neutralizing Effect of Adding Oligonucleotide With Complementary Strand Sequence to Nucleic Acid Aptamer: a) Pse08-29 ss1-4A (69); b) Pse08-29 ss1-2A (67); and c) Pse08-08 (64), in Human Plasma

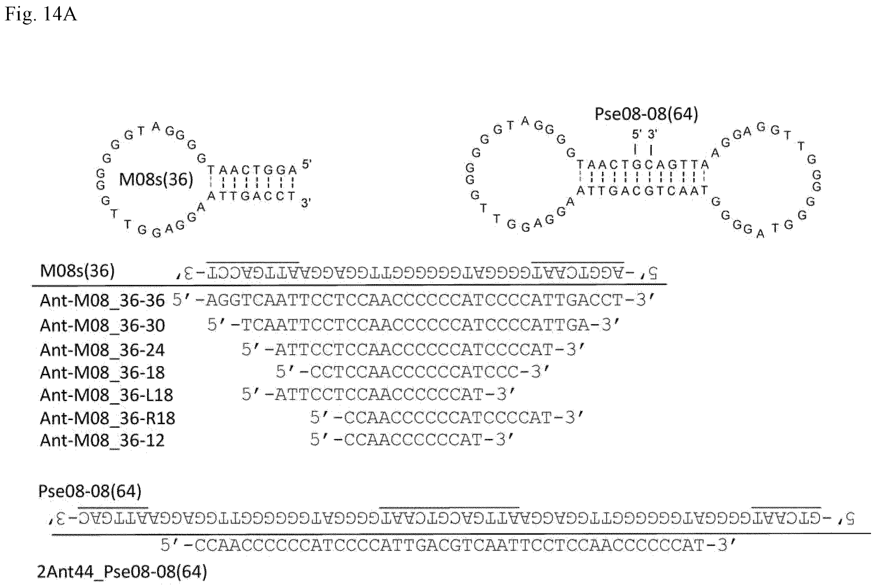

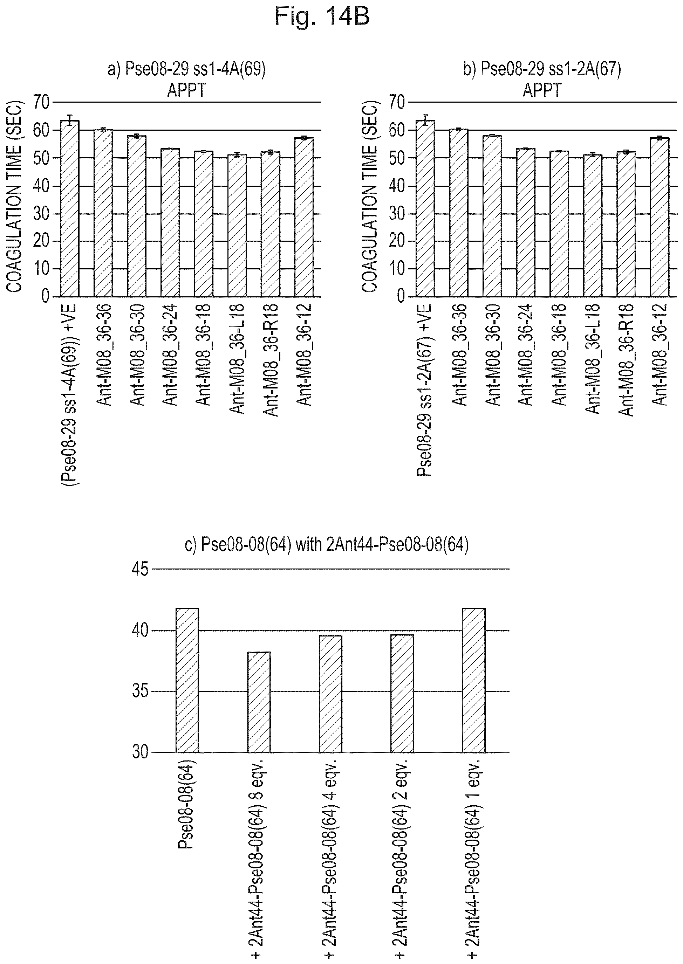

The concentrations of the aptamer Pse08-29 ss1-4A (69) or Pse08-29 ss1-2A (67) and the complementary strand sequence oligonucleotide (Ant-M08_36-XX; see Fig. 14A) were each adjusted to 150 µM, and 1000 µM using PBS followed by heating at 95°C for 3 minutes using a dry bath incubator (Major Science Co., Ltd. Taiwan), and then immediately cooled in a heat block that had been left still at 25°C in advance to perform annealing. To a sample cup (Sysmex Co., Ltd., Japan), 145 µL of normal human plasma (George King Bio-Medical Inc., Kansas, USA), 2.5 µL of a) Pse08-29 ss1-4A (69) or b) Pse08-29 ss1-2A (67), and 2.5 µL of the complementary strand sequence were added to incubate at 37°C for 1 minute. Next, 50 µL of an APTT reagent (Sysmex Co., Ltd., Japan) was mixed to incubate at 37°C for 2 minutes, and then, 50 µL of 0.2 M calcium chloride was added, and the APTT was immediately measured by tracking the time course of the scattered light intensity at 660 nm using an automated blood coagulation analyzer (Sysmex Co., Ltd., Japan) under the following conditions:
Nucleic acid aptamer : 0.33 µM (1 eqv.)
Complementary strand sequence oligonucleotide : 2.64 µM (8 eqv.)
Normal human plasma : 32 v/v%
Temperature : 37°C
Two measurements.
As a control, aptamers alone (a) Pse08-29 ss1-4A (69) + VE; b) Pse08-29 ss1-2A (67) + VE) were used.

Similarly, APTT was measured using c) Pse08-08 (64) (concentration: 0.33 µM) and the complementary strand sequence oligonucleotide (2Ant44 _Pse08-08 (64)) (concentrations: 0.33 µM to 2.64 µM).

The results are shown in Fig. 14B. A reduction in anticoagulation effect was observed for all sequences examined, and of the four GG sequences predicted to form a quadruplex structure, the reduction in anticoagulation effect was greater for sequences that formed complementary strands with three of the GG sequences. In addition, the anticoagulation effect of Pse08-08 (64) was reduced in its concentration-dependent manner by the neutralizing agent sequence.

The detailed sequence information is shown below.

### [Table 7]

**Table 7**

| Name | Sequence | SEQ ID NO: |
|---|---|---|
| TBA15 | GGTTGGTGTGGTTGG | 1 |
| NU172 | CGCCTAGGTTGGGTAGGGTGGTGGCG | 2 |
| TBA29 | AGTCCGTGGTAGGGCAGGTTGGGGTGACT | 3 |
| M08s-Cbp-TBA29 | AGGTCAGATGATGGGGATGGGGGGTTGGAGGAATGGATGACCTAGTCCGTGGTAGGGCAGGTTGGGGTGACT | 4 |
| M08s-TA5bp-TBA29 | TAAGGTCAGATGATGGGGATGGGGGGTTGGAGGAATGGATGACCTTATATAGTCCGTGGTAGGGCAGGTTGGGGTGACTATA | 5 |
| M08s-TA17bp-TBA29 | TATATATAAGGTCAGATGATGGGGATGGGGGGTTGGAGGAATGGATGACCTTATATATATATATATATAGTCCGTGGTAGGGCAGGTTGGGGTGACTATATATATA | 6 |
| M08s-TA21bp-TBA29 | TATATATATAAGGTCAGATGATGGGGATGGGGGGTTGGAGGAATGGATGACCTTATATATATATATATATATATAGTCCGTGGTAGGGCAGGTTGGGGTGACTATATATATATA | 7 |
| M08s-ATGC5bp-TBA29 | AAAGGTCAGATGATGGGGATGGGGGGTTGGAGGAATGGATGACCTTTACGAGTCCGTGGTAGGGCAGGTTGGGGTGACTCGT | 8 |
| M08s-ATGC17bp-TBA29 | GTCACTAGAGGTCAGATGATGGGGATGGGGGGTTGGAGGAATGGATGACCTCTAGTGACTGATTTACGAGTCCGTGGTAGGGCAGGTTGGGGTGACTCGTAAATCA | 9 |
| M08s-ATGC21bp-TBA29 | CACTAGACTGAGGTCAGATGATGGGGATGGGGGGTTGGAGGAATGGATGACCTCAGTCTAGTGACTGATTTACGAGTCCGTGGTAGGGCAGGTTGGGGTGACTCGTAAATCAGT | 10 |
| lin(M08s-TBA29) | AGGTCAGATGATGGGGATGGGGGGTTGGAGGAATGGATGACCTTTTTTTTTTTTTTTTTTTTAGTCCGTGGTAGGGCAGGTTGGGGTGACT | 11 |
| pse(M08s-M03s) | CAGTCACTAGAGGTCAGATGATGGGGATGGGGGGTTGGAGGAATGGATGACCTCTAGTGACTGATTTACGAGGTCAGATGGTGGTCGGGGTGGTGGGATGAGGGTTCTGACCTCGTAAAT | 12 |
| pse(M08s-TBA29) | GTCACTAGAGGTCAGATGATGGGGATGGGGGGTTGGAGGAATGGATGACCTCTAGTGACTGATTTACGAGTCCGTGGTAGGGCAGGTTGGGGTGACTCGTAAATCA | 13 |
| M08s | AGGTCAGATGATGGGGATGGGGGGTTGGAGGAATGGATGACCT | 14 |
| 08ad | CCCCATCCCCATCATCTGACCTCTAGTGACTG | 15 |
| [M08s]c | CCCCATCCCCATCATCTGACCT | 16 |
| [L-M08s]c | CCCCATCCCCATCATCTGACCTCTAGTGAC | 11 |
| M08s ring | AGATGATGGG GATGGGGGGT TGGAGGAATG GAT | 18 |
| M03s ring | ATGGTGGTCG GGGTGGTGGG ATGAGGGTT | 19 |
| TBA29 ring | CCGTGGTAGG GCAGGTTGGG GTG | 20 |
| M03s | AGGTCAGATG GTGGTCGGGG TGGTGGGATG AGGGTTCTGA CCT | 21 |
| pse(M08s-M08s) | GTCACTAGAG GTCAGATGAT GGGGATGGGG GGTTGGAGGA ATGGATGACC TCTAGTGACT GATTTACGAG GTCAGATGAT GGGGATGGGG GGTTGGAGGA ATGGATGACC TCGTAAATCA | 22 |
| M08s partial | AGGTCAGATG ATGGGGATGG G | 23 |
| beta-X-gamma linker | CTAGTGACTG ATTTACG | 24 |
| delta-Y-alpha linker | CGTAAATCAG TCACTAG | 25 |
| Sticky end20A-1 | GTCAGATGATGGGGATGGGGGGTTGGAGGAATGGATGACCTAGTCTAGTGACTGATTTACGAGT | 26 |
| Sticky end20A-2 | CCGTGGTAGGGCAGGTTGGGGTGACTCGTAAATCAGTCACTAGACTAG | 27 |
| Sticky end20B-1 | AGGTCAGATGATGGGGATGGGGGGTTGGAGGAATGGATGACCTAGTCTAGTGACTGATTTA | 28 |
| Sticky end20B-2 | GTCCGTGGTAGGGCAGGTTGGGGTGACTCGTAAATCAGTCACTAGACT | 29 |
| Sticky end20C-1 | CTAGGTCAGATGATGGGGATGGGGGGTTGGAGGAATGGATGACCTAGTCTAGTGACTGATTTAC | 30 |
| Sticky end20C-2 | GAGTCCGTGGTAGGGCAGGTTGGGGTGACTCGTAAATCAGTCACTAGA | 31 |
| Sticky end20D-1 | GACTAGGTCAGATGATGGGGATGGGGGGTTGGAGGAATGGATGACCTAGTCTAGTGACTGATTT | 32 |
| Sticky end20D-2 | ACGAGTCCGTGGTAGGGCAGGTTGGGGTGACTCGTAAATCAGTCACTA | 33 |
| Sticky end20E-1 | TAGACTAGGTCAGATGATGGGGATGGGGGGTTGGAGGAATGGATGACCTAGTCTAGTGACTGAT | 34 |
| Sticky end20E-2 | TTACGAGTCCGTGGTAGGGCAGGTTGGGGTGACTCGTAAATCAGTCAC | 35 |
| Sticky end19A-1 | GTCAGATGATGGGGATGGGGGGTTGGAGGAATGGATGACCTGTCTAGTGACTGATTTACGAGT | 36 |
| Sticky end19A-2 | CCGTGGTAGGGCAGGTTGGGGTGACTCGTAAATCAGTCACTAGACAG | 37 |
| Sticky end19B-1 | AGGTCAGATGATGGGGATGGGGGGTTGGAGGAATGGATGACCTGTCTAGTGACTGATTTACGA | 38 |
| Sticky end19B-2 | GTCCGTGGTAGGGCAGGTTGGGGTGACTCGTAAATCAGTCACTAGAC | 39 |
| Sticky end1 9C-1 | ACAGGTCAGATGATGGGGATGGGGGGTTGGAGGAATGGATGACCTGTCTAGTGACTGATTTAC | 40 |
| Sticky end19C-2 | GAGTCCGTGGTAGGGCAGGTTGGGGTGACTCGTAAATCAGTCACTAG | 41 |
| Sticky end19D-1 | AGACAGGTCAGATGATGGGGATGGGGGGTTGGAGGAATGGATGACCTGTCTAGTGACTGATTT | 42 |
| Sticky end19D-2 | ACGAGTCCGTGGTAGGGCAGGTTGGGGTGACTCGTAAATCAGTCACT | 43 |
| Sticky end19E-1 | CTAGACAGGTCAGATGATGGGGATGGGGGGTTGGAGGAATGGATGACCTGTCTAGTGACTGAT | 44 |
| Sticky end19E-2 | TTACGAGTCCGTGGTAGGGCAGGTTGGGGTGACTCGTAAATCAGTCA | 45 |
| Sticky end18A-1 | GTCAGATGATGGGGATGGGGGGTTGGAGGAATGGATGACCTTCTAGTGACTGATTTACGAGT | 46 |
| Sticky end18A-2 | CCGTGGTAGGGCAGGTTGGGGTGACTCGTAAATCAGTCACTAGAAG | 47 |
| Sticky end18B-1 | AGGTCAGATGATGGGGATGGGGGGTTGGAGGAATGGATGACCTTCTAGTGACTGATTTACGA | 48 |
| Sticky end18B-2 | GTCCGTGGTAGGGCAGGTTGGGGTGACTCGTAAATCAGTCACTAGA | 49 |
| Sticky end18C-1 | GAAGGTCAGATGATGGGGATGGGGGGTTGGAGGAATGGATGACCTTCTAGTGACTGATTTAC | 50 |
| Sticky end18C-2 | GAGTCCGTGGTAGGGCAGGTTGGGGTGACTCGTAAATCAGTCACTA | 51 |
| Sticky end18D-1 | TAGAAGGTCAGATGATGGGGATGGGGGGTTGGAGGAATGGATGACCTTCTAGTGACTGATTT | 52 |
| Sticky end18D-2 | ACGAGTCCGTGGTAGGGCAGGTTGGGGTGACTCGTAAATCAGTCAC | 53 |
| Sticky end18E-1 | ACTAGAAGGTCAGATGATGGGGATGGGGGGTTGGAGGAATGGATGACCTTCTAGTGACTGAT | 54 |
| Sticky end18E-2 | TTACGAGTCCGTGGTAGGGCAGGTTGGGGTGACTCGTAAATCAGTC | 55 |
| Sticky end17A-1 | GTCAGATGATGGGGATGGGGGGTTGGAGGAATGGATGACCTCTAGTGACTGATTTACGAGT | 56 |
| Sticky end17A-2 | CCGTGGTAGGGCAGGTTGGGGTGACTCGTAAATCAGTCACTAGAG | 57 |
| Sticky end17B-1 | AGGTCAGATGATGGGGATGGGGGGTTGGAGGAATGGATGACCTCTAGTGACTGATTTACGA | 58 |
| Sticky end17B-2 | GTCCGTGGTAGGGCAGGTTGGGGTGACTCGTAAATCAGTCACTAG | 59 |
| Sticky end17C-1 | AGAGGTCAGATGATGGGGATGGGGGGTTGGAGGAATGGATGACCTCTAGTGACTGATTTAC | 60 |
| Sticky end17C-2 | GAGTCCGTGGTAGGGCAGGTTGGGGTGACTCGTAAATCAGTCACT | 61 |
| Sticky end17D-1 | CTAGAGGTCAGATGATGGGGATGGGGGGTTGGAGGAATGGATGACCTCTAGTGACTGATTT | 62 |
| Sticky end17D-2 | ACGAGTCCGTGGTAGGGCAGGTTGGGGTGACTCGTAAATCAGTCA | 63 |
| Sticky end17E-1 | CACTAGAGGTCAGATGATGGGGATGGGGGGTTGGAGGAATGGATGACCTCTAGTGACTGAT | 64 |
| Sticky end17E-2 | TTACGAGTCCGTGGTAGGGCAGGTTGGGGTGACTCGTAAATCAGT | 65 |
| Sticky end16A-1 | GTCAGATGATGGGGATGGGGGGTTGGAGGAATGGATGACCTTAGTGACTGATTTACGAGT | 66 |
| Sticky end16A-2 | CCGTGGTAGGGCAGGTTGGGGTGACTCGTAAATCAGTCACTAAG | 67 |
| Sticky end16B-1 | AGGTCAGATGATGGGGATGGGGGGTTGGAGGAATGGATGACCTTAGTGACTGATTTACGA | 68 |
| Sticky end16B-2 | GTCCGTGGTAGGGCAGGTTGGGGTGACTCGTAAATCAGTCACTA | 69 |
| Sticky end16C-1 | TAAGGTCAGATGATGGGGATGGGGGGTTGGAGGAATGGATGACCTTAGTGACTGATTTAC | 70 |
| Sticky end16C-2 | GAGTCCGTGGTAGGGCAGGTTGGGGTGACTCGTAAATCAGTCAC | 71 |
| Sticky end16D-1 | ACTAAGGTCAGATGATGGGGATGGGGGGTTGGAGGAATGGATGACCTTAGTGACTGATTT | 72 |
| Sticky end16D-2 | ACGAGTCCGTGGTAGGGCAGGTTGGGGTGACTCGTAAATCAGTC | 73 |
| Sticky end16E-1 | TCACTAAGGTCAGATGATGGGGATGGGGGGTTGGAGGAATGGATGACCTTAGTGACTGAT | 74 |
| Sticky end16E-2 | TTACGAGTCCGTGGTAGGGCAGGTTGGGGTGACTCGTAAATCAG | 75 |
| Sticky end15A-1 | GTCAGATGATGGGGATGGGGGGTTGGAGGAATGGATGACCTAGTGACTGATTTACGAGT | 76 |
| Sticky end15A-2 | CCGTGGTAGGGCAGGTTGGGGTGACTCGTAAATCAGTCACTAG | 77 |
| Sticky end15B-1 | AGGTCAGATGATGGGGATGGGGGGTTGGAGGAATGGATGACCTAGTGACTGATTTACGA | 78 |
| Sticky end15B-2 | GTCCGTGGTAGGGCAGGTTGGGGTGACTCGTAAATCAGTCACT | 79 |
| Sticky end15C-1 | CTAGGTCAGATGATGGGGATGGGGGGTTGGAGGAATGGATGACCTAGTGACTGATTTAC | 80 |
| Sticky end15C-2 | GAGTCCGTGGTAGGGCAGGTTGGGGTGACTCGTAAATCAGTCA | 81 |
| Sticky end15D-1 | CACTAGGTCAGATGATGGGGATGGGGGGTTGGAGGAATGGATGACCTAGTGACTGATTT | 82 |
| Sticky end15D-2 | ACGAGTCCGTGGTAGGGCAGGTTGGGGTGACTCGTAAATCAGT | 83 |
| Sticky end15E-1 | GTCACTAGGTCAGATGATGGGGATGGGGGGTTGGAGGAATGGATGACCTAGTGACTGAT | 84 |
| Sticky end15E-2 | TTACGAGTCCGTGGTAGGGCAGGTTGGGGTGACTCGTAAATCA | 85 |
| Sticky end14A-1 | GTCAGATGATGGGGATGGGGGGTTGGAGGAATGGATGACCTGTGACTGATTTACGAGT | 86 |
| Sticky end14A-2 | CCGTGGTAGGGCAGGTTGGGGTGACTCGTAAATCAGTCACAG | 87 |
| Sticky end14B-1 | AGGTCAGATGATGGGGATGGGGGGTTGGAGGAATGGATGACCTGTGACTGATTTACGA | 88 |
| Sticky end14B-2 | GTCCGTGGTAGGGCAGGTTGGGGTGACTCGTAAATCAGTCAC | 89 |
| Sticky end14C-1 | ACAGGTCAGATGATGGGGATGGGGGGTTGGAGGAATGGATGACCTGTGACTGATTTAC | 90 |
| Sticky end14C-2 | GAGTCCGTGGTAGGGCAGGTTGGGGTGACTCGTAAATCAGTC | 91 |
| Sticky end14D-1 | TCACAGGTCAGATGATGGGGATGGGGGGTTGGAGGAATGGATGACCTGTGACTGATTT | 92 |
| Sticky end14D-2 | ACGAGTCCGTGGTAGGGCAGGTTGGGGTGACTCGTAAATCAG | 93 |
| Sticky end14E-1 | AGTCACAGGTCAGATGATGGGGATGGGGGGTTGGAGGAATGGATGACCTGTGACTGAT | 94 |
| Sticky end14E-2 | TTACGAGTCCGTGGTAGGGCAGGTTGGGGTGACTCGTAAATC | 95 |
| Sticky end13A-1 | GTCAGATGATGGGGATGGGGGGTTGGAGGAATGGATGACCTTGACTGATTTACGAGT | 96 |
| Sticky end13A-2 | CCGTGGTAGGGCAGGTTGGGGTGACTCGTAAATCAGTCAAG | 97 |
| Sticky end13B-1 | AGGTCAGATGATGGGGATGGGGGGTTGGAGGAATGGATGACCTTGACTGATTTACGA | 98 |
| Sticky end13B-2 | GTCCGTGGTAGGGCAGGTTGGGGTGACTCGTAAATCAGTCA | 99 |
| Sticky end13C-1 | CAAGGTCAGATGATGGGGATGGGGGGTTGGAGGAATGGATGACCTTGACTGATTTAC | 100 |
| Sticky end13C-2 | GAGTCCGTGGTAGGGCAGGTTGGGGTGACTCGTAAATCAGT | 101 |
| Sticky end13D-1 | GTCAAGGTCAGATGATGGGGATGGGGGGTTGGAGGAATGGATGACCTTGACTGATTT | 102 |
| Sticky end13D-2 | ACGAGTCCGTGGTAGGGCAGGTTGGGGTGACTCGTAAATCA | 103 |
| Sticky end13E-1 | CAGTCAAGGTCAGATGATGGGGATGGGGGGTTGGAGGAATGGATGACCTTGACTGAT | 104 |
| Sticky end13E-2 | TTACGAGTCCGTGGTAGGGCAGGTTGGGGTGACTCGTAAAT | 105 |
| M08s-1(36) loop | TGGGGATGGGGGGTTGGAGGAA | 106 |
| M08s-1(36) stem-loop | AGGTCAATGGGGATGGGGGGTTGGAGGAATTGACCT | 107 |
| Pse08-29 ss1-TTACG(70) | AGGTCAATGGGGATGGGGGGTTGGAGGAATTGACCTTTACGAGTCCGTGGTAGGGCAGGTTGGGGTGACT | 108 |
| Pse08-29 ss1-5A(70) | AGGTCAATGGGGATGGGGGGTTGGAGGAATTGACCTAAAAAAGTCCGTGGTAGGGCAGGTTGGGGTGACT | 109 |
| Pse08-29 ss1-4A(69) | AGGTCAATGGGGATGGGGGGTTGGAGGAATTGACCTAAAAAGTCCGTGGTAGGGCAGGTTGGGGTGACT | 110 |
| Pse08-29 ss1-2A(67) | AGGTCAATGGGGATGGGGGGTTGGAGGAATTGACCTAAAGTCCGTGGTAGGGCAGGTTGGGGTGACT | 111 |
| Pse08-29 ss1-5T(70) | AGGTCAATGGGGATGGGGGGTTGGAGGAATTGACCTTTTTTAGTCCGTGGTAGGGCAGGTTGGGGTGACT | 112 |
| Pse08-29 ss1-4T(69) | AGGTCAATGGGGATGGGGGGTTGGAGGAATTGACCTTTTTAGTCCGTGGTAGGGCAGGTTGGGGTGACT | 113 |
| Pse08-29 ss1-2T(67) | AGGTCAATGGGGATGGGGGGTTGGAGGAATTGACCTTTAGTCCGTGGTAGGGCAGGTTGGGGTGACT | 114 |
| Pse08-29 ss1-0A(65) | AGGTCAATGGGGATGGGGGGTTGGAGGAATTGACCTAGTCCGTGGTAGGGCAGGTTGGGGTGACT | 115 |
| Pse08-29 ss1-0Aa(65) | TAGGTCAATGGGGATGGGGGGTTGGAGGAATTGACCAGTCCGTGGTAGGGCAGGTTGGGGTGAC | 116 |
| Pse08-29 ss1-0Ab(65) | CTAGGTCAATGGGGATGGGGGGTTGGAGGAATTGACCAGTCCGTGGTAGGGCAGGTTGGGGTGA | 117 |
| Pse08-29 ss1-0Ac(65) | ACTAGGTCAATGGGGATGGGGGGTTGGAGGAATTGACCAGTCCGTGGTAGGGCAGGTTGGGGTG | 118 |
| Pse08-29 ss2-CGTAA(70) | AGTCCGTGGTAGGGCAGGTTGGGGTGACTCGTAAAGGTCAATGGGGATGGGGGGTTGGAGGAATTGACCT | 119 |
| Pse08-29 ss2-5A(70) | AGTCCGTGGTAGGGCAGGTTGGGGTGACTAAAAAAGGTCAATGGGGATGGGGGGTTGGAGGAATTGACCT | 120 |
| Pse08-29 ss2-4A(69) | AGTCCGTGGTAGGGCAGGTTGGGGTGACTAAAAAGGTCAATGGGGATGGGGGGTTGGAGGAATTGACCT | 121 |
| Pse08-29 ss2-2A(67) | AGTCCGTGGTAGGGCAGGTTGGGGTGACTAAAGGTCAATGGGGATGGGGGGTTGGAGGAATTGACCT | 122 |
| Pse08-29 ss2-5T(70) | AGTCCGTGGTAGGGCAGGTTGGGGTGACTTTTTTAGGTCAATGGGGATGGGGGGTTGGAGGAATTGACCT | 123 |
| Pse08-29 ss2-4T(69) | AGTCCGTGGTAGGGCAGGTTGGGGTGACTTTTTAGGTCAATGGGGATGGGGGGTTGGAGGAATTGACCT | 124 |
| Pse08-29 ss2-2T(67) | AGTCCGTGGTAGGGCAGGTTGGGGTGACTTTAGGTCAATGGGGATGGGGGGTTGGAGGAATTGACCT | 125 |
| Pse08-29 ss2-0B(65) | AGTCCGTGGTAGGGCAGGTTGGGGTGACTAGGTCAATGGGGATGGGGGGTTGGAGGAATTGACCT | 126 |
| Pse08-29 ss2-0Ba(65) | GTCCGTGGTAGGGCAGGTTGGGGTGACTAGGTCAATGGGGATGGGGGGTTGGAGGAATTGACCTA | 127 |
| Pse08-29 ss2-0Bb(65) | TCCGTGGTAGGGCAGGTTGGGGTGACTAGGTCAATGGGGATGGGGGGTTGGAGGAATTGACCTAG | 128 |
| Pse08-29 ss2-0Bc(65) | CCGTGGTAGGGCAGGTTGGGGTGACTAGGTCAATGGGGATGGGGGGTTGGAGGAATTGACCTAGT | 129 |
| Pse08-08(72) | AGGTCAATGGGGATGGGGGGTTGGAGGAATTGACCTAGGTCAATGGGGATGGGGGGTTGGAGGAATTGACCT | 130 |
| Pse08-08(68) | GGTCAATGGGGATGGGGGGTTGGAGGAATTGACCGGTCAATGGGGATGGGGGGTTGGAGGAATTGACC | 131 |
| Pse08-08(64) | GTCAATGGGGATGGGGGGTTGGAGGAATTGACGTCAATGGGGATGGGGGGTTGGAGGAATTGAC | 132 |
| Pse08-08(60) | TCAATGGGGATGGGGGGTTGGAGGAATTGATCAATGGGGATGGGGGGTTGGAGGAATTGA | 133 |
| Pse08-08(72-GC) | CGGTCAATGGGGATGGGGGGTTGGAGGAATTGACCGCGGTCAATGGGGATGGGGGGTTGGAGGAATTGACCG | 134 |
| Pse08-08(60-GC) | GCAATGGGGATGGGGGGTTGGAGGAATTGCGCAATGGGGATGGGGGGTTGGAGGAATTGC | 135 |
| Pse08-08 (72) | AGGTCAATGGGGATGGGGGGTTGGAGGAATTGACCTAGGTCAATGGGGATGGGGGGTTGGAGGAATTGACCT | 136 |
| Pse08-08 (68) | GGTCAATGGGGATGGGGGGTTGGAGGAATTGACCGGTCAATGGGGATGGGGGGTTGGAGGAATTGACC | 137 |
| Pse08-08 (64) | GTCAATGGGGATGGGGGGTTGGAGGAATTGACGTCAATGGGGATGGGGGGTTGGAGGAATTGAC | 138 |
| Pse08-08 (60) | TCAATGGGGATGGGGGGTTGGAGGAATTGATCAATGGGGATGGGGGGTTGGAGGAATTGA | 139 |
| Pse08-08 (72-GC) | CGGTCAATGGGGATGGGGGGTTGGAGGAATTGACCGCGGTCAATGGGGATGGGGGGTTGGAGGAATTGACCG | 140 |
| Pse08-08 (60-GC) | GCAATGGGGATGGGGGGTTGGAGGAATTGCGCAATGGGGATGGGGGGTTGGAGGAATTGC | 141 |
| Ant-M08_36-36 | AGGTCAATTCCTCCAACCCCCCATCCCCATTGACCT | 142 |
| Ant-M08_36-30 | TCAATTCCTCCAACCCCCCATCCCCATTGA | 143 |
| Ant-M08_36-24 | ATTCCTCCAACCCCCCATCCCCAT | 144 |
| Ant-M08_36-18 | CCTCCAACCCCCCATCCC | 145 |
| Ant-M08_36-L18 | ATTCCTCCAACCCCCCAT | 146 |
| Ant-M08_36-R18 | CCAACCCCCCATCCCCAT | 147 |
| Ant-M08_36-12 | CCAACCCCCCAT | 148 |
| 2Ant44_Pse08-08(64) | CCAACCCCCCATCCCCATTGACGTCAATTCCTCCAACCCCCCAT | 149 |

one oligonucleotide while maintaining its activity. The present invention can increase the accuracy of oligonucleotide synthesis (i.e., synthesis of unintended oligonucleotides is less likely to occur), making it suitable for use as a medicine.

## Claims

1. A DNA aptamer containing the following primary structure: 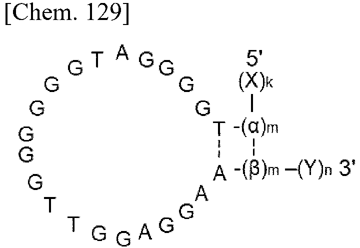 wherein
k, m, and n represent the numbers of DNA bases;
k is 0, 1, 2, or 3;
m is an integer selected from the group consisting of 1 to 15;
n is 0, 1, 2, or 3;
X and Y are each independently any bases;
α and β are bases; and
5'-(α)ₘ-3' and 5'-(β)ₘ-3' form an antiparallel double-stranded structure.

2. The DNA aptamer according to claim 1, containing the following primary structure:

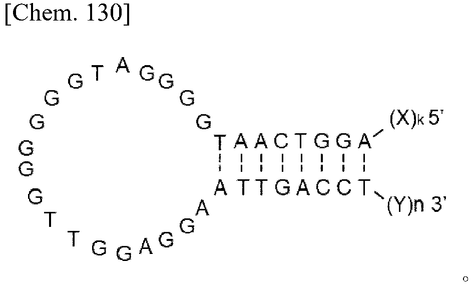

3. The DNA aptamer according to claim 1, containing the sequence of M08s-1 (36) (SEQ ID NO: 107).

4. A bivalent DNA aptamer, comprising
two DNA aptamers that bind to exosite I of thrombin;
two DNA aptamers that bind to exosite II of thrombin; or
a DNA aptamer that binds to exosite I of thrombin and a DNA aptamer that binds to exosite II,
wherein the DNA aptamers are linked to each other via duplex DNA.

5. The bivalent DNA aptamer according to claim 4, wherein the bivalent DNA aptamer is in a single-stranded form and comprises any of the following primary structures: 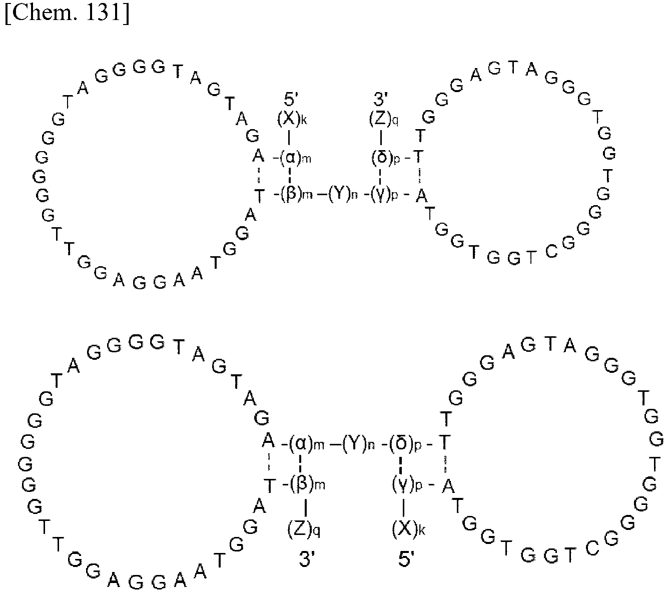 wherein
k, m, n, p, and q represent the numbers of DNA bases;
k is 0, 1, 2, or 3;
m is an integer selected from the group consisting of 1 to 15;
n is 0, 1, 2, 3, 4, or 5;
p is an integer selected from the group consisting of 1 to 17;
q is 0, 1, 2, or 3;
X, Y, and Z are each independently any bases;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

6. The bivalent DNA aptamer according to claim 4, wherein the bivalent DNA aptamer is in a single-stranded form and contains any of the following primary structures: 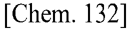 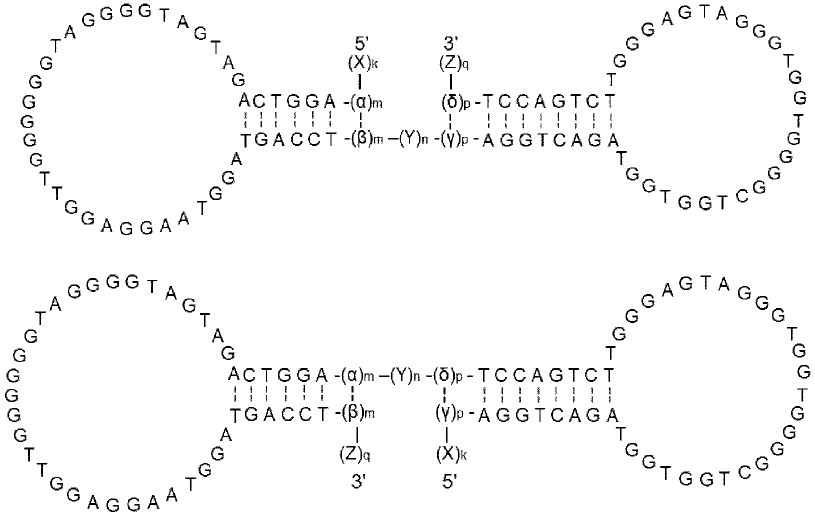 wherein
k, m, n, p, and q represent the numbers of DNA bases;
k is 0, 1, 2, or 3;
m is an integer selected from the group consisting of 1 to 10;
n is 0, 1, 2, 3, 4, or 5;
p is an integer selected from the group consisting of 1 to 10;
q is 0, 1, 2, or 3;
X, Y, and Z are each independently any bases;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

7. The bivalent DNA aptamer according to claim 4, wherein the bivalent DNA aptamer is in a single-stranded form and contains any of the following primary structures: 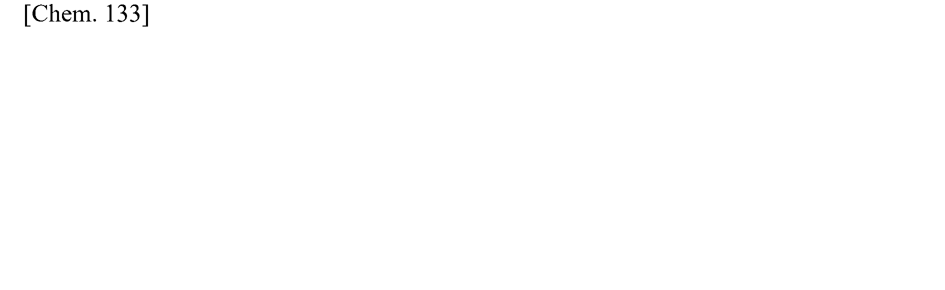 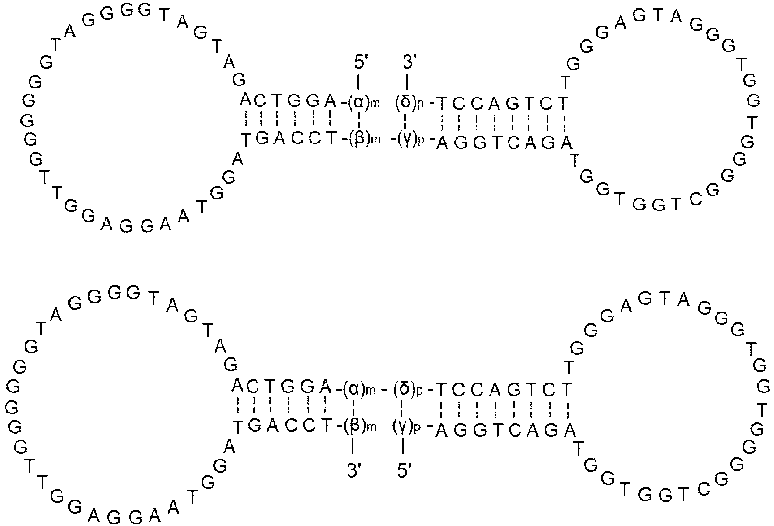 wherein
m and p represent the numbers of DNA bases;
m is an integer selected from the group consisting of 1 to 10;
p is an integer selected from the group consisting of 1 to 10;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

8. The bivalent DNA aptamer according to claim 4, wherein the bivalent DNA aptamer is in a single-stranded form and contains any of the following primary structures: 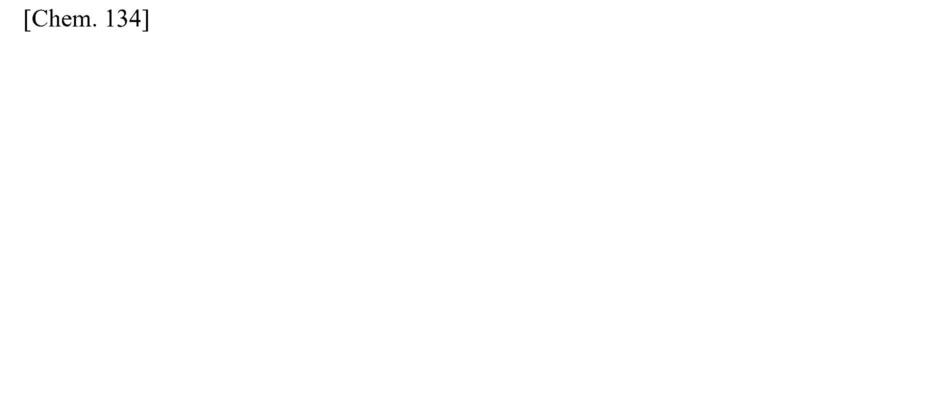 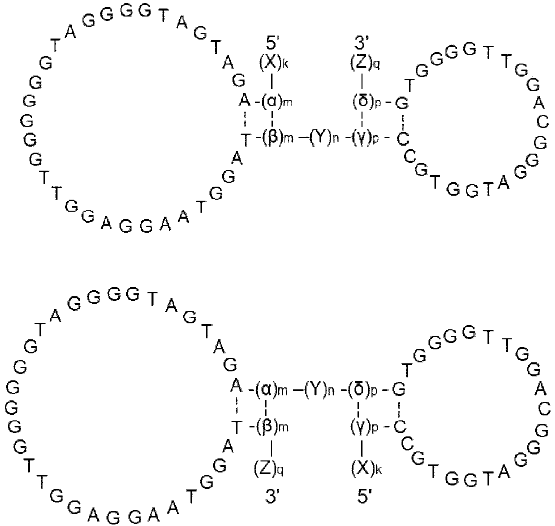 wherein
k, m, n, p, and q represent the numbers of DNA bases;
k is 0, 1, 2, or 3;
m is an integer selected from the group consisting of 1 to 15;
n is 0, 1, 2, 3, 4, or 5;
p is an integer selected from the group consisting of 1 to 13;
q is 0, 1, 2, or 3;
X, Y, and Z are each independently any bases;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

9. The bivalent DNA aptamer according to claim 1, wherein the bivalent DNA aptamer is in a single-stranded form and contains any of the following primary structures: 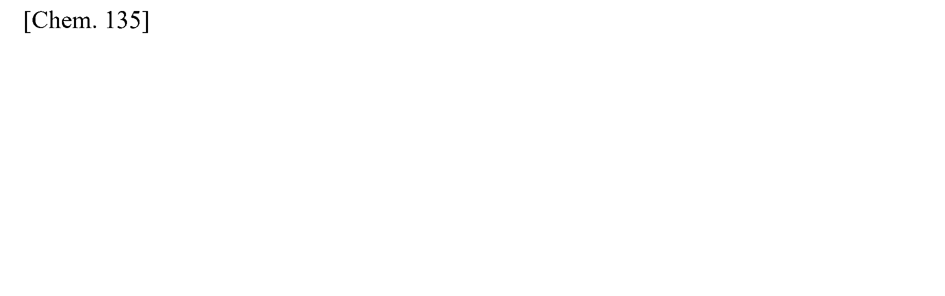 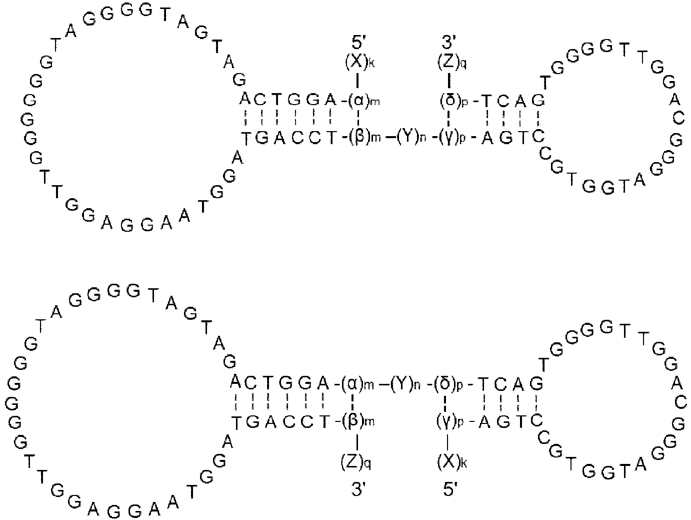 wherein
k, m, n, p, and q represent the numbers of DNA bases;
k is 0, 1, 2, or 3;
m is an integer selected from the group consisting of 1 to 10;
n is 0, 1, 2, 3, 4, or 5;
p is an integer selected from the group consisting of 1 to 10;
q is 0, 1, 2, or 3;
X, Y, and Z are each independently any bases;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

10. The bivalent DNA aptamer according to claim 1, wherein the bivalent DNA aptamer is in a single-stranded form and contains any of the following primary structures: 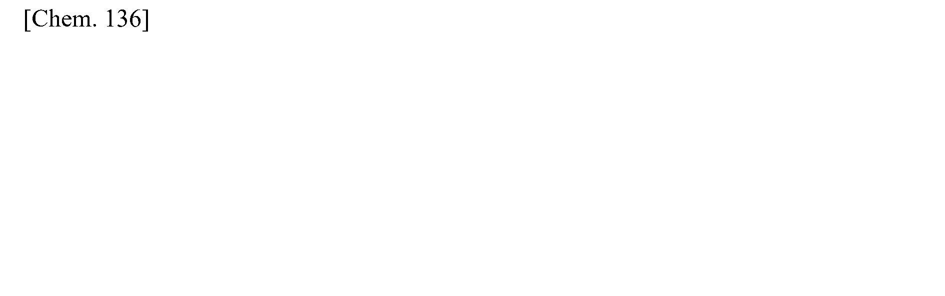 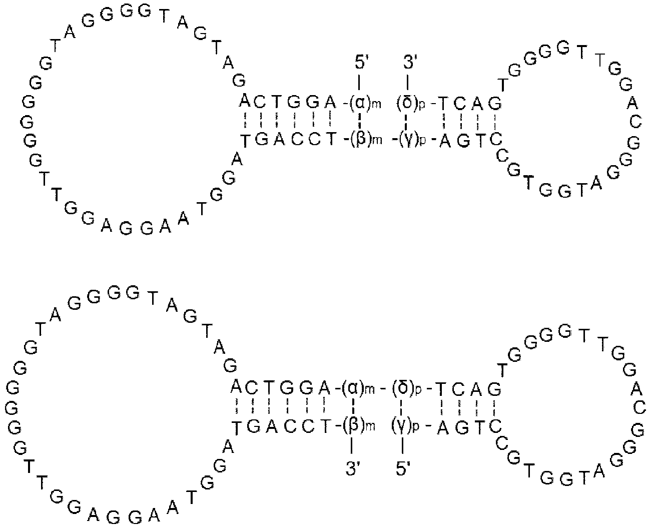 wherein
m and p represent the numbers of DNA bases;
m is an integer selected from the group consisting of 1 to 10;
p is an integer selected from the group consisting of 1 to 10;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

11. The bivalent DNA aptamer according to claim 4, wherein the bivalent DNA aptamer is in a single-stranded form and contains the following primary structure: 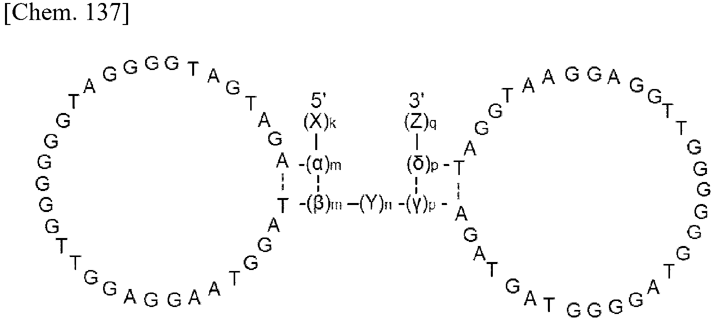 wherein
k, m, n, p, and q represent the numbers of DNA bases;
k is 0, 1, 2, or 3;
m is an integer selected from the group consisting of 1 to 15;
n is 0, 1, 2, 3, 4, or 5;
p is an integer selected from the group consisting of 1 to 15;
q is 0, 1, 2, or 3;
X, Y, and Z are each independently any bases;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

12. The bivalent DNA aptamer according to claim 4, wherein the bivalent DNA aptamer is in a single-stranded form and contains the following primary structure: 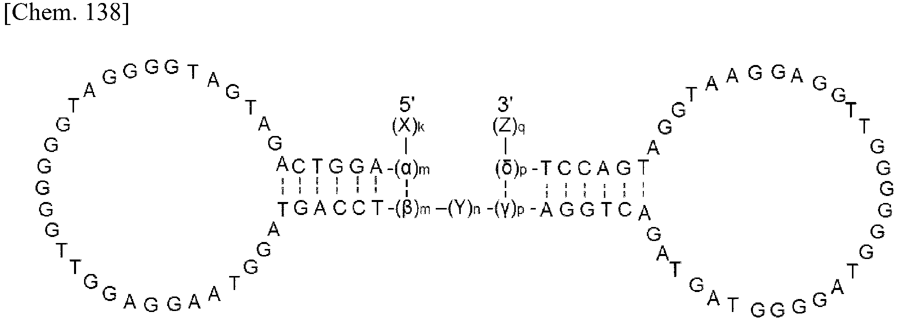 wherein
k, m, n, p, and q represent the numbers of DNA bases;
k is 0, 1, 2, or 3;
m is an integer selected from the group consisting of 1 to 10;
n is 0, 1, 2, 3, 4, or 5;
p is an integer selected from the group consisting of 1 to 10;
q is 0, 1, 2, or 3;
X, Y, and Z are each independently any bases;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

13. The bivalent DNA aptamer according to claim 4, wherein the bivalent DNA aptamer is in a single-stranded form and contains the following primary structure: 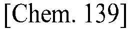 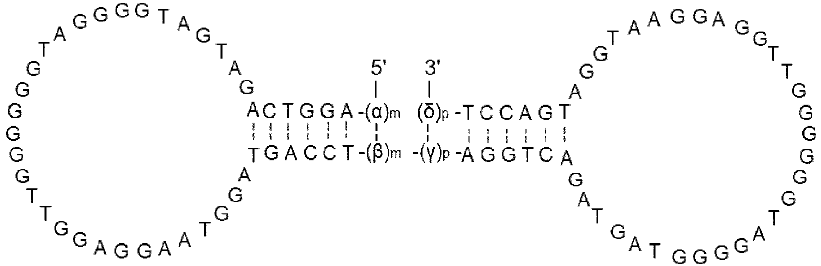 wherein
m and p represent the numbers of DNA bases;
m is an integer selected from the group consisting of 1 to 10;
p is an integer selected from the group consisting of 1 to 10;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

14. The bivalent DNA aptamer according to claim 4, wherein the bivalent DNA aptamer is in a single-stranded form and contains any of the following primary structures 1) to 5):
1) 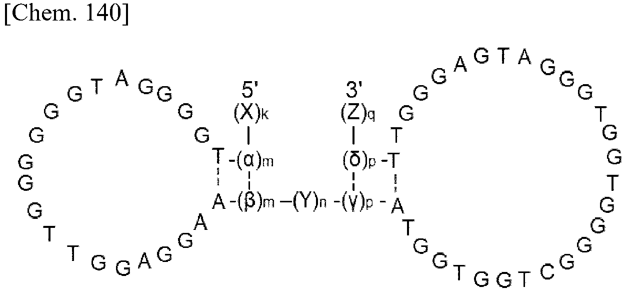
2) 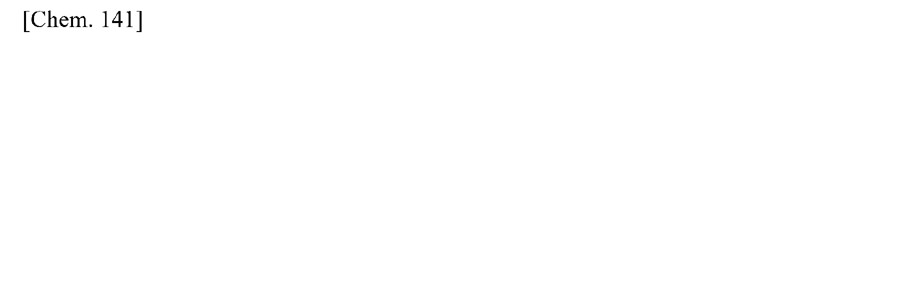 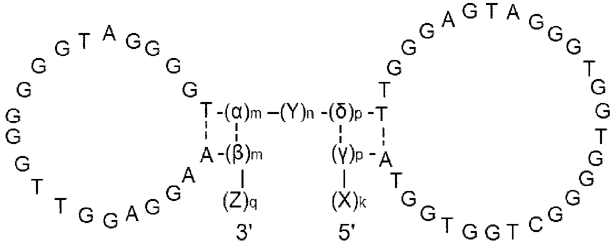
3) 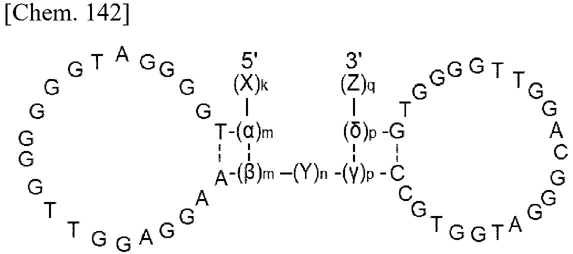
4) 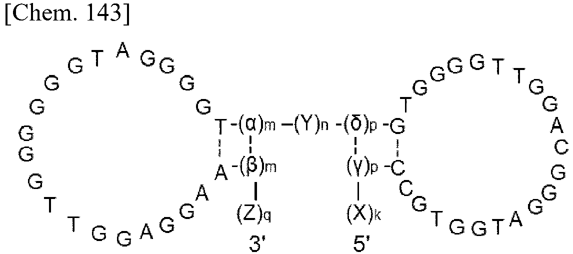
5) 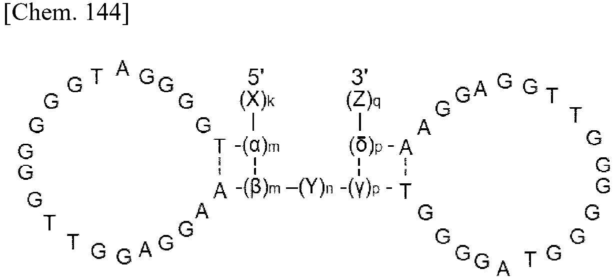 wherein
k, m, n, p, and q represent the numbers of DNA bases;
k is 0, 1, 2, or 3;
m is an integer selected from the group consisting of 1 to 10;
n is 0, 1, 2, 3, 4, or 5;
p is an integer selected from the group consisting of 1 to 10;
q is 0, 1, 2, or 3;
X, Y, and Z are each independently any bases;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

15. The bivalent DNA aptamer according to claim 1, wherein the bivalent DNA aptamer is in a single-stranded form and contains any of the following primary structures 1) to 5):
1) 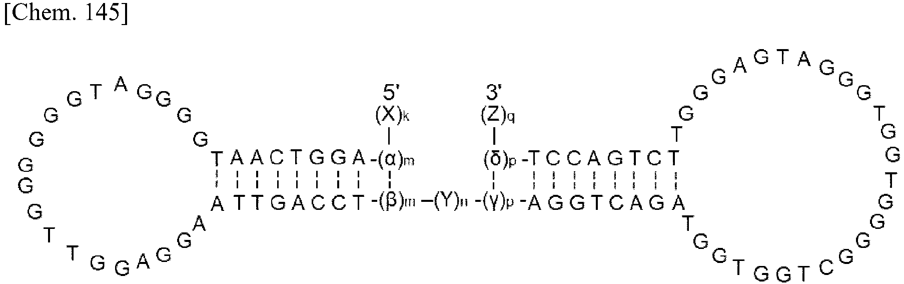
2) 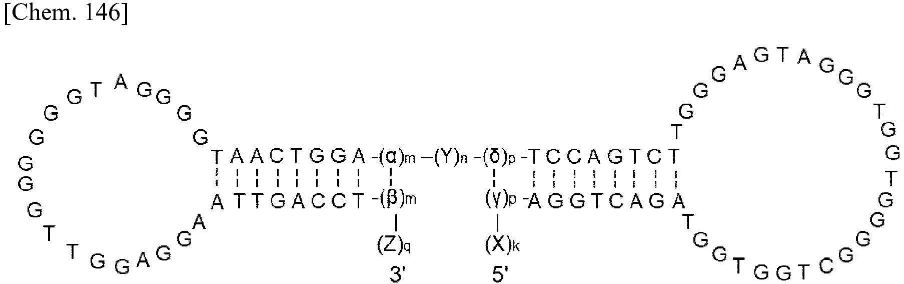
3) 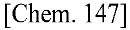 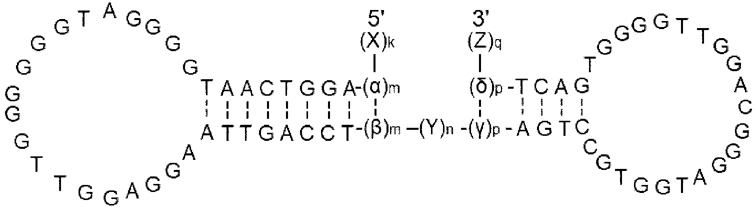
4) 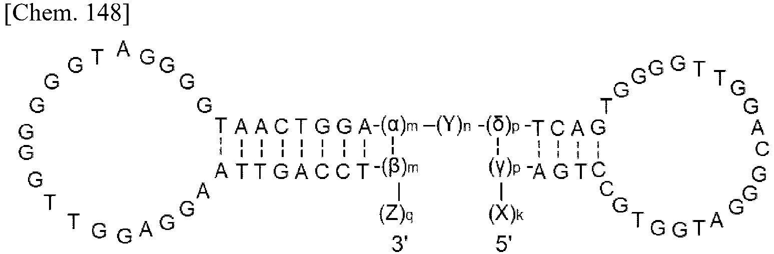
5) 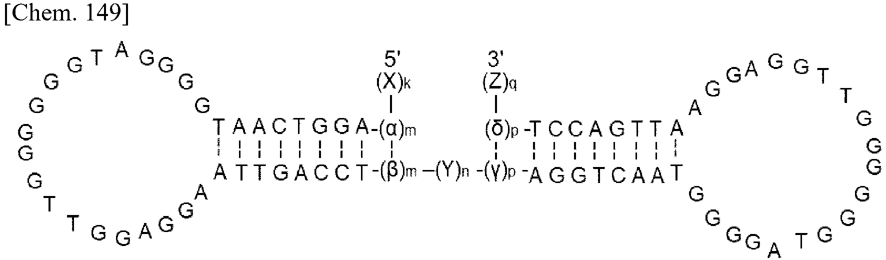 wherein
k, m, n, p, and q represent the numbers of DNA bases;
k is 0, 1, 2, or 3;
m is an integer selected from the group consisting of 1 to 10;
n is 0, 1, 2, 3, 4, or 5;
p is an integer selected from the group consisting of 1 to 10;
q is 0, 1, 2, or 3;
X, Y, and Z are each independently any bases;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

16. The bivalent DNA aptamer according to claim 4, wherein the bivalent DNA aptamer is in a single-stranded form and contains any of the following primary structures 1) to 5):
1) 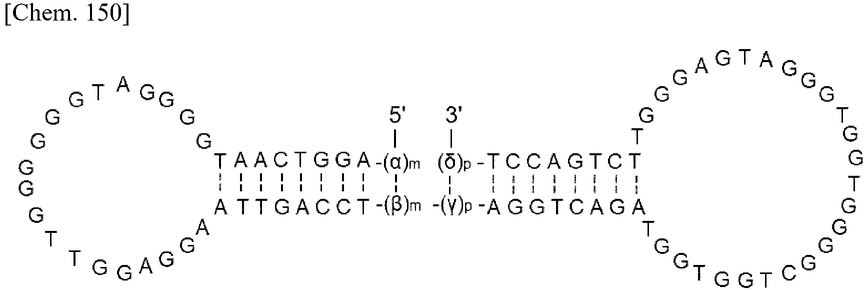
2) 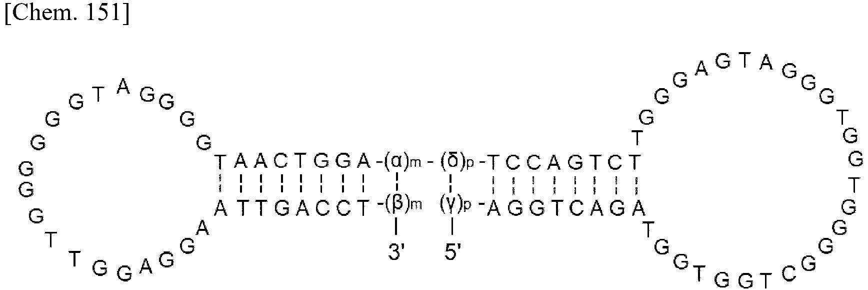
3) 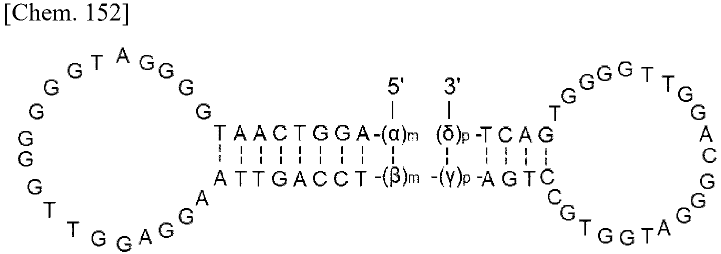
4) 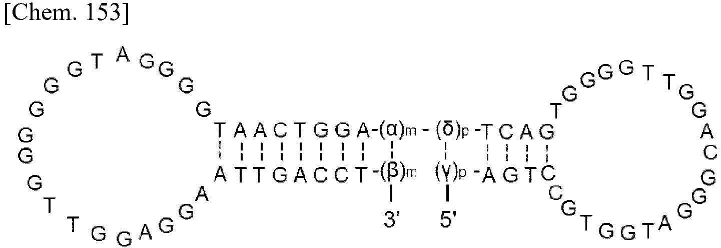
5) 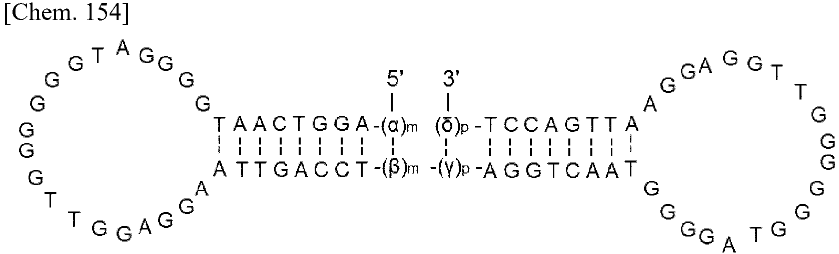 wherein
m and p represent the numbers of DNA bases;
m is an integer selected from the group consisting of 1 to 10;
p is an integer selected from the group consisting of 1 to 10;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

17. The single-stranded bivalent DNA aptamer according to any one of claims 5 to 16, wherein each set of α and β is bases that form a base pair; and/or each set of γ and δ is bases that form a base pair.

18. The single-stranded bivalent DNA aptamer according to any one of claims 5 to 16, wherein m + p equals 5 to 21.

19. The single-stranded bivalent DNA aptamer according to any one of claims 5 to 16, wherein m + p equals 5 to 17.

20. The single-stranded bivalent DNA aptamer according to claim 4, comprising the sequence of pse (M08s-M03s) (SEQ ID NO: 12).

21. The single-stranded bivalent DNA aptamer according to claim 4, comprising the sequence of pse (M08s-TBA29) (SEQ ID NO: 13).

22. The single-stranded bivalent DNA aptamer according to claim 4, comprising the sequence of pse (M08s-M08s) (SEQ ID NO: 22).

23. The single-stranded bivalent DNA aptamer according to claim 4, comprising the sequence of M08s-1 (36) (SEQ ID NO: 107) and the sequence of TBA29 (SEQ ID NO: 3).

24. The single-stranded bivalent DNA aptamer according to claim 4, comprising a sequence selected from the group consisting of Pse08-29 ssl-TTACG (70) (SEQ ID NO: 108); Pse08-29 ssl-5A (70) (SEQ ID NO: 109); Pse08-29 ss1-4A (69) (SEQ ID NO: 110); Pse08-29 ssl-2A (67) (SEQ ID NO: 111); Pse08-29 ssl-5T (70) (SEQ ID NO: 112); Pse08-29 ssl-4T (69) (SEQ ID NO: 113); Pse08-29 ssl-2T (67) (SEQ ID NO: 114); Pse08-29 ssl-0A (65) (SEQ ID NO: 115); Pse08-29 ss1-0Aa (65) (SEQ ID NO: 116); Pse08-29 ssl-0Ab (65) (SEQ ID NO: 117); Pse08-29 ss1-0Ac (65) (SEQ ID NO: 118); Pse08-29 ss2-CGTAA (70) (SEQ ID NO: 119); Pse08-29 ss2-5A (70) (SEQ ID NO: 120); Pse08-29 ss2-4A (69) (SEQ ID NO: 121); Pse08-29 ss2-2A (67) (SEQ ID NO: 122); Pse08-29 ss2-5T (70) (SEQ ID NO: 123); Pse08-29 ss2-4T (69) (SEQ ID NO: 124); Pse08-29 ss2-2T (67) (SEQ ID NO: 125); Pse08-29 ss2-0B (65) (SEQ ID NO: 126); Pse08-29 ss2-0Ba (65) (SEQ ID NO: 127); Pse08-29 ss2-0Bb (65) (SEQ ID NO: 127); and Pse08-29 ss2-0Bc (65) (SEQ ID NO: 129).

25. The single-stranded bivalent DNA aptamer according to claim 4, comprising two copies of the sequence of M08s-1 (36) (SEQ ID NO: 107).

26. The single-stranded bivalent DNA aptamer according to claim 4, comprising a sequence selected from the group consisting of Pse08-08 (72) (SEQ ID NO: 130); Pse08-08 (68) (SEQ ID NO: 131); Pse08-08 (64) (SEQ ID NO: 132); Pse08-08 (60) (SEQ ID NO: 133); Pse08-08 (72-GC) (SEQ ID NO: 134); and Pse08-08 (60-GC) (SEQ ID NO: 135).

27. The bivalent DNA aptamer according to claim 4, wherein the bivalent DNA aptamer is in the following double-stranded form and consists of:
an oligonucleotide A comprising the sequence of 5'-(α)ₘ-AGATGATGGGGATGGGGGGTTGGAGGAATGGAT-(β)ₘ-(X)ₙ-3', and
an oligonucleotide B comprising the sequence of 5'-(γ)ₚ-ATGGTGGTCGGGGTGGTGGGATGAGGGTTCTGACCT-(δ)ₘ-(Y)ₙ-3': 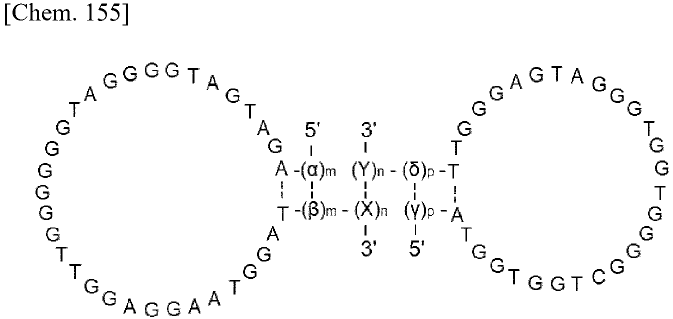
wherein
m, n, and p represent the numbers of DNA bases;
m is an integer selected from the group consisting of 0 to 25;
n is an integer selected from the group consisting of 6 to 25;
p is an integer selected from the group consisting of 0 to 27;
X, Y, a, β, γ, and δ are bases;
5'-(X)ₙ-3' and 5'-(Y)ₙ-3' form an antiparallel double-stranded structure;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

28. The bivalent DNA aptamer according to claim 4, wherein the DNA aptamer is in the following double-stranded form and consists of:
an oligonucleotide A comprising the sequence of 5'-(α)ₘ-AGGTCAGATGATGGGGATGGGGGGTTGGAGGAATGGATGACCT-(β)ₘ-(X)ₙ-3', and
an oligonucleotide B comprising the sequence of 5'-(γ)ₚ-AGGTCAGATGGTGGTCGGGGTGGTGGGATGAGGGTT-(δ)ₘ-(Y)ₙ-3': 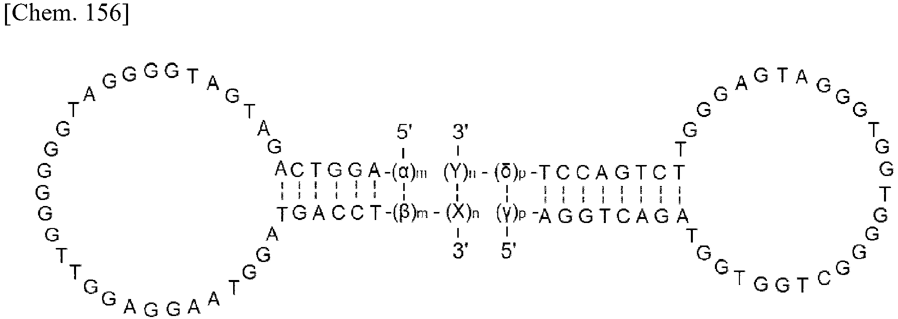
wherein
m, n, and p represent the numbers of DNA bases;
m is 0 to 20;
n is an integer selected from the group consisting of 6 to 25;
p is an integer selected from the group consisting of 0 to 20;
X, Y, α, β, γ, and δ are bases;
5'-(X)ₙ-3' and 5'-(Y)ₙ-3' form an antiparallel double-stranded structure;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

29. The bivalent DNA aptamer according to claim 4, wherein the bivalent DNA aptamer is in the following double-stranded form and consists of:
an oligonucleotide A comprising the sequence of 5'-(Y)ₙ-(α)ₘ-AGATGATGGGGATGGGGGGTTGGAGGAATGGAT-(β)ₘ-3', and
an oligonucleotide B comprising the sequence of 5'-(X)ₙ-(γ)ₚ-AYGGTGGTCGGGGTGGTGGGATGAGGGTT-(δ)ₘ-3': 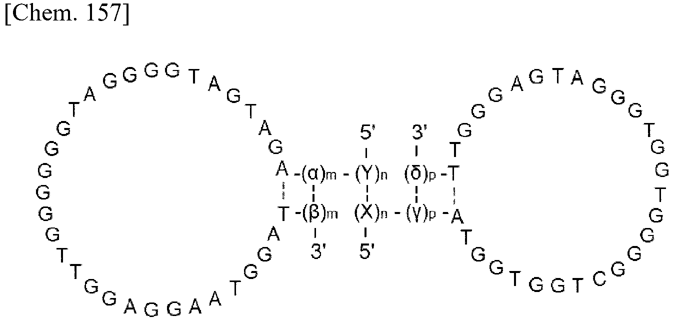
wherein
m, n, and p represent the numbers of DNA bases;
m is an integer selected from the group consisting of 0 to 25;
n is an integer selected from the group consisting of 6 to 25;
p is an integer selected from the group consisting of 0 to 27;
X, Y, α, β, γ, and δ are bases;
5'-(X)ₙ-3' and 5'-(Y)ₙ-3' form an antiparallel double-stranded structure;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

30. The bivalent DNA aptamer according to claim 4, wherein the bivalent DNA aptamer is in the following double-stranded form and consists of:
an oligonucleotide A comprising the sequence of 5'-(Y)ₙ-(α)ₘ-AGGTCAGATGATGGGGATGGGGGGTTGGAGGAATGGATGACCT-(β)ₘ-3', and
an oligonucleotide B comprising the sequence of 5'-(X)ₙ-(γ)ₚ-AGGTCAGATGGTGGTCGGGGTGGTGGGATGAGGGTTCTGACCT-(δ)ₘ-3': 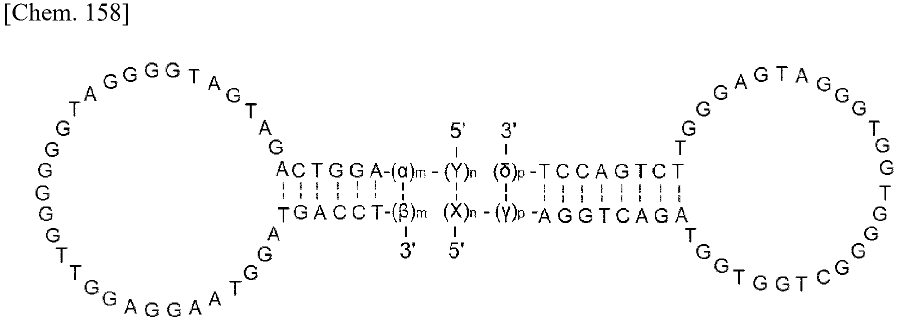
wherein
m, n, and p represent the numbers of DNA bases;
m is an integer selected from the group consisting of 0 to 20;
n is an integer selected from the group consisting of 6 to 25;
p is an integer selected from the group consisting of 0 to 20;
X, Y, α, β, γ, and δ are bases;
5'-(X)ₙ-3' and 5'-(Y)ₙ-3' form an antiparallel double-stranded structure;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

31. The bivalent DNA aptamer according to claim 4, wherein the bivalent DNA aptamer is in the following double-stranded form and consists of:
an oligonucleotide A comprising the sequence of 5'-(α)ₘ-AGATGATGGGGATGGGGGGTTGGAGGAATGGAT-(β)ₘ-(X)ₙ-3', and
an oligonucleotide B comprising the sequence of 5'-(γ)ₚ-CCGTGGTAGGGCAGGTTGGGGTG-(δ)ₘ-(Y)ₙ-3': 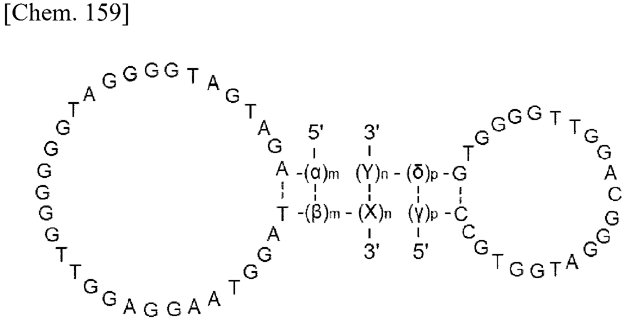
wherein
m, n, and p represent the numbers of DNA bases;
m is an integer selected from the group consisting of 0 to 25;
n is an integer selected from the group consisting of 6 to 25;
p is an integer selected from the group consisting of 0 to 23;
X, Y, α, β, γ, and δ are bases;
5'-(X)ₙ-3' and 5'-(Y)ₙ-3' form an antiparallel double-stranded structure;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

32. The bivalent DNA aptamer according to claim 4, wherein the bivalent DNA aptamer is in the following double-stranded form and consists of:
an oligonucleotide A comprising the sequence of 5'-(α)ₘ-AGGTCAGATGATGGGGATGGGGGGTTGGAGGAATGGATGACCT-(β)ₘ-(X)ₙ-3', and
an oligonucleotide B comprising the sequence of 5'-(γ)ₚ-AGTCCGTGGTAGGGCAGGTTGGGGTGACT-(δ)ₚ-(Y)ₙ-3': 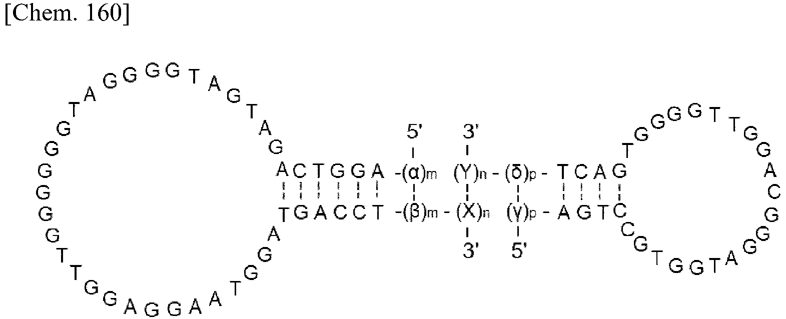
wherein
m, n, and p represent the numbers of DNA bases;
m is 0 to 20;
n is an integer selected from the group consisting of 6 to 25;
p is an integer selected from the group consisting of 0 to 20;
X, Y, α, β, γ, and δ are bases;
5'-(X)ₙ-3' and 5'-(Y)ₙ-3' form an antiparallel double-stranded structure;
5'-(α)ₘ-3' and 5'-(β)ₘ-3' form an antiparallel double-stranded structure; and
5'-(γ)ₚ-3' and 5'-(6)ₚ-3' form an antiparallel double-stranded structure.

33. The bivalent DNA aptamer according to claim 4, wherein the bivalent DNA aptamer is in the following double-stranded form and consists of:
an oligonucleotide A comprising the sequence of 5'-(Y)ₙ-(α)ₘ-AGGTCAGATGATGGGGATGGGGGGTTGGAGGAATGGATGACCT-(β)ₘ-(X)ₙ-3', and
an oligonucleotide B comprising the sequence of 5'-(X)ₙ-(γ)ₚ-CGTGGTAGGGCAGGTTGGGGT-(δ)ₚ-3': 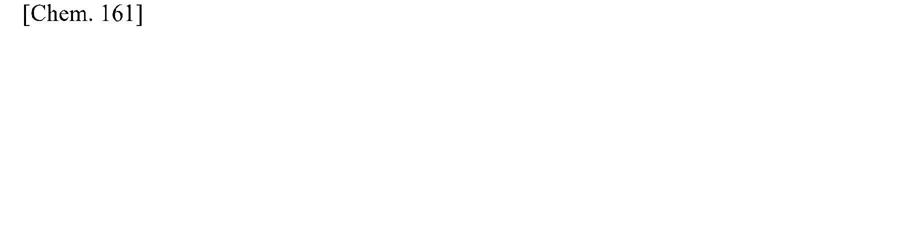 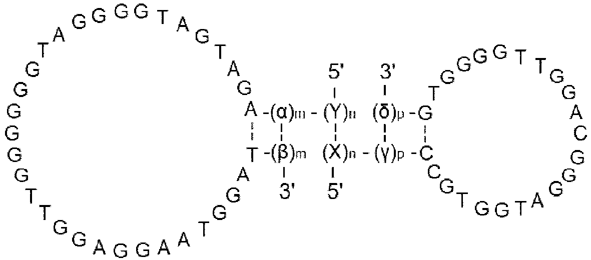
wherein
m, n, and p represent the numbers of DNA bases;
m is an integer selected from the group consisting of 0 to 25;
n is an integer selected from the group consisting of 6 to 25;
p is an integer selected from the group consisting of 0 to 23;
X, Y, α, β, γ, and δ are bases;
5'-(X)ₙ-3' and 5'-(Y)ₙ-3' form an antiparallel double-stranded structure;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

34. The bivalent DNA aptamer according to claim 4, wherein the DNA aptamer is in the following double-stranded form and consists of:
an oligonucleotide A comprising the sequence of 5'-(Y)ₙ-(α)ₘ-AGGTCAGATGATGGGGATGGGGGGTTGGAGGAATGGATGACCT-(β)ₘ-(X)ₙ-3', and
an oligonucleotide B comprising the sequence of 5'-(X)ₙ-(γ)ₚ-AGTCCGTGGTAGGGCAGGTTGGGGTGACT-(δ)ₚ-3': 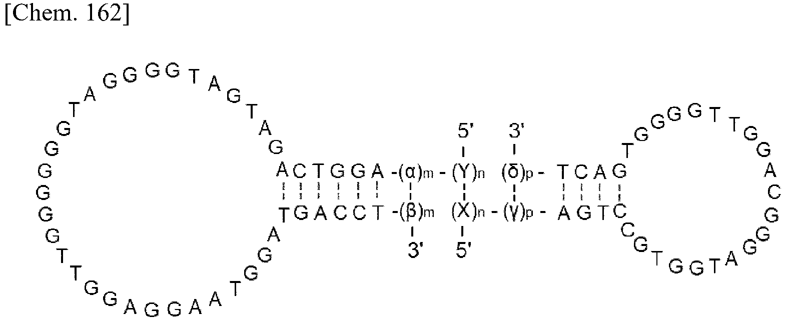
wherein
m, n, and p represent the numbers of DNA bases;
m is 0 to 20;
n is an integer selected from the group consisting of 6 to 25;
p is an integer selected from the group consisting of 0 to 20;
X, Y, α, β, γ, and δ are bases;
5'-(X)ₙ-3' and 5'-(Y)ₙ-3' form an antiparallel double-stranded structure;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

35. The bivalent DNA aptamer according to claim 4, wherein the DNA aptamer is in the following double-stranded form and consists of:
an oligonucleotide A comprising the sequence of 5'-(α)ₘ-AGATGATGGGGATGGGGGGTTGGAGGAATGGAT-(β)ₘ-(X)ₙ-3', and
an oligonucleotide B comprising the sequence of 5'-(y)ₚ-AGATGATGGGGATGGGGGGTTGGAGGAATGGAT-(δ)ₚ-(Y)ₙ-3': 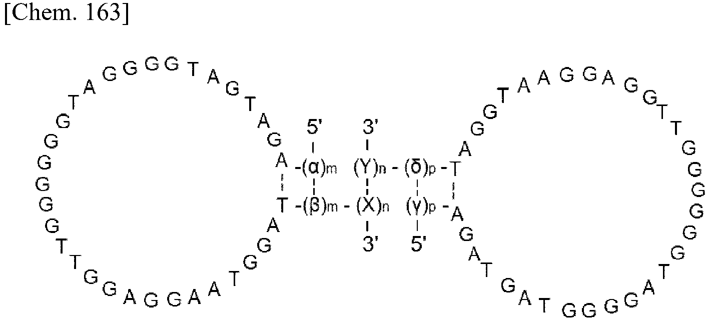
wherein
m, n, and p represent the numbers of DNA bases;
m is an integer selected from the group consisting of 0 to 25;
n is an integer selected from the group consisting of 6 to 25;
p is an integer selected from the group consisting of 0 to 25;
X, Y, α, β, γ, and δ are bases;
5'-(X)ₙ-3' and 5'-(Y)ₙ-3' form an antiparallel double-stranded structure;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

36. The bivalent DNA aptamer according to claim 4, wherein the DNA aptamer is in the following double-stranded form and consists of:
an oligonucleotide A comprising the sequence of 5'-(α)ₘ-AGGTCAGATGATGGGGATGGGGGGTTGGAGGAATGGATGACCT-(β)ₘ-(X)ₙ-3', and
an oligonucleotide B comprising the sequence of 5'-(γ)ₚ-AGGTCAGATGATGGGGATGGGGGGTTGGAGGAATGGATGACCT-(δ)ₚ-(Y)ₙ-3': 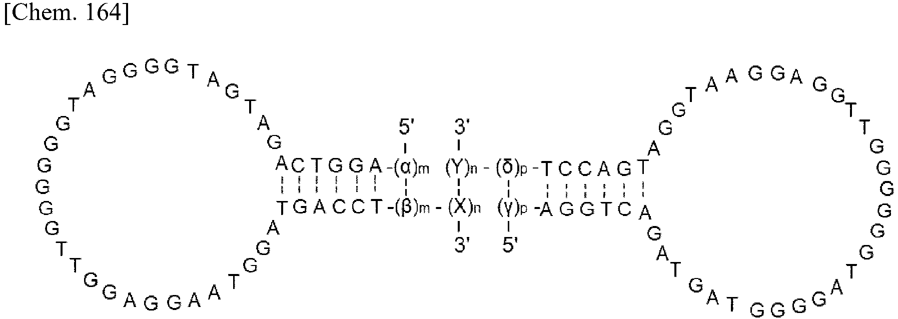
wherein
m, n, and p represent the numbers of DNA bases;
m is 0 to 20;
n is an integer selected from the group consisting of 6 to 25;
p is an integer selected from the group consisting of 0 to 20;
X, Y, α, β, γ, and δ are bases;
5'-(X)ₙ-3' and 5'-(Y)ₙ-3' form an antiparallel double-stranded structure;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

37. The bivalent DNA aptamer according to claim 4, wherein the DNA aptamer is in the following double-stranded form and consists of:
an oligonucleotide A comprising the sequence of 5'-(Y)ₙ-(α)ₘ-AGATGATGGGGATGGGGGGTTGGAGGAATGGAT-(β)ₘ-3', and
an oligonucleotide B comprising the sequence of 5'-(X)ₙ-(γ)ₚ-AGATGATGGGGATGGGGGGTTGGAGGAATGGAT-(δ)ₚ-3': 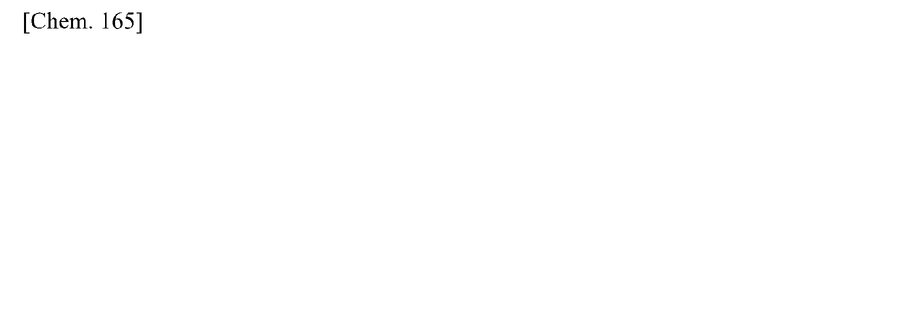 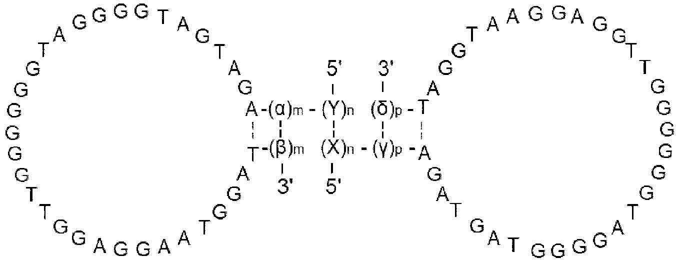
wherein
m, n, and p represent the numbers of DNA bases;
m is an integer selected from the group consisting of 0 to 25;
n is an integer selected from the group consisting of 6 to 25;
p is an integer selected from the group consisting of 0 to 25;
X, Y, α, β, γ, and δ are bases;
5'-(X)ₙ-3' and 5'-(Y)ₙ-3' form an antiparallel double-stranded structure;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

38. The bivalent DNA aptamer according to claim 4, wherein the DNA aptamer is in the following double-stranded form and consists of:
an oligonucleotide A comprising the sequence of 5'-(Y)ₙ-(α)ₘ-AGGTCAGATGATGGGGATGGGGGGTTGGAGGAATGGATGACCT-(β)ₘ-3', and
an oligonucleotide B comprising the sequence of 5'-(X)ₙ-(γ)ₚ-AGGTCAGATGGTGGTCGGGGTGGTGGGATGAGGGTTCTGACCT-(δ)ₚ-3': 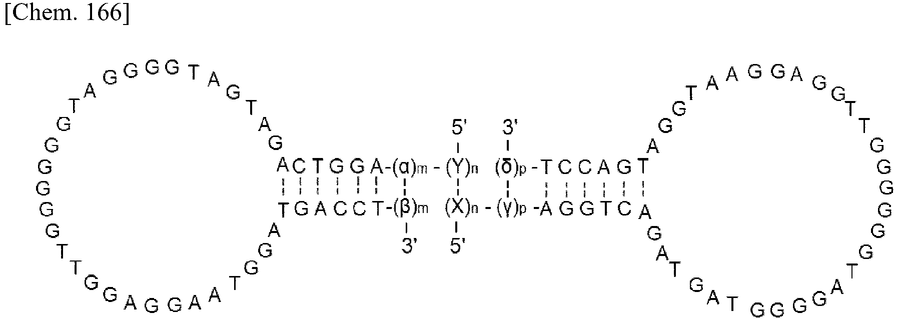
wherein
m, n, and p represent the numbers of DNA bases;
m is an integer selected from the group consisting of 0 to 20;
n is an integer selected from the group consisting of 6 to 25;
p is an integer selected from the group consisting of 0 to 20;
X, Y, α, β, γ, and δ are bases;
5'-(X)ₙ-3' and 5'-(Y)ₙ-3' form an antiparallel double-stranded structure;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure.

39. The double-stranded bivalent DNA aptamer according to any one of claims 27 to 38, wherein
each set of X and Y is bases that form a base pair,
each set of α and β is bases that form a base pair; and/or
each set of γ and δ is bases that form a base pair.

40. The double-stranded bivalent DNA aptamer according to any one of claims 27 to 38, wherein m + n + p equals 5 to 21.

41. The double-stranded bivalent DNA aptamer according to any one of claims 28, 30, 32, 34, 36, and 38, wherein the sequence of 5'-(β)ₘ-(X)ₙ- (γ)ₚ-3' is 5'-CTAGTGACTGATTTACG-3', and the sequence of 5'-(δ)ₚ-(Y)ₙ-(α)ₘ-3' is 5'-CGTAAATCAGTCACTAG-3'.

42. The double-stranded bivalent DNA aptamer according to claim 41, wherein m is 0, n is 17, and p is 0.

43. The double-stranded bivalent DNA aptamer according to claim 42, wherein the sequence of 5'-(X)ₙ-3' is 5'-CTAGTGACTGATTTACG-3', and the sequence of 5'-(Y)ₙ-3' is 5'-CGTAAATCAGTCACTAG-3'.

44. The bivalent DNA aptamer according to claim 4, wherein the DNA aptamer is in a double-stranded form and consists of a combination of oligonucleotides selected from the group consisting of the following 1 to 40 combinations of oligonucleotides A and oligonucleotides B:
**[Table 8]**
| Combination | Oligonucleotide A | | Oligonucleotide B | |
|---|---|---|---|---|
| | Name | SEQ ID NO: | Name | SEQ ID NO: |
| 1 | Sticky end20A-1 | 26 | Sticky end20A-2 | 27 |
| 2 | Sticky end20B-1 | 28 | Sticky end20B-2 | 29 |
| 3 | Sticky end20C-1 | 30 | Sticky end20C-2 | 31 |
| 4 | Sticky end20D-1 | 32 | Sticky end20D-2 | 33 |
| 5 | Sticky end20E-1 | 34 | Sticky end20E-2 | 35 |
| 6 | Sticky end19A-1 | 36 | Sticky end19A-2 | 37 |
| 7 | Sticky end19B-1 | 38 | Sticky end19B-2 | 39 |
| 8 | Sticky end19C-1 | 40 | Sticky end19C-2 | 41 |
| 9 | Sticky end19D-1 | 42 | Sticky end19D-2 | 43 |
| 10 | Sticky end19E-1 | 44 | Sticky end19E-2 | 45 |
| 11 | Sticky end18A-1 | 46 | Sticky end18A-2 | 47 |
| 12 | Sticky end18B-1 | 48 | Sticky end18B-2 | 49 |
| 13 | Sticky end18C-1 | 50 | Sticky end18C-2 | 51 |
| 14 | Sticky end18D-1 | 52 | Sticky end18D-2 | 53 |
| 15 | Sticky end18E-1 | 54 | Sticky end18E-2 | 55 |
| 16 | Sticky end17A-1 | 56 | Sticky end17A-2 | 57 |
| 17 | Sticky end17B-1 | 58 | Sticky end17B-2 | 59 |
| 18 | Sticky end17C-1 | 60 | Sticky end17C-2 | 61 |
| 19 | Sticky end17D-1 | 62 | Sticky end17D-2 | 63 |
| 20 | Sticky end17E-1 | 64 | Sticky end17E-2 | 65 |
| 21 | Sticky end16A-1 | 66 | Sticky end16A-2 | 67 |
| 22 | Sticky end16B-1 | 68 | Sticky end16B-2 | 69 |
| 23 | Sticky end16C-1 | 70 | Sticky end16C-2 | 71 |
| 24 | Sticky end16D-1 | 72 | Sticky end16D-2 | 73 |
| 25 | Sticky end16E-1 | 74 | Sticky end16E-2 | 75 |
| 26 | Sticky end15A-1 | 76 | Sticky end15A-2 | 77 |
| 27 | Sticky end15B-1 | 78 | Sticky end15B-2 | 79 |
| 28 | Sticky end15C-1 | 80 | Sticky end15C-2 | 81 |
| 29 | Sticky end15D-1 | 82 | Sticky end15D-2 | 83 |
| 30 | Sticky end15E-1 | 84 | Sticky end15E-2 | 85 |
| 31 | Sticky end14A-1 | 86 | Sticky end14A-2 | 87 |
| 32 | Sticky end14B-1 | 88 | Sticky end14B-2 | 89 |
| 33 | Sticky end14C-1 | 90 | Sticky end14C-2 | 91 |
| 34 | Sticky end14D-1 | 92 | Sticky end14D-2 | 93 |
| 35 | Sticky end14E-1 | 94 | Sticky end14E-2 | 95 |
| 36 | Sticky end13A-1 | 96 | Sticky end13A-2 | 97 |
| 37 | Sticky end13B-1 | 98 | Sticky end13B-2 | 99 |
| 38 | Sticky end13C-1 | 100 | Sticky end13C-2 | 101 |
| 39 | Sticky end13D-1 | 102 | Sticky endl3D-2 | 103 |
| 40 | Sticky end13E-1 | 104 | Sticky end13E-2 | 105 |

45. A pharmaceutical composition for inhibiting blood coagulation, comprising the DNA aptamer according to any one of claims 1 to 3.

46. A pharmaceutical composition for inhibiting blood coagulation, comprising the bivalent DNA aptamer according to any one of claims 4 to 44.

47. A neutralizing agent for a pharmaceutical composition for inhibiting blood coagulation,
wherein the pharmaceutical composition comprises the bivalent DNA aptamer according to any one of claims 5 to 22, and 27 to 44, and
the neutralizing agent comprises a nucleic acid comprising a complementary sequence to 5'-AGGTCAGATGATGGGGATGGG-3' (SEQ ID NO: 23).

48. A neutralizing agent for a pharmaceutical composition for inhibiting blood coagulation,
wherein the pharmaceutical composition comprises the bivalent DNA aptamer according to any one of claims 5 to 22, and 27 to 44, and
the neutralizing agent comprises a nucleic acid comprising a sequence described in [L-M08s]c (SEQ ID NO: 17) or a nucleic acid comprising a sequence described in 08ad (SEQ ID NO: 15).

49. A neutralizing agent for a pharmaceutical composition for inhibiting blood coagulation,
wherein the pharmaceutical composition comprises a DNA aptamer comprising the sequence of M08s-1 (36) (SEQ ID NO: 107), and
the neutralizing agent comprises a nucleic acid comprising a sequence selected from the group consisting of Ant-M08_36-36 (SEQ ID NO: 142); Ant-M08_36-30 (SEQ ID NO: 143); Ant-M08_36-24 (SEQ ID NO: 144); Ant-M08_36-18 (SEQ ID NO: 145); Ant-M08_36-L18 (SEQ ID NO: 146); Ant-M08_36-R18 (SEQ ID NO: 147); and Ant-M08_36-12 (SEQ ID NO: 148).

50. A neutralizing agent for a pharmaceutical composition for inhibiting blood coagulation,
wherein the pharmaceutical composition comprises a DNA aptamer comprising the sequence of Pse08-08 (64) (SEQ ID NO: 138), and
the neutralizing agent comprises a nucleic acid comprising the sequence of 2Ant44 _Pse08-08 (64) (SEQ ID NO: 149).

51. A method for producing a pharmaceutical composition for inhibiting blood coagulation comprising a bivalent DNA aptamer, comprising the steps of:
1) preparing or providing a test single-stranded oligonucleotide comprising any of the following structures: 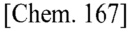 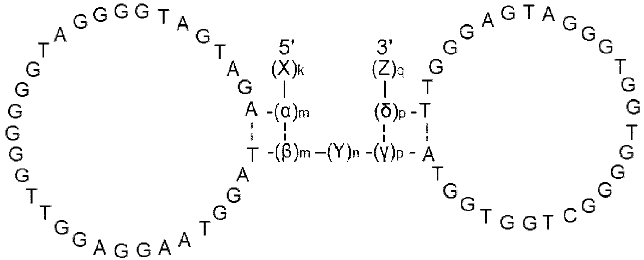 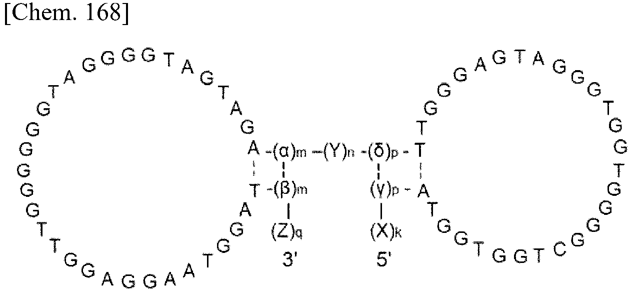 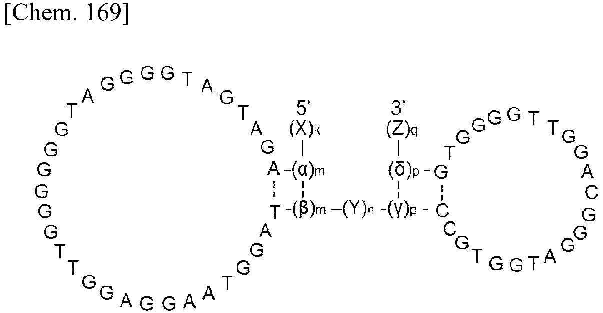 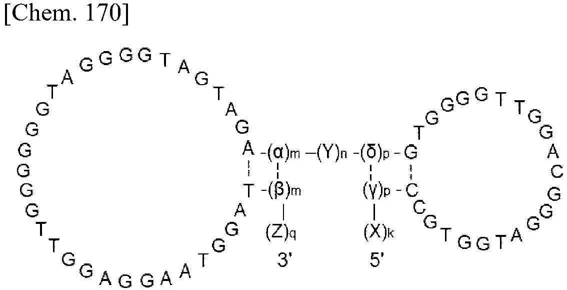 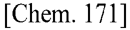 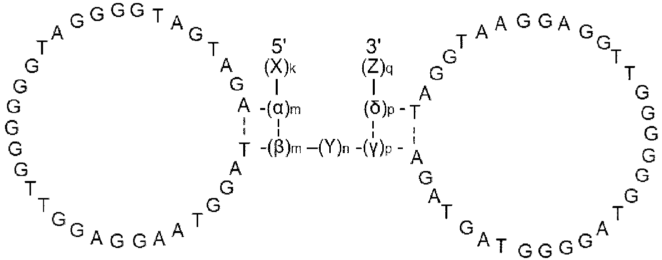 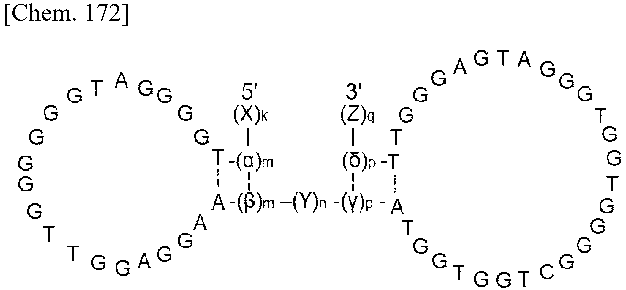 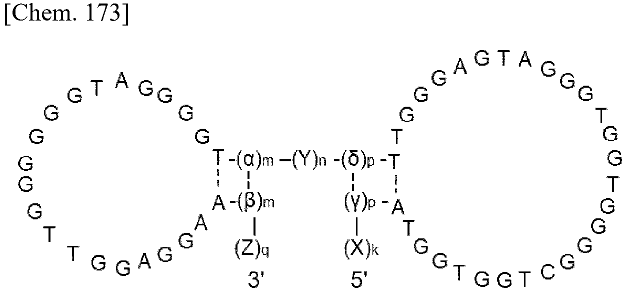 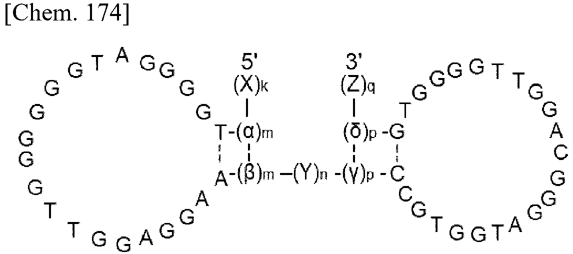 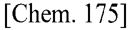 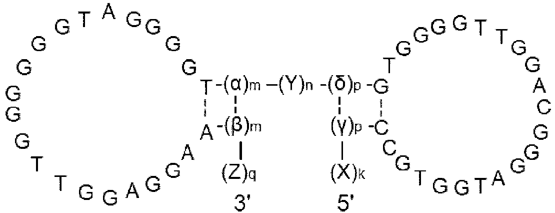 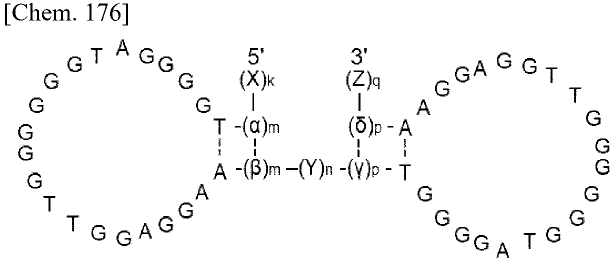 wherein
k, m, n, p, and q represent the numbers of DNA bases;
k is 0, 1, 2, or 3;
m is an integer selected from the group consisting of 1 to 15;
n is 0, 1, 2, 3, 4, or 5;
p is an integer selected from the group consisting of 1 to 15;
q is 0, 1, 2, or 3;
X, Y, and Z are each independently any bases;
α, β, γ, and δ are bases;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure;
and
2) examining an affinity of the test oligonucleotide for thrombin,
wherein
when the affinity of the test oligonucleotide for thrombin is superior to that of the DNA aptamer consisting of the sequence of lin (M08s-TBA29) (SEQ ID NO: 11), the test oligonucleotide is to be contained in the pharmaceutical composition as a bivalent DNA aptamer for inhibiting blood coagulation.

52. A method for producing a pharmaceutical composition for inhibiting blood coagulation comprising a bivalent DNA aptamer, comprising the steps of:
1) preparing or providing a test double-stranded oligonucleotide comprising any of the following structures: 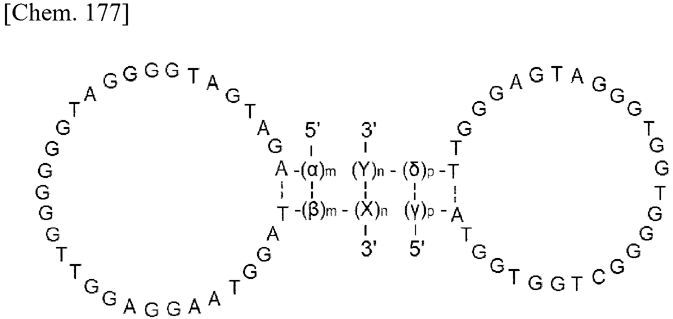 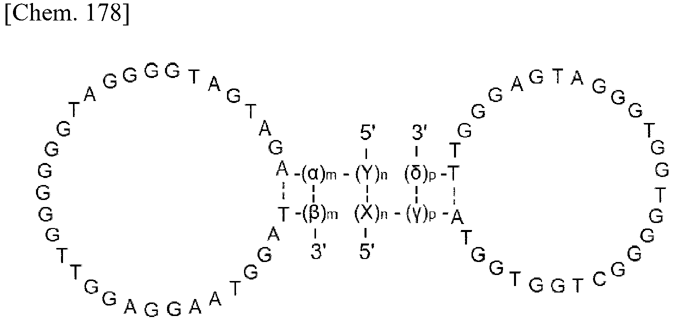 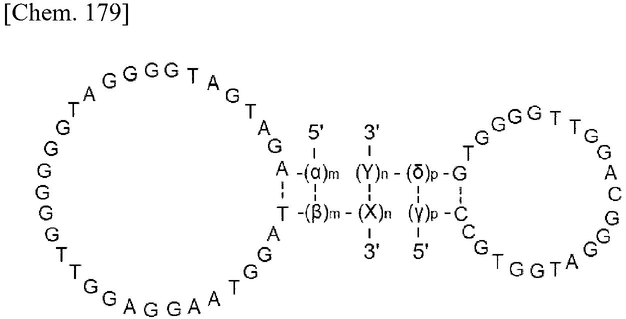 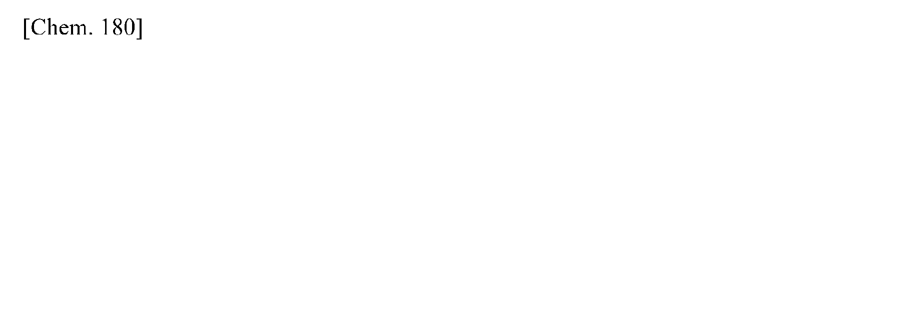 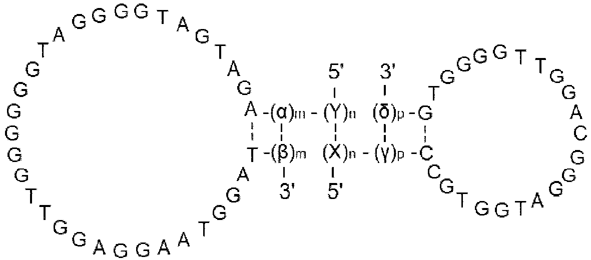 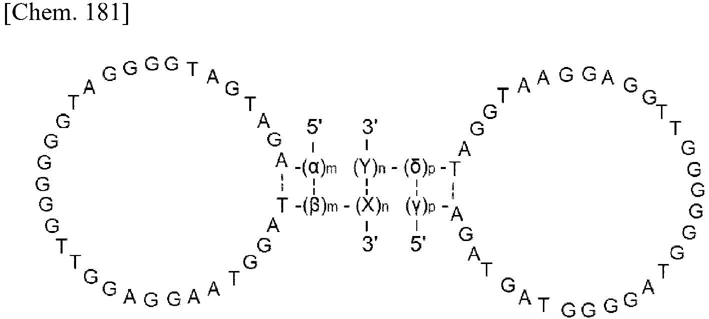 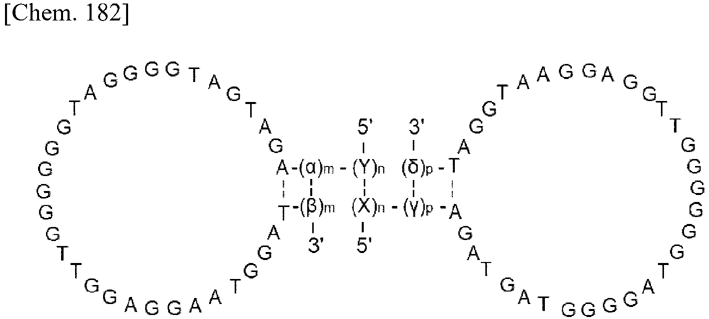 wherein
m, n, and p represent the numbers of DNA bases;
m is an integer selected from the group consisting of 0 to 25;
n is an integer selected from the group consisting of 6 to 25;
p is an integer selected from the group consisting of 0 to 25;
X, Y, α, β, γ, and δ are bases;
5'-(X)ₙ-3' and 5'-(Y)ₙ-3' form an antiparallel double-stranded structure;
5'-(α)ₘ-3' and 5'-(β)ₘ-3' form an antiparallel double-stranded structure; and
5'-(γ)ₚ-3' and 5'-(δ)ₚ-3' form an antiparallel double-stranded structure;
and
2) examining an affinity of the test oligonucleotide for thrombin,
wherein
when the affinity of the test oligonucleotide for thrombin is superior to that of the DNA aptamer consisting of the sequence of lin (M08s-TBA29) (SEQ ID NO: 11), the test oligonucleotide is to be contained in the pharmaceutical composition as a bivalent DNA aptamer for inhibiting blood coagulation.

53. The production method according to claim 52, wherein
each set of α and β is bases that form a base pair; and/or
each set of γ and δ is bases that form a base pair.

54. The production method according to claim 52, wherein n is 0, and m + p equals 5 to 21.

55. The production method according to claim 52, wherein
each set of X and Y is bases that form a base pair,
each set of α and β is bases that form a base pair; and/or
each set of γ and δ is bases that form a base pair.

56. The production method according to claim 52, wherein m + n + p equals 5 to 21.

57. The production method according to claim 52, wherein the sequence of 5'-(β)ₘ-(X)ₙ-(γ)ₚ-3' is 5'-CTAGTGACTGATTTACG-3', and the sequence of 5'-(δ)ₚ-(Y)ₙ-(α)ₘ-3' is 5'-CGTAAATCAGTCACTAG-3'.

58. The production method according to claim 52, wherein m is 0, n is 17, and p is 0.
